(19) Europäisches Patentamt
European Patent Office
Office européen des brevets

(11) EP 0 687 253 B1

(12) # EUROPÄISCHE PATENTSCHRIFT

(45) Veröffentlichungstag und Bekanntmachung des Hinweises auf die Patenterteilung:
**16.12.1998 Patentblatt 1998/51**

(21) Anmeldenummer: **94909909.7**

(22) Anmeldetag: **02.03.1994**

(51) Int Cl.[6]: **C07D 213/74**, A61K 31/44

(86) Internationale Anmeldenummer:
**PCT/EP94/00608**

(87) Internationale Veröffentlichungsnummer:
**WO 94/20467 (15.09.1994 Gazette 1994/21)**

(54) **4-AMINOPYRIDINE, VERFAHREN ZU IHRER HERSTELLUNG SOWIE DIESE VERBINDUNGEN ENTHALTENDE ARZNEIMITTEL**

4-AMINOPYRIDINES, PROCESS FOR PREPARING THE SAME AND MEDICAMENTS CONTAINING THE SAME

4-AMINOPYRIDINES, LEUR PROCEDE DE PREPARATION ET MEDICAMENTS CONTENANT CES COMPOSES

(84) Benannte Vertragsstaaten:
**AT BE CH DE DK ES FR GB GR IE IT LI LU NL PT SE**

(30) Priorität: **03.03.1993 DE 4306506**
**21.04.1993 DE 4312966**

(43) Veröffentlichungstag der Anmeldung:
**20.12.1995 Patentblatt 1995/51**

(73) Patentinhaber: **BOEHRINGER MANNHEIM GMBH**
**68298 Mannheim (DE)**

(72) Erfinder:
- **VON DER SAAL, Wolfgang**
  **D-69469 Weinheim (DE)**
- **HECK, Reinhard**
  **D-67269 Grünstadt (DE)**
- **LEINERT, Herbert**
  **D-64646 Heppenheim (DE)**
- **POLL, Thomas**
  **D-68307 Mannheim (DE)**
- **STEGMEIER, Karlheinz**
  **D-64646 Heppenheim (DE)**
- **MICHEL, Helmut**
  **D-68307 Mannheim (DE)**

(56) Entgegenhaltungen:
**EP-A- 0 356 176**
**EP-A- 0 359 389**
**EP-A- 0 542 363**
**EP-A- 0 565 896**
**WO-A-93/09133**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 687 253 B1

## Beschreibung

Die Erfindung betrifft neue 4-Aminopyridine der allgemeinen Formel I

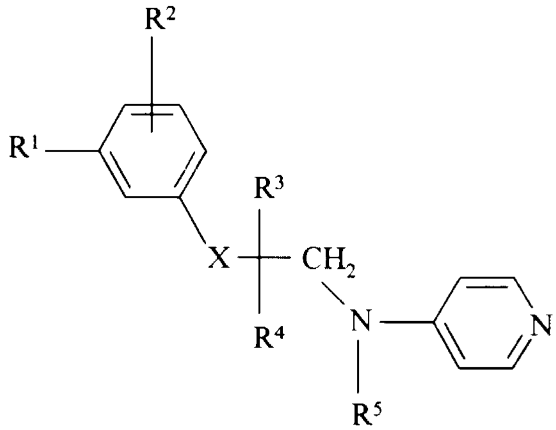

in der

$R^1$ die Gruppe $R^6$-SO-$NR^7$-, $R^6$-$SO_2$-$NR^7$-, $R^6$-$NR^7$-SO-, $R^6$-$NR^7$-$SO_2$-, $R^6$-SO-O-, $R^6$-$SO_2$-O-, $R^6$-O-SO- oder $R^6$-O-$SO_2$- bedeutet,

$R^2$ ein Wasserstoff- oder Halogenatom, eine Cyano-, $C_1$-$C_6$-Alkyl-, $C_1$-$C_6$-Alkoxy- oder Halogen-$C_1$-$C_6$-alkylgruppe bedeutet,

X ein Sauerstoffatom, ein Schwefelatom oder die NH-Gruppe bedeutet,

$R^3$ und $R^4$ gleich oder verschieden sind und Wasserstoffatome oder $C_1$-$C_6$-Alkylgruppen bedeuten,

$R^5$ ein Wasserstoffatom, eine $C_1$-$C_6$-Alkylgruppe oder die Aralkylgruppe bedeutet,

$R^6$ eine $C_1$-$C_6$-Alkyl-, $C_3$-$C_7$-Cycloalkyl-, Aryl-, Aralkyl- oder ein mono-, bi- oder tricyclisches aromatisches System mit Heteroatomen wie Stickstoff, Sauerstoff und Schwefel bedeutet, das mit einer $C_1$-$C_6$-Alkylgruppe verknüpft sein kann, das ebenso wie die Arylreste ein- oder mehrfach durch Nitro, Halogen, Nitril, Hydroxy, Amino, Carboxy, $C_1$-$C_6$-Alkoxycarbonyl, $C_2$-$C_6$-Alkenyloxycarbonyl, $C_2$-$C_6$-Alkinyloxycarbonyl, Aralkoxycarbonyl, $C_1$-$C_6$-Alkyl, $C_3$-$C_7$-Cycloalkyl, $C_2$-$C_6$-Alkenyl, $C_2$-$C_6$-Alkinyl, Cyan-$C_1$-$C_6$-alkyl, $C_1$-$C_6$-Alkoxy, $C_2$-$C_6$-Alkenyloxy, $C_2$-$C_6$-Alkinyloxy, Aralkoxy, Cyan-$C_1$-$C_6$-alkyloxy, $C_1$-$C_6$-Alkylthio, $C_1$-$C_6$-Alkylsulfinyl, $C_1$-$C_6$-Alkylsulfonyl, Amino, $C_1$-$C_6$-Alkylamino, Di-$C_1$-$C_6$-alkylamino, Aralkylamino, Di-aralkyl-amino, $C_1$-$C_6$-Alkylsulfonylamino, $C_1$-$C_6$-Alkylcarbonylamino, Formylamino, Aminocarbonyl, $C_1$-$C_6$-Alkylaminocarbonyl, Di-$C_1$-$C_6$-alkylaminocarbonyl oder durch eine oder mehrere der Gruppen -Y-$CO_2R^8$, -S-Y-$CO_2R^8$, -O-Y-$CO_2R^8$, -NH-Y-$CO_2R^8$, -S-Y-$CONR^8R^9$, -O-Y-$CONR^8R^9$ oder -NH-Y-$CONR^8R^9$ substituiert sein kann, wobei die Alkyl-, Alkenyl- oder Alkinylfragmente ein- oder mehrfach durch Halogen-, Hydroxy-, $C_1$-$C_6$-Alkoxy-, $C_1$-$C_6$-Alkylcarbonyloxy-, Amino- oder Carboxygruppen substituiert sein können,

$R^7$ ein Wasserstoffatom, einen $C_1$-$C_6$-Alkyl-, $C_3$-$C_7$-Cycloalkyl-, $C_2$-$C_6$-Alkenyl- oder $C_2$-$C_6$-Alkinylrest bedeutet, wobei diese Reste ein- oder merfach durch Halogen, Hydroxy, $C_1$-$C_6$-Alkoxy, Amino, $C_1$-$C_6$-Alkylamino, Di-$C_1$-$C_6$-alkylamino, Carboxy, $C_1$-$C_6$-Alkylcarbonyl oder $C_1$-$C_6$-Alkoxycarbonyl substituiert sein können, oder die $C_1$-$C_6$-Alkoxycarbonyl-, Cyan-$C_1$-$C_6$-alkyl-, Aryl-, Aralkyl- oder ein mono-, bi- oder tricyclisches aromatisches System mit Heteroatomen wie Stickstoff, Sauerstoff und Schwefel bedeutet, das mit einer $C_1$-$C_6$-Alkylgruppe verknüpft sein kann, das ebenso wie die Arylreste ein- oder mehrfach durch Halogen, Nitril, $C_1$-$C_6$-Alkyl, $C_2$-$C_6$-Alkenyl, $C_2$-$C_6$-Alkinyl, Halogen-$C_1$-$C_6$-alkyl, $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, Halogen- $C_1$-$C_6$-alkoxy, Hydroxy, Carboxy, Hydroxy-$C_1$-$C_6$-alkyl, Carboxy-$C_1$-$C_6$-alkyl, $C_1$-$C_6$-Alkoxycarbonyl, Amino,

$C_1$-$C_6$-Alkylamino, Di-$C_1$-$C_6$-alkylamino, $C_1$-$C_6$-Alkylsulfonylamino, $C_1$-$C_6$-Alkylcarbonylamino, Formylamino, Aminocarbonyl oder Phenyl substituiert sein kann, oder eine Gruppe -Y-$CO_2R^8$ oder -Y-$CONR^8R^9$ bedeutet,

Y eine lineare oder verzweigte $C_1$-$C_6$-Alkylenkette bedeutet,

$R^8$ und $R^9$ gleich oder verschieden sind und Wasserstoffatome, Aralkyl-, $C_3$-$C_7$-Cycloalkyl- oder $C_1$-$C_6$-Alkylgruppen bedeuten, die ein oder mehrfach durch Halogen, Hydroxy, $C_1$-$C_6$-Alkoxy, $C_1$-$C_6$-Alkylcarbonyloxy, Amin oder Carboxy substitiert sein können, oder $R^8$ und $R^9$ zusammen mit dem N-Atom, an das sie gebunden sind, einen gesättigten Ring bilden, der ein zusätzliches Sauerstoff, Schwefel- oder Stickstoffatome enthalten kann,

sowie Hydrate, Solvate und physiologisch verträgliche Salze davon. Gegenstand der Erfindung sind auch die optisch aktiven Formen, die Racemate und die Diastereomerengemische dieser Verbindungen.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln.

Die Aminopyridine der allgemeinen Formel I, ihre Solvate und ihre Salze hemmen sowohl die durch Thrombin induzierte Gerinnung von Fibrinogen im Blut als auch die durch Thrombin induzierte Aggregation der Blutplättchen. Sie verhindern damit die Entstehung von Gerinnungsthromben und von plättchenreichen Thromben und können bei der Bekämpfung und Verhütung von Krankheiten, wie Thrombose, Apoplexie, Herzinfarkt, Entzündungen und Arteriosklerose, verwendet werden. Ferner haben diese Verbindungen einen Effekt auf Tumorzellen und verhindern die Bildung von Metastasen. Somit können sie als Antitumormittel eingesetzt werden.

Thrombin, das letzte Enzym der Gerinnungskaskade, spaltet Fibrinogen zu Fibrin, das durch den Faktor XIIIa quervernetzt und zu einem unlöslichen Gel wird. das die Matrix für einen Thrombus bildet. Thrombin aktiviert durch Proteolyse seines Rezeptors auf den Blutplättchen die Plättchenaggregation und trägt auf diesem Weg ebenfalls zur Thrombusbildung bei. Bei der Verletzung eines Blutgefäßes sind diese Prozesse notwendig, um eine Blutung zu stoppen. Unter normalen Umständen sind keine meßbaren Thrombin-Konzentrationen im Blutplasma vorhanden. Ansteigen der Thrombinkonzentration kann zur Ausbildung von Thromben und damit zu thromboembolischen Krankheiten führen, die vor allem in den Industriestaaten sehr häufig auftreten.

Thrombin wird im Plasma in Form des Prothrombins bereitgehalten und durch den Faktor Xa aus diesem freigesetzt. Thrombin aktiviert die Faktoren V, VIII und XI, wodurch dann der Faktor X in Xa umgewandelt wird. Thrombin katalysiert dadurch seine eigene Freisetzung, weshalb es zu sehr rasch ansteigenden Thrombin-Konzentrationen kommen kann.

Thrombin-Inhibitoren können deshalb die Freisetzung des Thrombins. die plättcheninduzierte und die plasmatische Blutgerinnung hemmen.

Neben Thrombin existieren noch eine ganze Reihe von Serinproteasen, die Peptidsubstrate neben einer basischen Aminosäure spalten. Um Nebenwirkungen gering zu halten. sollten die Thrombininhibitoren selektiv sein, d. h. sie sollten andere Serinproteasen nur wenig oder garnicht hemmen. Besonders Trypsin als unspezifischste Serin-Protease kann von den verschiedensten Hemmern leicht gehemmt werden. Trypsinhemmung kann zu Pancreas-Stimulation und zu Pancreas-Hypertrophie führen (J.D.Geratz. Am. J. Physiol. 216, (1969) S. 812).

Plasma enthält das Protein Plasminogen, das durch Aktivatoren in Plasmin umgewandelt wird. Plasmin ist ein proteolytisches Enzym, dessen Aktivität der des Trypsins ähnelt. Es dient zur Auflösung der Thromben, indem es Fibrin abbaut. Hemmung des Plasmins hätte also gerade den gegenteiligen Effekt, den man mit der Hemmung des Thrombins erzielen möchte.

Synthetische Thrombin-Inhibitoren sind schon lange bekannt. Ausgehend vom Fibrinogen, dem natürlichen Substrat des Thrombins, wurden Substanzen des (D)-Phe-Pro-Arg-Typs synthetisiert. Solche Tripeptide ahmen die Aminosäuresequenz vor der Spaltstelle am Fibrinogen nach. Um gute Inhibitoren zu erhalten, wurde die Carboxylatgruppe des Arginins dabei so verändert, daß die Hydroxygruppe des Serin-195 der active site des Thrombins mit ihr reagieren kann. Dies ist beispielsweise dadurch möglich, daß man die Carboxylatgruppe durch die Aldehydfunktion ersetzt. Entsprechende (D)-Phe-Pro-Arginale sind in der Patentanmeldung EP-A-185390 beschrieben.

Zu einem zweiten Typ von Thrombin-Inhibitoren wurde das als Trypsin-Inhibitor bekannte Benzamidin zur Grundlage genommen. Die so erhaltenen Inhibitoren unterscheiden sich von den (D)-Phe-Pro-Arg-Typen nicht nur im chemischen Aufbau, sondern auch in der Art der Inhibierung: das Serin-195 des Thrombins bindet nicht an diese Inibitoren. Dies geht aus Röntgenstruktur-Unteruchungen eindeutig hervor (W. Bode. D. Turk, J. Stürzebecher, Eur. J. Biochem. 193, 175-182 (1990)). Zu dieser zweiten Klasse von Thrombin-Inhibitoren gehört das N$\alpha$-(2-Naphthylsulfonylglycyl)-4-amidino-(R,S)-phenylalanin-piperidid ("NAPAP", DD 235866).

Überraschend wurde nun gefunden, daß Verbindungen der allgemeinen Formel I, die keine strukturellen Gemeinsamkeiten mit den bekannten Thrombin-Inhibitoren aufweisen, selektive Thrombin-Inhibitoren sind.

Die in den Definitionen von $R^1$ - $R^9$ genannten Alkyl- oder Alkoxyfragmente enthalten 1-6 Kohlenstoffatome, wobei diese Fragmente geradkettig oder verzweigt sein können. Das gleich trifft zu für die entsprechenden Alkenyl- oder Alkinylfragmente. Cycloalkylgruppen sind Ringe mit drei bis sieben Kohlenstoffatomen. Im Falle einer Halogenalkyl- oder Halogenalkoxygruppe kann die Alkyl- oder Alkoxygruppe ein-, zwei- oder dreifach durch Halogen substituiert sein. Bevorzugt kommt im Fall der dreifach durch Halogen substituierten Gruppen die Trifluormethyl- oder Trifluormethoxygruppe in Frage. Halogen bedeutet in allen Fällen Fluor, Chlor, Brom oder Iod. Unter Aralkyl- und Aralkoxygruppen versteht man vorzugsweise die Benzyl- oder Benzyloxygruppe. In denjenigen Fällen, in denen die genannten Gruppen ein- oder mehrfach substituiert sein können, kommt insbesondere die ein-, zwei- oder dreifache Substitution in Frage. Im Fall der Sechsringe bei den Aryl- oder Hetarylgruppen können die Substituenten unabhängig voneinander in ortho-, meta- oder para-Stellung stehen.

Bedeutet in der allgemeinen Formel I einer der Substituenten $R^2$ - $R^9$ eine Alkylgruppe, oder bedeutet $R^6$ eine durch eine oder mehrere Alkylgruppen substituierte Aryl- oder Heteroarylgruppe, so sind unter den Alkylgruppen geradkettige oder verzweigte Alkylgruppen mit 1 bis 6 Kohlenstoffatomen zu verstehen, vorzugsweise die Methyl-, Ethyl-, Propyl-, Isopropyl-, Butyl-, Isobutyl-, tert.Butyl-, Pentyl- und die Hexylgruppe. Unter den Halogenatomen als Substituenten an den Alkylgruppen versteht man Fluor, Chlor, Brom oder Iod, bevorzugt Fluor und Chlor. Bevorzugt sind die Trifluormethyl, Chlormethyl, 2-Chlorethyl- und die 3-Chlorpropylgruppe. Sind die Alkylgruppen durch Hydroxygruppen substituiert, so sind bevorzugt die Hydroxymethyl, 2-Hydroxyethyl-, 3-Hydroxypropyl-, 1,2-Dihydroxyethyl- und die 2.3-Dihydroxypropylgruppe. Sind die Alkylgruppen durch Alkoxygruppen substituiert, so sind die Methoxymethyl-, Ethoxymethyl-, Methoxyethyl- und die Ethoxyethylgruppe bevorzugt. Sind die Alkylgruppen durch Aminogruppen substituiert, so sind die Aminomethyl-, 2-Aminoethyl-. 3-Aminopropyl-, 4-Aminobutyl- und die 5-Aminopentylgruppe bevorzugt. Sind die Alkylgruppen durch Carboxygruppen substituiert, so sind die Carboxymethyl-, 1-Carboxyethyl-, 2-Carboxyethyl- und die 2-Methyl-1-carboxyethylgruppe besonders bevorzugt.

Bedeutet in der allgemeinen Formel I $R^6$, $R^7$, $R^8$ oder $R^9$ eine Cycloalkylgruppe, oder bedeutet $R^6$ eine mit einer Cycloalkylgruppe substituierte Aryl- oder Heteroarylgruppe, so sind unter den Cycloalkvlgruppen Ringe mit 3 bis 7 Gliedern zu verstehen, bevorzugt die Cyclopropyl-, Cyclobutyl-, Cyclopentyl-, Cyclohexyl- und die Cycloheptylgruppe. Die Cycloalkylgruppe kann in allen Fällen auch über eine Alkylgruppe verknüpft sein. so daß eine Cycloalkyl-alkylgruppe als Substituent resultiert. Besonders bevorzugt sind dabei die Cyclopropylmethyl- und die Cyclohexylmethylgruppe.

Bedeutet in der allgemeinen Formel $R^6$ eine mit einem Alkenylrest substituierte Aryl- oder Heteroarylgruppe, oder bedeutet $R^7$ einen Alkenylrest, so sind darunter geradkettige oder verzweigte Reste mit 3 bis 6 Gliedern zu verstehen, vorzugsweise der Allyl-. Butenyl- und iso-Butenylrest.

Bedeutet in der allgemeinen Formel $R^6$ eine mit einem Alkinylrest substituierte Aryl- oder Heteroarylgruppe, oder bedeutet $R^7$ einen Alkinylrest, so sind darunter geradkettige oder verzweigte Reste mit 3 bis 6 Gliedern zu verstehen, vorzugsweise der Propargylrest.

Bedeutet in der allgemeinen Formel I $R^6$ eine mit einem Alkoxycarbonyl-, Alkenyloxycarbonyl- oder Alkinyloxycarbonylrest substituierte Aryl- oder Heteroarylgruppe, so sind darunter geradkettige oder verzweigte Reste mit 2 bis 6 Kohlenstoffatomen zu verstehen, vorzugsweise die Methoxycarbonyl-, Ethoxycarbonyl- und Allyloxycarbonylgruppe.

Bedeutet in der allgemeinen Formel I $R^6$ eine mit einem Alkoxyrest substituierte Aryl- oder Heteroarylgruppe, so sind darunter geradkettige oder verzweigte Reste mit 1 bis 6 Kohlenstoffatomen zu verstehen, vorzugsweise die Methoxy-, Ethoxy-, Propyloxy-, Butyloxy- und die Pentyloxygruppe. Sind die Alkoxygruppen durch Hydroxygruppen substituiert, so sind bevorzugt die 2-Hydroxyethoxy-, 3-Hydroxypropyloxy-, und die 2,3-Dihydroxypropyloxygruppe. Sind die Alkoxygruppen durch Alkoxygruppen substituiert, so sind die Methoxyethoxy- und die Ethoxyethoxygruppe bevorzugt. Sind die Alkoxygruppen durch Aminogruppen substituiert, so sind die 2-Aminoethoxy- und die 3-Aminopropyloxygruppe bevorzugt.

Bedeutet in der allgemeinen Formel $R^6$ eine mit einem Alkenyloxyrest substituierte Aryl- oder Heteroarylgruppe, so sind darunter geradkettige oder verzweigte Reste mit 3 bis 6 Kohlenstoffatomen zu verstehen, vorzugsweise die Allyloxygruppe.

Bedeutet in der allgemeinen Formel I $R^6$ eine mit einem Alkinyloxyrest substituierte Aryl- oder Heteroarylgruppe, so sind darunter geradkettige oder verzweigte Reste mit 1 bis 6 Kohlenstoffatomen zu verstehen, vorzugsweise die Propargyloxygruppe.

Bedeutet in der allgemeinen Formel I $R^6$ eine mit einem Alkylthio-, Alkylsulfinyl- oder Alkylsulfonylrest substituierte Aryl- oder Heteroarylgruppe, so sind darunter geradkettige oder verzweigte Reste mit 1 bis 6 Kohlenstoffatomen zu verstehen, vorzugsweise die Methylthio, Methylsulfinyl- und die Methylsulfonylgruppe.

Bedeutet in der allgemeinen Formel I $R^6$ eine mit einem Alkylamino- oder Dialkylaminorest substituierte Aryl- oder Heteroarylgruppe, so sind darunter geradkettige oder verzweigte Reste mit 1 bis 6 Kohlenstoffatomen zu verstehen, vorzugsweise die Methylamino, Dimethylamino- und die Diethylaminogruppe.

Bedeutet in der allgemeinen Formel I $R^6$ eine mit einem Alkylsulfonylaminorest substituierte Aryl- oder Heteroarylgruppe, so sind darunter geradkettige oder verzweigte Reste mit 1 bis 6 Kohlenstoffatomen zu verstehen, vorzugsweise die Methylsulfonylaminogruppe.

Bedeutet in der allgemeinen Formel I $R^6$ eine mit einem Alkylcarbonylaminorest substituierte Aryl- oder Heteroarylgruppe, so sind darunter geradkettige oder verzweigte Reste mit l bis 6 Kohlenstoffatomen zu verstehen, vorzugsweise die Acetylaminogruppe.

Bedeutet in der allgemeinen Formel I $R^6$ eine mit einem Alkylaminocarbonyl- oder Dialkylaminocarbonylrest substituierte Aryl- oder Heteroarylgruppe, so sind darunter geradkettige oder verzweigte Reste mit 1 bis 6 Kohlenstoffatomen zu verstehen. vorzugsweise die Methylaminocarbonyl-, Dimethylaminocarbonyl- und die Diethylaminocarbonylgruppe.

Unter den Aralkylgruppen für $R^6$ oder $R^7$ ist die Benzylgruppe besonders bevorzugt.

Als Arylrest $R^6$ oder $R^7$, allein oder in Verbindung mit einer Alkylkette, sind aromatische Kohlenwasserstoffe mit 6-14 C-Atomen, insbesondere der Phenyl-, der Biphenyl-, der Naphthyl-, Tetrahydronaphthyl-, Indanyl- oder der Fluorenylrest zu verstehen.

Als Heteroarylrest für $R^6$ sind mono-. bi- und tricyclische Aromaten mit Heteroatomen wie Stickstoff, Sauerstoff und Schwefel zu verstehen, beispielsweise Furan, Thiophen, Pyrrol, Oxazol, Isoxazol, Thiazol, Isothiazol, Imidazol, Pyrazol, Triazol, Tetrazol, Pyridin, Pyrazin, Pyrimidin, Pyridazin, Triazin, Tetrazin, Benzothiophen, Dibenzothiophen, Benzimidazol, Carbazol, Benzofuran, Benzofurazan, Benzo-2,1,3-thiadiazol, Chinolin, Isochinolin, Chinazolin.

Unter der Alkylengruppe Y in der allgemeinen Formel I versteht man lineare oder verzweigte Kohlenstoffketten mit 1 bis 6 Kohlenstoffatomen, vorzugsweise die Methylen-, Ethylen- oder Propylengruppe.

Bilden die Substituenten $R^8$ und $R^9$ zusammen mit dem Stickstoffatom an das sie gebunden sind, einen Ring, so sind darunter Ringe mit 4 bis 7 Glieder zu verstehen, besonders der Pyrrolidin-, der Piperidin- und der Homopiperidinring. Enthält dieser Cyclus zusätzliche Heteroatome, so sind darunter der Morpholin, Thiomorpholin und der Piperazinring bevorzugt.

Der Rest $R^2$ am Phenylring der allgemeinen Formel I kann in beliebigen Positionen zum Fragment X (Sauerstoffatom oder NH-Gruppe) stehen. Besonders bevorzugt ist jedoch eine Anordnung, in der alle drei Substituenten des Phenylringes der allgemeinen Formel I in meta-Stellung zueinander stehen.

$R^1$ bedeutet insbesondere die Gruppen $R^6$-$SO_2$-$NR^7$-, $R^6$-$NR^7$-$SO_2$-, $R^6$-$SO_2$-O-, oder $R^6$-O-$SO_2$-.

$R^2$ bedeutet insbesondere ein Wasserstoff-, Chlor- oder Bromatom, oder eine $C_1$-$C_6$-Alkylgruppe, wie z.B. eine Methyl- oder Ethylgruppe-, oder eine $C_1$-$C_6$-Alkoxygruppe, wie z.B. die Methoxygruppe oder die Trifluormethylgruppe.

X ist insbesondere ein Sauerstoffatom oder die NH-Gruppe.

$R^3$ und $R^4$ können gleich oder verschieden sein und stellen bevorzugt Wasserstoffatome oder $C_1$-$C_6$-Alkylgruppen, insbesondere Wasserstoffatome oder die Methylgruppe dar.

$R^5$ ist insbesondere ein Wasserstoffatom, eine $C_1$-$C_6$-Alkylgruppe (wie z.B. eine Methylgruppe) oder die Benzylgruppe.

$R^6$ ist insbesondere eine $C_1$-$C_6$-Alkylgruppe (wie z.B. die Isopropylgruppe), eine $C_3$-$C_7$-Cycloalkylgruppe (wie z. B. die Cyclopentyl- oder Cyclohexylgruppe), eine unsubstituierte oder ein- oder mehrfach durch Fluor, Chlor, $C_1$-$C_6$-Alkyl (wie z.B. Methyl. Ethyl, tert.Butyl), $C_1$-$C_6$-Alkoxy (wie z.B. Methoxy), Nitro, Amino, Hydroxy, Carboxy. Benzyloxycarbonyl, $C_1$-$C_6$-Alkoxycarbonly (wie z.B. Methoxycarbonyl), Trifluormethyl oder die Gruppe -O-Y-$CO_2R^8$ substituierte Phenylgruppe; eine Naphthyl-, Tetrahydronaphthyl-, Biphenyl- oder Indanylgruppe, eine Thienyl-, Pyrazolyl- oder Pyridylgruppe, eine Benzthienyl oder Benzothiadiazinylgruppe oder die Benzylgruppe.

$R^7$ ist insbesondere ein Wasserstoffatom, eine $C_1$-$C_6$-Alkyl- oder $C_2$-$C_6$-Alkenylgruppe (wie z.B. eine Methyl-, Ethyl-, n-Propyl-, Allyl-, i-Propylgruppe) oder eine Aralkylgruppe (wie z.B. die Benzylgruppe), eine $C_1$-$C_6$-Alkoxycarbonylgruppe ( wie z.B. die Ethoxycarbonylgruppe), eine Cyanoalkylgruppe (wie z.B. die Cyanomethylgruppe), eine Hydroxyalkylgruppe (wie z. B. die Hydroxyethyl- oder Dihydroxypropylgruppe), oder eine Aminoalkylgruppe (wie z.B. die Aminoethylgruppe), eine Gruppe -Y-$COR^8$ oder eine Gruppe -Y-$CONR^8R^9$.

Y ist insbesondere eine Methylen-, Propylen, Butylen oder Pentylengruppe.

$R^8$ ist insbesondere ein Wasserstoffatom oder eine Alkylgruppe (wie z.B. die Methyl- oder Ethylgruppe), eine Hydroxyalkylgruppe (wie z.B. die Hydroxyethyl-, Hydroxypropyl- oder Dihydroxypropylgruppe) oder eine Aminoalkylgruppe (wie z.B. die Aminoethylgruppe).

$R^9$ ist insbesondere ein Wasserstoffatom oder eine Alkylgruppe (wie z.B. die Methylgruppe).

Bevorzugt sind Verbindungen der allgemeinen Formel I,

(I) 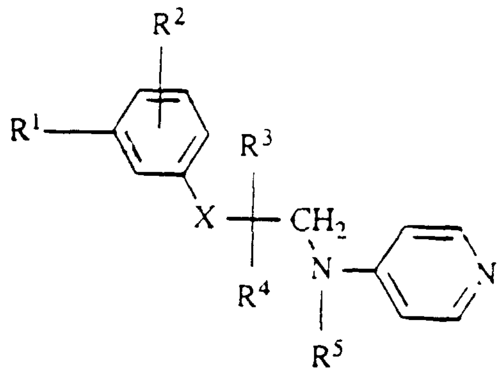

in denen

- $R^1$ die Gruppen $R^6$-$SO_2$-$NR^7$-, $R^6$-$NR^7$-$SO_2$-, $R^6$-$SO_2$-O-, oder $R^6$-O-$SO_2$- bedeutet,
- $R^2$ ein Wasserstoff-, Chlor- oder Bromatom, eine Methyl-, Ethyl-, Methoxy- oder die Trifluormethylgruppe bedeutet,
- X ein Sauerstoffatom oder die NH-Gruppe bedeutet,
- $R^3$ und $R^4$ gleich oder verschieden sind und Wasserstoffatome oder Methylgruppen bedeuten,
- $R^5$ ein Wasserstoffatom, eine Methyl- oder Benzylgruppe bedeutet,
- $R^6$ eine Isopropyl-, Cyclopentyl- oder Cyclohexylgruppe, eine unsubstituierte oder ein- oder mehrfach durch Fluor, Chlor, Methyl, Ethyl, tert.Butyl, Methoxy, Nitro, Amino, Hydroxy, Carboxy, Benzyloxycarbonyl, Methoxycarbonyl, Trifluormethyl oder die Gruppe -O-Y-$CO_2R^8$ substituierte Phenylgruppe, eine Naphthyl-, Tetrahydronaphthyl-, Biphenyl-, oder Indanylgruppe, eine Thienyl-, Pyrazolyl- oder Pyridylgruppe, eine Benzthienyl oder Benzothidiazinylgruppe oder die Benzylgruppe bedeutet,
- $R^7$ ein Wasserstoffatom, einen Methyl-, Ethyl-, n-Propyl-, Allyl-, i-Propyl- oder eine Benzylgruppe, eine Ethoxycarbonyl-, Hydroxyethyl-, Dihydroxypropyl-, Cyanomethyl- oder Aminoethylgruppe, eine Gruppe -Y-$COR^8$ oder eine Gruppe -Y-$CONR^8R^9$ bedeutet,
- Y eine Methylen-, Propylen, Butylen oder Pentylengruppe bedeutet,
- $R^8$ ein Wasserstoffatom oder eine Methyl-, Ethyl-, Hydroxyethyl-, Hydroxypropyl-, Dihydroxypropyl- oder Aminoethylgruppe bedeutet,
- $R^9$ ein Wasserstoffatom oder eine Methylgruppe bedeutet,

Die Herstellung von Verbindungen der allgemeinen Formel I geschieht nach an sich bekannten Verfahren.
Man geht aus von den Verbindungen der allgemeinen Formel II,

(II)

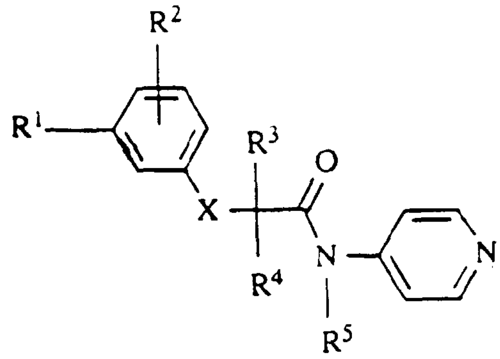

die man nach gängigen Verfahren reduziert. Als Reduktionsmittel kommen in Betracht komplexe Bor- und Aluminiumhydride, Borwasserstoffkomplexe, Aluminiumhydrid, das man in situ durch Umsetzung von $LiAlH_4$ mit $AlCl_3$ oder $H_2SO_4$ herstellt oder ein Gemisch aus $AlCl_3$ und $NaBH_4$.

Die Verbindungen der allgemeinen Formel II stellt man her durch Umsetzung von Verbindungen der allgemeinen Formel III

(III)

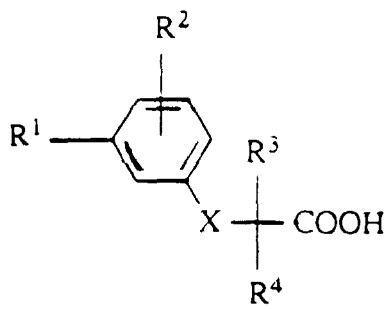

mit 4-Aminopyridin, dessen Amino-N-Atom den Rest $R^5$ trägt. Diese Umsetzung geschieht durch Reaktion von äquimolaren Mengen des 4-Aminopyridins und der Carbonsäure der allgemeinen Formel III in Gegenwart eines wasserentziehenden Mittels wie Polyphosphorsäure, einem sauren Kationenaustauscher, Schwefelsäurehalogenid, 2-Halogenpyridiniumsalz, Dicyclohexylcarbodiimid oder N,N'-Carbonyldiimidazol. Man kann diese Reaktion auch in zwei Stufen ablaufen lassen, wobei man die Carbonsäure zuerst in ein reaktionsfähiges Derivat. z.B. ein Säurechlorid. ein Säureazid oder Imidazolid umwandelt und dann mit dem 4-Aminopyridin zur Reaktion bringt.

Die Carbonsäuren der allgemeinen Formel III stellt man aus den Estern der allgemeinen Formel IV her,

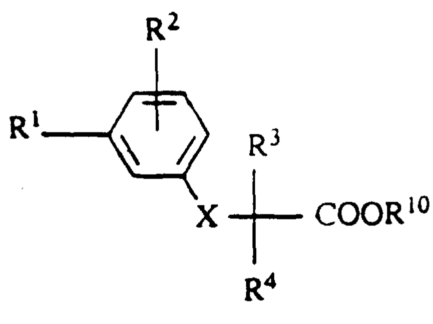

(IV)

in der $R^{10}$ eine Alkyl- oder Benzylgruppe bedeutet. Je nach Art dieser Gruppe erfolgt die Reaktion entweder mit Hilfe von Basen oder Säuren oder hydrogenolytisch. Ist $R^{10}$ eine Methyl- oder Ethylgruppe, so erfolgt die Umsetzung bevorzugt mit Natronlauge oder Kalilauge in Methanol, Ethanol oder in Wasser. Ist $R^{10}$ eine tert.Butylgruppe, so erfolgt die Reaktion mit einer Säure, vorzugsweise Salzsäure, Ameisensäure oder Trifluoressigsäure. Ist $R^{10}$ eine Benzylgruppe, so erfolgt die Reaktion bevorzugt hydrogenolytisch in Gegenwart eines Katalysators wie Palladium auf Kohle

oder mit Platin.

Aus Verbindungen der allgemeinen Formel IV. in der $R^1$ die Gruppe $R^6$-NH-SO-, $R^6$-NH-$SO_2$-, $R^6$-SO-NH- oder $R^6$-$SO_2$-NH- bedeutet, kann man durch Alkylierung eine andere Verbindung der allgemeinen Formel IV herstellen, in der $R^1$ die Gruppe $R^6$-$NR^{7'}$-SO-, $R^6$-$NR^{7'}$-$SO_2$-, $R^6$-SO-$NR^{7'}$- oder $R^6$-$SO_2$-$NR^{7'}$- bedeutet. Als Alkylierungsmittel verwendet man Verbindungen der allgemeinen Formel $R^{7'}$-Z, wobei $R^{7'}$ die gleiche Bedeutung wie $R^7$ mit der Ausnahme des Wasserstoffatoms. der Phenyl- und Heteroarylgruppe hat und Z eine reaktive Gruppe wie Halogen, vorzugsweise Brom, Chlor oder ein Sulfat bedeutet. Diese Reaktionen werden vorzugsweise in einem Lösungsmittel wie Aceton. Ether, Toluol oder Dimethylformamid bei Temperaturen zwischen -30°C und 100°C, vorzugsweise bei Raumtemperatur in Gegenwart einer Base wie Natriumhydrid oder Calciumcarbonat durchgeführt.

Die Verbindungen der allgemeinen Formel IV stellt man aus den Verbindungen der allgemeinen Formel V her,

(V)

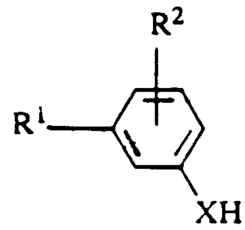

die man mit α-Halogenestern der allgemeinen Formel Hal-$CR^3R^4$-$CO_2R^{10}$ umsetzt. Unter Hal ist ein Halogenatom zu verstehen, vorzugsweise Chlor und Brom. Diese Reaktionen werden vorzugsweise in einem Lösungsmittel wie Aceton, Ether, Toluol oder Dimethylformamid bei Temperaturen zwischen -30°C und 100°C, vorzugsweise bei Raumtemperatur in Gegenwart einer Base wie Natriumhydrid oder Calciumcarbonat durchgeführt.

Die Verbindungen der allgemeinen Formel IV, in der $R_1$ die Gruppe $R^6$-SO-O-, $R^6$-$SO_2$-O-, $R^6$-SO_NH- oder $R^6$-$SO_2$-NH- bedeutet, stellt man aus den Verbindungen der allgemeinen Formel VI her,

(VI)

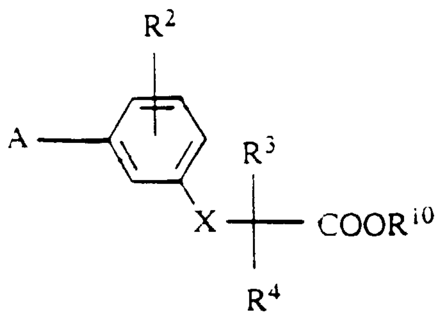

indem man sie mit einem Sulfinychlorid $R^6$-SOCl bzw. Sulfonylchlorid $R^6$-$SO_2$Cl umsetzt. A bedeutet dabei eine Hydroxy- oder Aminogruppe $NHR^7$. Die Umsetzung erfolgt zweckmäßig unter Zusatz eines säurebindenden Mittels, wie z.B. Alkaliacetat, Alkalihydroxid, Calciumoxid, Calciumcarbonat. Magnesiumcarbonat oder mit organischen Basen wie Pyridin, Triethylamin, N-Methylmorpholin oder Di-isopropylmethylamin, wobei als inerte Lösungsmittel z.B. Ether, Methylenchlorid, Dioxan, Toluol oder ein Überschuß des tertiären Amins dienen. Beim Einsatz anorganischer Säurebinder verwendet man als Reaktionsmedium z.B. Wasser, wäßriges Ethanol oder wäßriges Dioxan.

Die Verbindungen der allgemeinen Formel IV, in der $R_1$ die Gruppe $R^6$-O-SO-, $R^6$-O-$SO_2$-, $R^6$-$NR^7$-SO- oder $R^6$-$NR^7$-$SO_2$- bedeutet, kann man aus den Verbindungen der allgemeinen Formel VII herstellen,

(VII)

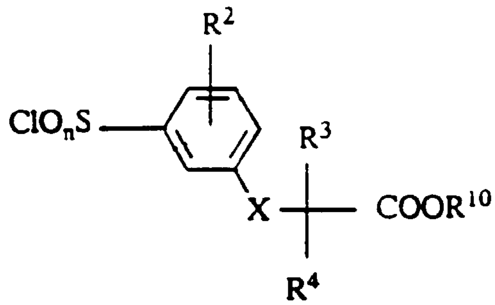

in der n gleich eins (Sulfinylchloride) oder gleich 2 (Sulfonylchloride) bedeutet. Man setzt mit Verbindungen der allgemeinen Formel $R^6$-OH bzw. $R^6$-NH-$R^7$ um. Die Umsetzung erfolgt zweckmäßig unter Zusatz eines säurebindenden Mittels, wie z.B. Alkaliacetat, Alkalihydroxid, Calciumoxid, Calciumcarbonat, Magnesiumcarbonat oder mit organischen Basen wie Pyridin, Triethylamin, N-Methylmorpholin oder Di-isopropylmethylamin, wobei als inerte Lösungsmittel z.B. Ether, Methylenchlorid, Dioxan. Toluol oder ein Überschuß des tertiären Amins dienen. Beim Einsatz anorganischer Säurebinder verwendet man als Reaktionsmedium z.B. Wasser, waßriges Ethanol oder waßriges Dioxan.

Die Verbindungen der allgemeinen Formel V und VII sind literaturbekannt (Methoden der Organischen Chemie (Houben-Weyl), Thieme Verlag, Stuttgart 1955: S. 285: M. Quaedvlieg, Aliphatische Sulfinsäuren; S. 299: F. Muth. Aromatische Sulfinsäuren; S. 343: M. Quaedvlieg, Aliphatische Sulfonsäuren; S.429: F. Muth. Aromatische Sulfonsauren; S. 599: F. Muth, Funktionelle N-Derivate der Arylsulfonsäuren; S. 659: F. Muth. Aromatische Sulfonsäureester) oder können nach den dort beschriebenen Methoden hergestellt werden. Die Verbindungen der allgemeinen Formel VI sind Literaturbekannt (Sobotka. Austin, J. Am. Chem. Soc. 74. 3813 (1952)) oder können nach den dort beschriebenen Methoden hergestellt werden.

Eine weitere Methode zur Herstellung von Verbindungen der allgemeinen Formel I besteht in der Umsetzung von Verbindungen der allgemeinen Formel VIII

(VIII)

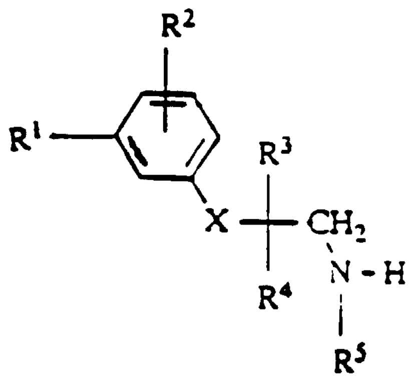

mit einem Pyridin-Derivat, das in Position 4 eine nucleofuge Abgangsgruppe aufweist. Als solche Abgangsgruppen kommen in Frage Halogene, vorzugsweise Brom, Chlor und Fluor, sowie Nitro-, Alkoxy- und Phenoxygruppen. Zur Erleichterung der Reaktion kann das 4-Aminopyridinderivat weitere Halogenatome, vorzugsweise Chlor enthalten. Bevorzugte Derivate sind Pentachlorpyridin und 4-Nitro-tetrachlorpyridin. Diese Reaktion führt man vorzugsweise in einem inertem Lösungsmittel, wie z.B. Toluol, Dioxan, Dimethylformamid, Dimethylacetamid, Methylenchlorid oder Ethanol bei Temperaturen zwischen Raumtemperatur und Siedetemperatur des Lösungsmittels durch, vorzugsweise zwischen 20 und 40°C. Enthält das Pyridinderivat weitere Chloratome. so schließt sich an die nucleophile Reaktion eine Enthalogenierungsreaktion an, z.B. durch katalytische Hydrierung.

Die Verbindungen der allgemeinen Formel VIII stellt man durch Reduktion der Verbindungen der allgemeinen Formel IX her,

(IX)

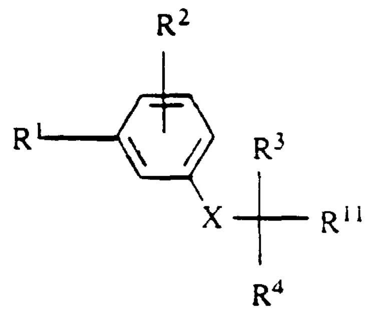

in der $R^{11}$ eine Nitrilgruppe oder eine Amidgruppe $CONHR^5$ bedeutet. Als Reduktionsmittel kommen in Betracht komplexe Bor- und Aluminiumhydride, Borwasserstoffkomplexe. Aluminiumhydrid, das man in situ durch Umsetzung von $LiAlH_4$ mit $AlCl_3$ oder Schwefelsäure herstellt, oder ein Gemisch aus $AlCl_3$ und $NaBH_4$.

Die Verbindungen der allgemeinen Formel IX stellt man aus den Verbindungen der allgemeinen Formel III her. Diese Umsetzung geschieht durch Reaktion von äquimolaren Mengen eines Amins $H_2NR^5$ und der Carbonsäure der allgemeinen Formel III in Gegenwart eines wasserentziehenden Mittels wie Polyphosphorsäure, einem sauren Kationenaustauscher, Schwefelsäurehalogenid, 2-Halogenpyridiniumsalz, Dicyclohexylcarbodiimid oder N,N'-Carbonyldiimidazol. Man kann diese Reaktion auch in zwei Stufen ablaufen lassen, wobei man die Carbonsäure zuerst in ein reaktionsfähiges Derivat. z.B. ein Säurechlorid oder ein Säureazid umwandelt und dann mit dem Ammoniak zur Reaktion bringt.

Ein weiteres Verfahren zur Herstellung von Verbindungen der allgemeinen Formel I geht aus von den Verbindungen der allgemeinen Formel X

(X)

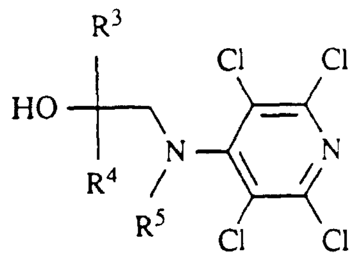

die man nach literaturbekannten Verfahren (M. M. Boudakian. in Heterocyclic Compounds. Vol 14. Suppl. Part. 2, (R. A. Abramovitch, Ed.), Wiley, New York 1974, Seite 407.) durch Umsetzung der käuflichen Aminoethanole $HO-CR^3R^4-CH_2-NHR^5$ mit Pentachlorpyridin oder 4-Nitrotetrachlorpyridin in einem inerten Lösungsmittel wie Dioxan, Tetrahydrofuran, Methylenchlorid oder Ethanol bei Temperaturen zwischen -10°C und der Siedetemperatur des Lösungsmittels erhält. Die Hydroxygruppe der Verbindungen der allgemeinen Formel X wandelt man in eine Abgangsgruppe W um und erhält dadurch die Verbindungen der allgemeinen Formel XI

(XI)

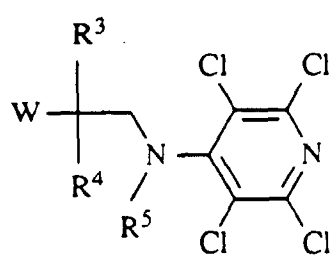

in der W ein Halogenatom wie Chlor oder Brom oder ein Sulfonsäureester wie Tosyloxy bedeutet. Die Umwandlung

der Hydroxygruppe in ein Halogenatom erfolgt mit einem Halogenierungsmittel wie Thionylchlorid oder Phosphorylchlorid, die Umwandlung in einen Sulfonsäureester durch Umsetzung mit einem Sulfonylchlorid wie Tosylchlorid.

Die Verbindungen der allgemeinen Formel XI setzt man nun mit Verbindungen der allgemeinen Formel V' um

(V')

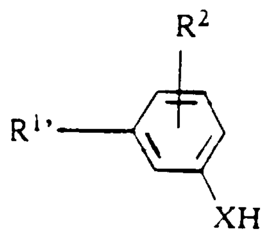

in der $R^{1'}$ die gleiche Bedeutung wie $R^1$ hat. zusätzlich aber auch eine geschützte Hydroxygruppe oder Aminogruppe sein kann. Unter der geschützten Hydroxygruppe versteht man die Benzyloxygruppe oder die Acetyloxygruppe. Unter der geschützten Aminogruppe versteht man bevorzugt die tert.Butyloxycarbamoylgruppe, die Benzyloxycarbamoylgruppe, die Dibenzylaminogruppe oder die Phthalimidogruppe. Dabei entstehen die Verbindungen der allgemeinen Formel XII. Die Umsetzung von Verbindungen der allgemeinen Formel V' mit Verbindungen der allgemeinen Formel X an Stelle von XI nach Mitsunobu in Gegenwart von Triphenylphosphin und Diazodicarbonsäurediethylester oder -piperidid führt ebenfalls zu Verbindungen der allgemeinen Formel XII.

(XII)

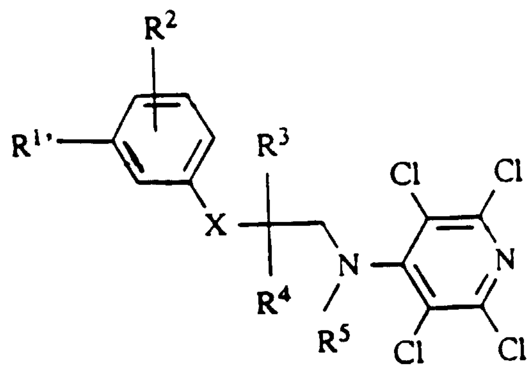

Ist $R^{1'}$ in den Verbindungen der allgemeinen Formel XII eine geschützte Hydroxygruppe oder eine geschützte Aminogruppe, so wird nun im nächsten Schritt die Schutzgruppe entfernt. Dies geschieht für die Benzylschutzgruppen durch Hydrogenolyse in Gegenwart eines Katalysators wie Palladium auf Kohle, für die tert.Butylcarbamoylgruppe durch eine starke Säure wie Trifluoressigsäure und für die Acetylgruppe durch eine Base wie Natronlauge. Dabei entstehen Verbindungen der allgemeinen Formel XIII,

(XIII)

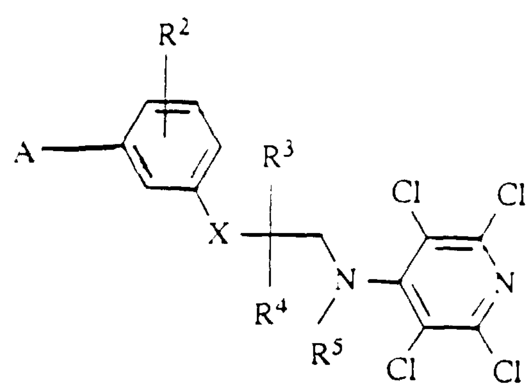

die man durch Umsetzung mit Sulfinylchloriden oder Sulfonylchloriden in die Verbindungen der allgemeinen Formel XIV überführt,

(XIV)

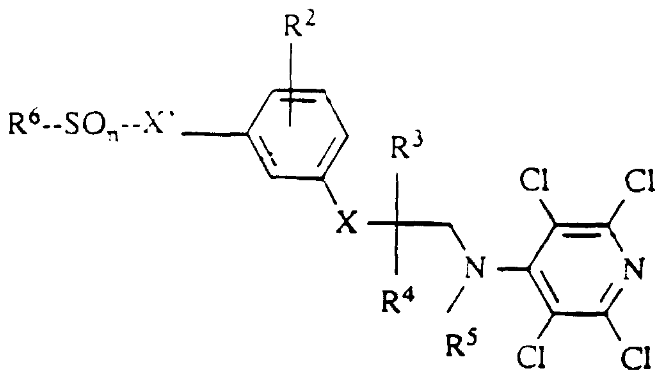

in der n = 1 oder 2 sein kann und X' das Sauerstoffatom oder die Iminogruppe NH bedeutet. Bedeutet X eine Iminogruppe, so werden Verbindungen der allgemeinen Formel XIV nun umgewandelt in die Verbindungen der allgemeinen Formel XV,

(XV)

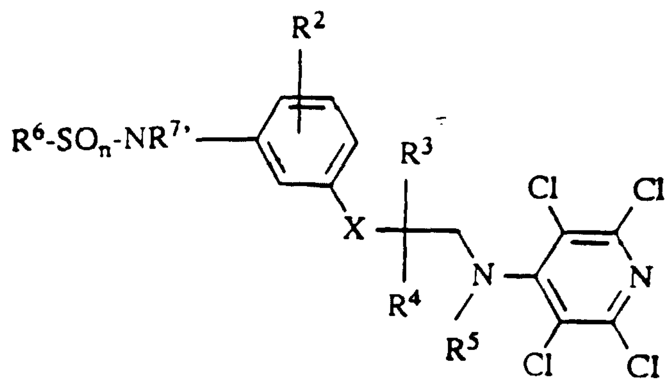

in der $R^{7'}$ die gleiche Bedeutung wie $R^7$ hat mit der Ausnahme des Wasserstoffatoms. Dies geschieht durch Umsetzung mit den Alkylierungsmitteln $R^{7'}$-Y, wie es bei Alkylierungen der allgemeinen Formel IV beschrieben ist.

Die Verbindungen der allgemeinen Formel I erhält man schließlich aus den Verbindungen der allgemeinen Formel XII, in der $R^{1'}$ die gleiche Bedeutung wie $R^1$ hat, aus den Verbindungen der allgemeinen Formel XIV, oder aus den Verbindungen der allgemeinen Formel XV durch Entfernung der Chloratome des Pyridinringes. Dies geschieht durch katalytische Hydrierung in Gegenwart eines Katalysators wie Raney-Nickel oder Palladium auf Kohle in Gegenwart einer Base wie Kaliumcarbonat, Natriumhydrogencarbonat oder Natriummethylat.

Zur Herstellung von Verbindungen der allgemeinen Formel I, in der $R^1$ die Gruppe $R^6$-SO-$NR^7$-, $R^6$-$SO_2$-$NR^7$-, $R^6$-SO-O- oder $R^6$-$SO_2$-O- bedeutet, besteht ein weiterer Syntheseweg in der Umsetzung von Verbindungen der allgemeinen Formel XVI

(XVI)

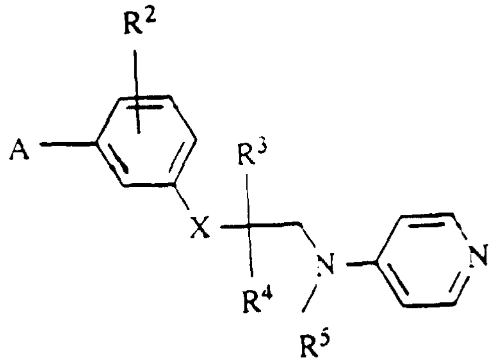

mit einem Sulfinylchlorid $R^6$-SOCl bzw. einem Sulfonylchlorid $R^6$-$SO_2Cl$. Die Umsetzung erfolgt wie für die Umsetzung mit Verbindungen der allgemeinen Formel VI beschrieben. A bedeutet dabei die Hydroxygruppe oder eine Aminogruppe $NHR^7$.

Verbindungen der allgemeinen Formel XVI stellt man aus den Verbindungen der allgemeinen Formel XVII her,

(XVII)

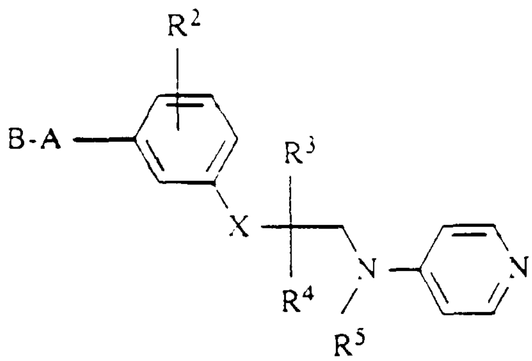

in der B eine Schutzgruppe bedeutet. die zur Herstellung von Verbindungen der allgemeinen Formel XVI abgespalten wird. Als Schutzgruppen B kommen in Frage die Benzylgruppe, die man hydrogenolytisch in Gegenwart eines Katalysators wie Palladium auf Kohle entfernt, die tert.Butyloxycarbonylgruppe, die man durch die Einwirkung von Säuren wie Trifluoressigsäure. Ameisensäure oder Salzsäure entfernt, oder eine aromatische Sulfonylgruppe wie die Benzolsulfonyl oder Tosylgruppe, die man durch Einwirkung von Alkali wie Natronlauge oder Kalilauge, entfernt.

Verbindungen der allgemeinen Formel XVII stellt man nach den gleichen Prinzipien her wie Verbindungen der allgemeinen Formel I. Vorzugsweise geht man aus von Verbindungen der allgemeinen Formal XVIII,

(XVIII)

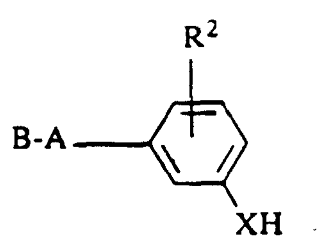

die man mit Halogenestern der allgemeinen Formel Hal-$CR^3R^4$-$CO_2R^{10}$ umsetzt, wie das für Umsetzungen mit Verbindungen der allgemeinen Formel V beschrieben ist. Dabei entstehen Verbindungen der allgemeinen Formel XIX,

(XIX)

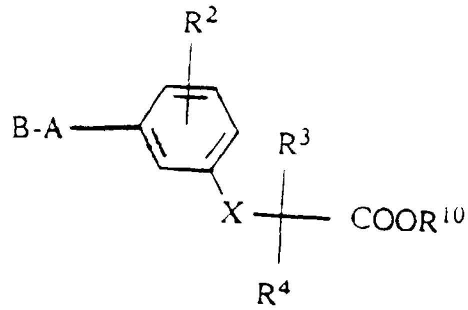

die man nach Verseifung des Esters und Aktivierung der Säurefunktion, wie bei den Verbindungen der allgemeinen Formel III beschrieben, mit 4-Aminopyridin oder N-$R^5$-4-Aminopyridin zu den Verbindungen der allgemeinen Formel XVI umsetzt.

Gewisse Verbindungen der allgemeinen Formel I lassen sich nachträglich in andere Verbindungen der allgemeinen Formel I umwandeln.

Dies betrifft Verbindungen der allgemeinen Formel I, in der die Gruppe $R^5$, $R^6$ oder $R^7$ die Benzylgruppe bedeutet, oder in der $R^6$ eine Aryl- oder Heteroarylgruppe bedeutet, die als Substituenten eine odere mehrere Benzyloxy-, Benzylamino- oder Benzyloxycarbonylgruppen tragen. Durch katalytische Hydrierung in Gegenwart eines Katalysators. vorzugsweise Palladium auf Kohle, wird die Benzylgruppe dabei durch das Wasserstoffatom ersetzt, Die Entfernung der Benzylgruppe gelingt auch durch Umsetzung mit einer starken Säure wie Trifluoressigsäure in Gegenwart von Mesitylen, Anisol oder Thioanisol.

Dies betrifft auch Verbindungen der allgemeinen Formel I, in denen $R^6$ eine Aryl- oder Heteroarylgruppe bedeutet, die als Substituenten eine odere mehrere Chloratome tragen. Durch katalytische Hydrierung in Gegenwart eines Katalysators, vorzugsweise Palladium auf Kohle, wird das Chloratom dabei durch das Wasserstoffatom ersetzt.

Dies betrifft auch Verbindungen der allgemeinen Formel I, in denen $R^6$ eine Aryl- oder Heteroarylgruppe bedeutet, die als Substituenten eine odere mehrere Nitrogruppen tragen. Durch katalytische Hydrierung in Gegenwart eines Katalysators, vorzugsweise Palladium auf Kohle, wird die Nitrogruppe dabei durch die Aminogruppe ersetzt.

Dies betrifft auch Verbindungen der allgemeinen Formel I, in denen $R^6$ eine Aryl- oder Heteroarylgruppe bedeutet. die als Substituenten eine Alkyloxycarbonyl-, Alkyloxycarbonylalkyl- oder Alkyloxycarbonylalkyloxygruppe tragen oder die Gruppe $R^7$ eine Alkoxycarbonylalkylgruppe bedeutet. Hier lassen sich durch Umsetzungen mit Säuren, wie Salzsäure, oder Basen, wie Natronlauge, aus den Alkoxycarbonylgruppen die freien Carbonsäuren herstellen. Setzt man diese Alkoxycarbonylgruppen mit einem Amin der allgemeinen Formel $NHR^8R^9$ um, so wird aus der Alkoxycarbonylgruppe die $CONR^8R^9$-Gruppe. Behandelt man diese Alkoxycarbonylgruppen mit einem Reduktionsmittel wie $LiAlH_4$, so entstehen daraus die entsprechenden Hydroxymethylgruppen.

Dies betrifft auch Verbindungen der allgemeinen Formel I, in denen $R^6$ eine Aryl- oder Heteroarylgruppe bedeutet, die als Substituenten eine oder mehrere Nitrile, Cyanalkyl-, Cyanalkyloxy-, Formylamino-, Alkylcarbonylamino-, Aminocarbonyl-, Alkylaminocarbonylgruppen oder eine Gruppe -S-Y-$CONHR^8$, -O-Y-$CONHR^8$, -NH-Y-$CONHR^8$ tragen, oder in der $R^7$ eine Cyanalkyl-, Aminocarbonylalkyl-, oder die Gruppe -Y-$CONHR^8$ bedeutet. Diese Gruppen lassen sich reduzieren, vorzugsweise mit $LiAlH_4$, wobei die entsprechenden Aminomethyl-Verbindungen entstehen.

Beispiele von physiologisch verwendbaren Salzen der Verbindungen der Formel I sind Salze mit physiologisch verträglichen Mineralsäuren, wie Salzsäure, Schwefelsäure, schweflige Säure oder Phosphorsäure, oder mit organischen Säuren, wie Methansulfonsäure, p-Toluolsulfonsäure, Essigsäure, Trifluoressigsäure, Zitronensäure, Fumarsäure, Maleinsäure, Weinsäure. Bernsteinsäure oder Salicylsäure. Die Verbindungen der Formel I mit freier Carboxygruppe können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-, Calcium- oder Tetramethylammoniumsalz.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten.

Zu reinen Enantiomeren der Verbindungen der Formel I kommt man entweder durch Racematspaltung (über Salzbildung mit optisch aktiven Säuren oder Basen). oder indem man in die Synthese optisch aktive Ausgangsstoffe einsetzt.

Zur Herstellung von Arzneimitteln werden die Substanzen der allgemeinen Formel I mit geeigneten pharmazeutischen Trägersubstanzen, Aroma-, Geschmacks- und Farbstoffen gemischt und beispielsweise als Tabletten oder

Dragees ausgeformt oder unter Zugabe entsprechender Hilfsstoffe in Wasser oder Öl, z.B. in Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethyloxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch einmal pro Tag 1-2 Tabletten mit 20-700 mg Wirkstoff gegeben werden müssen. Der Wirkstoff kann auch durch Injektion 1-8 mal pro Tag oder durch Dauerinfusion gegeben werden, wobei 50-2000 mg pro Tag normalerweise ausreichen.

Bevorzugt im Sinne der Erfindung sind außer den in den Beispielen genannten Verbindungen die folgenden:

1. Benzolsufinsäure-3-methyl-5-[2-(pyridin-4-ylamino)-ethoxy]-phenylester

2 N-{3-[2-(Pyridin-4-ylamino)-ethoxy]-phenyl}-benzolsulfinamid

3. 3-{3-Methyl-5-[2-(pyridin-4-ylamino)-ethoxy]-phenylaminosulfonyl}-phenoxyessigsäure

4. 2-{3-Methyl-5-[2-(pyridin-4-ylamino)-ethoxy]-phenylaminosulfonyl}-phenoxyessigsäure

5. 3-{3-Methyl-5-[2-(pyridin-4-ylamino)-ethoxy]-phenylaminosulfonyl}-phenoxyacetamid

6. N-(2-Hydroxyethyl)-3-{3-methyl-5-[2-(pyridin-4-ylamino)-ethoxy]-phenylaminosulfonyl}-phenoxy-acetamid

7. N-(2,3-Dihydroxypropyl)-3-{3-methyl-5-[2-(pyridin-4-ylamino)-ethoxy]-phenylaminosulfonyl}-phenoxy-acetamid

8. N-(2-Hydroxyethyl)-3-{3-methyl-5-[2-(pyridin-4-ylamino)-ethoxy]-phenyloxysulfonyl}-phenoxy-acetamid

9. 3-{3-Methyl-5-[2-(pyridin-4-ylamino)-ethoxy]-phenylaminosulfonyl}-phenoxyessigsäure-morpholid

10. Essigsäure 2-[2-(benzolsufonyl-{3-methyl-5-[2-(pyridin-4-ylamino)-ethoxy]-phenyl}-amino-acetylamino]-ethyl ester

11. Essigsäure 3-[2-(benzolsufonyl-(3-methyl-5-[2-(pyridin-4-ylamino)-ethoxy]-phenyl}-amino-acetylamino]-propyl ester

12. Essigsäure 2- [2-(2-methoxybenzolsufonyl-{3-methyl-5-[2-(pyridin-4-ylamino) ethoxy]-phenyl}-amino-acetylamino]-ethyl ester

13. N-{3-Cyano-5-[2-(pyridin-4-ylamino)-ethoxy]-phenyl}-benzolsulfonamid

**Beispiel 1**

N-{3-[2-(Pyridin-4-ylamino)-ethoxy]-phenyl}-2-naphthalin-sulfonamid

a) Zu 5.9 g (25 mmol) 3-Aminophenoxyessigsäureethylester und 6.9 mL Triethylamin in 100 mL Methylenchlorid tropfte man unter Eiskühlung bei 10 °C 6.3 g (28 mmol) Naphthalin- 2-sulfonylchlorid in 30 mL Methylenchlorid. Man rührte 1h bei Raumtemperatur, extrahierte mit Wasser und trocknete die organische Phase über Natriumsulfat. Man entfernte das Lösungsmittel i.Vak. und erhielt 9.6 g N-{3-[(Ethoxycarbonyl)-methoxy]-phenyl}-2-naphthalin-sulfonamid als Öl. MS m/e = 385.

b) Zu 9.6 g (25 mmol) dieser Verbindung in 100 mL Ethanol gab man 4.2 g (75 mmol) Kaliumhydroxid und rührte 1 h bei 70 °C. Man filtrierte, löste den Niederschlag in Wasser, säuerte mit konz. Salzsäure an und extrahierte mit

Essigester. Man entfernte das Lösungsmittel Vak, löste den Rückstand in 2 N Natronlauge, säuerte mit konz. Salzsäure an und extrahierte mit Essigester. Man trocknete die organische Phase über Natriumsulfat, filtrierte und entfernte das Lösungsmittel i. Vak. Der ölige Rückstand kristallisierte beim Stehen. Man erhielt 7.6 g (85%) N-{3-[(Carboxy)-methoxy]-pheny]}-2-naphthalinsulfonamid. Fp. 153 - 155 °C. FAB-MS: M+H = 358.

c) Zu 3 g (8.4 mmol) dieser Verbindung in 30 mL Tetrahydrofuran gab man bei 45 °C 2.7 g (16.8 mmol) 1,1-Carbonyldiimidazol und rührte 20 min. Dazu gab man 0.8g (8.4 mmol) 4-Aminopyridin und rührte 6 h bei 60 °C. Dann gab man nochmals 2.7 g (16.8 mmol) 1,1-Carbonyldiimidazol und 0.8 g (8.4 mmol) 4-Aminopyridin zu und rührte weitere 6 h bei 60 °C. Man entfernte das Lösungsmittel i. Vak., nahm den Rückstand in Essigester auf und extrahierte mit wäßrigem Natriumbicarbonat und mit Phosphatpuffer, pH = 7.0. Man trocknete die organische Phase mit Natriumsulfat, filtrierte und entfernte das Lösungsmittel i.Vak.. Man erhielt 2.5 g (69%) N-{3-[(Pyridin-4-ylaminocarbonyl)- methoxy]-phenyl}-2-naphthalinsulfonamid. Fp. 204 - 207 °C MS m/e = 433.

d) Unter Stickstoff gab man 2.0 g (4.6 mmol) dieser Verbindung zu 1.0 g (20.4 mmol) Lithiumaluminiumhydrid in 20mL Tetrahydrofuran und erhitzte l h unter Rückfluß zum Sieden. Man zersetzte überschüssiges $LiAlH_4$ mit Wasser, filtrierte und dampfte das Filtrat i.Vak ein. Man nahm den Rückstand in Essigester auf, extrahierte mit Wasser, trocknete die organische Phase mit Natriumsulfat, filtrierte und entfernte das Lösungsmittel i.Vak.. Den öligen Rückstand trennte man über eine reversed-phase Säule (RP-18; Laufmittel: Methanol / Wasser pH = 6.8, 7 : 3). Die gewünschte Fraktion wurde i. Vak. zur Trockene eingedampft und mit Essigester extrahiert. Man trocknete die organische Phase über Natriumsulfat, filtrierte und entfernte das Lösungsmittel i.Vak.. Man erhielt 0.3 g (16%) der Titelverbindung mit dem Fp. 90 - 91 °C. MS m/e = 419.

**Beispiel 2**

N-{3-[2-(Pyridin-4-ylamino)-ethoxy]-phenyl}-1-naphthalin-sulfonamid

Die Herstellung erfolgte analog Beispiel 1, nur daß in Stufe a) an Stelle von 2-Naphthalinsulfonylchlorid 1-Naphthalin-sulfonylchlorid verwendet wurde.

Zwischenstufen :

a) N-{3-[(Ethoxycarbonyl)-methoxy]-phenyl}-1-naphthalinsulfonamid als Öl. MS m/e = 385.

b) N-{3-[(Carboxy)-methoxy]-phenyl}-1-naphthalinsulfonamid. Fp. 147 - 149 °C. FAB-MS: M+H = 358.

c) N-{3-[(Pyridin-4-ylaminocarbonyl)-methoxy]-phenyl}-1-naphthalinsulfonamid. Fp. 210 - 211 °C (Zers.). MS m/e = 433.

d) Titelverbindung. Ausbeute 38%. Fp. 239 - 241 °C (Zers.). MS: pos. LSIMS m/e = 419.

**Beispiel 3**

4-Methyl-N-{3-[2-(pyridin-4-ylamino)-ethoxy]-phenyl}-benzolsulfonamid

Die Herstellung erfolgte analog Beispiel 1, nur daß in Stufe a) an Stelle von 2-Naphthalinsulfonylchlorid 4-Toluolsulfonylchlorid verwendet wurde.

Zwischenstufen :

a) 4-Methyl-N-{3-[(ethoxycarbonyl)-methoxy]-phenyl}-benzolsulfonamid. Fp.: 100 - 102 °C. MS m/e = 349.

b) 4-Methyl-N-{3-[(carboxy)-methoxy]-phenyl}-benzolsulfonamid. Fp. 170 - 173 °C. FAB-MS: M+H = 322.

c) 4-Methyl-N-{3-[(pyridin-4-ylaminocarbonyl)-methoxy]-phenyl}-benzolsulfonamid als Öl. MS m/e = 397.

d) Titelverbindung. Ausbeute 26%. Fp. 165 - 167 °C. MS: m/e = 383.

**Beispiel 4**

4-Fluor-N-{3-[2-(pyridin-4-ylamino)-ethoxy]-phenyl}-benzolsulfonamid-Hydrochlorid

Die Herstellung erfolgte analog Beispiel 1, nur daß in Stufe a) an Stelle von 2-Naphthalinsulfonylchlorid 4-Fluorbenzol-sulfonylchlorid verwendet wurde.

Zwischenstufen :

a) 4-Fluor-N-{3-[(ethoxycarbonyl)-methoxy]-phenyl}-benzolsulfonamid Fp.: 94 - 96 °C. MS m/e = 353.

b) 4-Fluor-N-{3-[(carboxy)-methoxy]-phenyl}-benzolsulfonamid. Fp.: 154 - 156 °C. FAB-MS: M+H = 326.

c) 4-Fluor-N-{3-[(pyridin-4-ylaminocarbonyl)-methoxy]-phenyl}-benzolsulfonamid. MS m/e = 401.

d) Titelverbindung. Die Base wurde mit Salzsäure in Ether angerieben: Ausbeute 21%. Fp. 203 - 205 °C. MS: m/e = 387.

**Beispiel 5**

4-Chlor-N-{3-[2-(pyridin-4-ylamino)-ethoxy]-phenyl}-benzolsulfonamid-Hydrochlorid

Die Herstellung erfolgte analog Beispiel 1, nur daß in Stufe a) an Stelle von 2-Naphthalinsulfonylchlorid 4-Chlorbenzol-sulfonylchlorid verwendet wurde.

Zwischenstufen :

a) 4-Chlor-N-{3-[(ethoxycarbonyl)-methoxy]-phenyl}-benzolsulfonamid. MS m/e = 369.

b ) 4-Chlor-N-{3-[(carboxy)-methoxy]-phenyl}-benzolsulfonamid. Fp.: 190 - 192 °C. FAB-MS: M+H = 342.

c) 4-Chlor-N-{3-[(pyridin-4-ylaminocarbonyl)-methoxy]-phenyl}-benzolsulfonamid. Fp.: 168 - 171 °C. MS: m/e = 417.

d) 0.65 g (1.55 mmol) aus Stufe c) in 15 mL trockenem Tetrahydrofuran wurde mit 1.79 mL (3.58 mmol) 2 M Borandimethylsulfid in Tetrahydrofuran versetzt. Man rührte 3 h bei 60 °C und setzte dann im Eisbad 10 mL Methanol zu. Dazu gab man 5 mL Chlorwasserstoff in Ether. Man entfernte das Lösungsmittel i. Vak. und rieb den Rückstand mit warmem Wasser an. Man erhielt 0.13 g der Titelverbindung mit Fp.: 241 - 243 °C. MS: m/e = 403.

**Beispiel 6**

4-Trifluormethyl-N-{3-[2-(pyridin-4-ylamino)-ethoxy]-phenyl}-benzolsulfonamid

Die Herstellung erfolgte analog Beispiel 1, nur daß in Stufe a) an Stelle von 2-Naphthalinsulfonylchlorid 4-Trifluormethyl-benzolsulfonylchlorid verwendet wurde.

Zwischenstufen :

a) 4-Trifluormethyl-N-{3-[(ethoxycarbonyl)-methoxy]-phenyl}-benzolsulfonamid. MS m/e = 403.

b) 4-Trifluormethyl-N-{3-[(carboxy)-methoxy]-phenyl}-benzolsulfonamid. Fp.: 155 - 158 °C. FAB-MS: M+H = 375.

c) 4-Trifluormethyl-N-{3-[(pyridin-4-ylamino-carbonyl)-methoxy]-phenyl}-benzolsulfonamid. Fp.: 66 - 68 °C. MS m/e = 451.

d) Titelverbindung. Fp.: 145 - 148 °C. MS: m/e = 437.

**Beispiel 7**

3-Trifluormethyl-N-{3-[2-(pyridin-4-ylamino)-ethoxy]-phenyl}-benzolsulfonamid

Die Herstellung erfolgte analog Beispiel 1, nur daß in Stufe a) an Stelle von 2-Naphthalinsulfonylchlorid 3-Trifluormethyl-benzolsulfonylchlorid verwendet wurde.

Zwischenstufen :

a) 3-Trifluormethyl-N-{3-[(ethoxycarbonyl)-methoxy]-phenyl}-benzolsulfonamid. MS m/e = 403.

b) 3-Trifluormethyl-N-{3-[(carboxy)-methoxy]-phenyl}-benzolsulfonamid. Fp.: 147 - 149 °C. FAB-MS: M+H = 375.

c ) 3-Trifluormethyl-N-{3-[(pyridin-4-ylamino-carbonyl)-methoxy]-phenyl}-benzolsulfonamid als Öl. MS m/e = 451.

d) Titelverbindung. Fp.: 185 - 187 °C. MS: m/e = 437.

**Beispiel 8**

N-{3-[2-(Pyridin-4-ylamino)-ethoxy]-phenyl}-cyclohexan-sulfonamid

Die Herstellung erfolgte analog Beispiel 1, nur daß in Stufe a) an Stelle von 2-Naphthalinsulfonylchlorid Cyclohexylsulfonylchlorid verwendet wurde.

Zwischenstufen :

a ) N-{3-[(Ethoxycarbonyl)-methoxy]-phenyl}-cyclohexansulfonamid. MS m/e = 341.

b) N-{3-[(Carboxy)-methoxy]-phenyl}-cyclohexan-sulfonamid. Fp.: 132 °C. FAB-MS: M+H = 313.

c ) N-{3-[(Pyridin-4-ylamino-carbonyl)-methoxy]-phenyl}-cyclohexansulfonamid als Öl. MS m/e = 389.

d) Titelverbindung. MS: m/e = 375.

**Beispiel 9**

N-{3-[2-(Pyridin-4-ylamino)-ethoxy]-phenyl}-benzolsulfonamid

Die Herstellung erfolgte analog Beispiel 1, nur daß in Stufe a) an Stelle von 2-Naphthalinsulfonylchlorid Benzolsulfonyl-chlorid verwendet wurde.

Zwischenstufen :

a) N-{3-[(Ethoxycarbonyl)-methoxy]-phenyl}-benzolsulfonamid als Öl. MS m/e = 335.

b) N-{3-[(Carboxy)-methoxy]-phenyl}-benzol-sulfonamid. Fp. 160 - 161 °C. FAB-MS: M+H = 307

c) N-{3-[(Pyridin-4-ylaminocarbonyl)-methoxy]-phenyl}-benzolsulfonamid Fp.: 151 - 156 °C. MS m/e = 383.

d) Titelverbindung. Ausbeute 26%. Fp. 182 - 184 °C. MS: m/e = 369.

**Beispiel 10**

N-{3-[1-Methyl-2-(pyridin-4-ylamino)-ethoxy]-phenyl}-benzolsulfonamid

a) Zu 8.4 g (40 mmol) 2-(3-Aminophenoxy)-propionsäure-ethylester und 6.1 mL (44 mmol) Triethylamin in 50 mL Methylenchlorid tropfte man unter Eiskühlung bei 10 °C 5.6mL (44 mmol) Benzolsulfonylchlorid. Man rührte 1 h bei Raumtemperatur. extrahierte mit Wasser, trocknete die organische Phase mit Natriumsulfat, filtrierte und entfernte das Lösungsmittel i. Vak.. Man erhielt 14 g N-{3-[-(Ethoxycarbonyl)-ethoxy]-phenyl}-benzolsulfonamid als Öl. MS: m/e = 349.

b) 14 g (40 mmol) dieser Verbindung und 6.7 g (120 mmol) Kaliumhydroxid in 100 mL Ethanol rührte man 1 h bei 70 °C. Man extrahierte zweimal mit Essigester, säuerte mit halbkonzentrierter Salzsäure an und extrahierte nochmals mit Essigester. Die vereinigten organischen Phasen trocknete man über Natriumsulfat, filtrierte und entfernte das Lösungsmittel i. Vak.. Man erhielt 9.2 g (72%) N-(3-[1-(Carboxy)-ethoxy]-phenyl}-benzolsulfonamid als Öl. MS: m/e = 321.

c) Wie in Beispiel 1. Stufe c). stellte man aus 4.8 g (15mmol) dieser Verbindung, 2.1 g (22.5 mmol) 4-Aminopyridin und 3.2 g (19.5 mmol) 1,1-Carbonyldiimidazol in 40 mL Tetrahydrofuran 3.4 g (57%) N-(3-[1-(Pyridin-4-yl-aminocarbonyl)-ethoxy]-phenyl}-benzolsulfonamid her. Fp. 142 - 144 °C. MS: m/e = 397.

d) Wie in Beispiel 1, Stufe d) stellte man aus 1.7 g (4.3mmol) dieser Verbindung und 0.65 g (17.2 mmol) Lithiumaluminiumhydrid in 20 mL Tetrahydrofuran 0.6 g (37%) der Titelverbindung her. Fp. 162 - 163 °C. MS: m/e = 383.

**Beispiel 11**

N-{3-[1,1-Dimethyl-2-(pyridin-4-ylamino)-ethoxy]-phenyl}-benzolsulfonamid

Die Verbindung wurde analog Beispiel 10 hergestellt. In der Stufe a) verwendete man an Stelle von 2-(3-Aminophenoxy)-propionsäure-ethylester 2-Methyl-2-(3-aminophenoxy)-propionsäure-ethylester.

a ) N-{3-[1-Methyl-1-(ethoxy-carbonyl)-ethoxy]-phenyl}-benzolsulfonamid als Öl. MS: m/e = 363.

b) N-{3-[1-Methyl-(carboxy)-ethoxy]-phenyl}-benzolsulfonamid als Öl. MS: m/e = 335.

c) N-{3-[1-Methyl-(pyridin-4-yl-amino-carbonyl)-ethoxy]-phenyl}-benzolsulfonamid. Fp. 141 - 143°C. MS: m/e = 411.

d) Titelverbindung als Öl. MS: m/e = 397.

**Beispiel 12**

N-Methyl-N-{3-[2-(pyridin-4-ylamino)-ethoxy]-phenyl}-benzolsulfonamid-Hydrochlorid

a ) Zu 8.4 g (25 mmol) N-{3-[(Ethoxycarbonyl)-methoxy]-phenyl}-benzolsulfonamid (Beispiel 9, Stufe a) und 3.5 g Kaliumcarbonat in 10 mL Dimethylformamid tropfte man bei 80 - 90 °C eine Lösung von 1.6 mL (25 mmol) Iodmethan in 10 mL Dimethylformamid. Man rührte 2 h bei dieser Temperatur weiter, ließ auf Raumtemperatur abkühlen, filtrierte und engte das Filtrat i.Vak. ein. Man nahm den Rückstand in Essigester auf und extrahierte mit Wasser. Man trocknete über Natriumsulfat. filtrierte und entfernte das Lösungsmittel i. Vak.. Man erhielt 8.6 g N-Methyl-N-{3-[(ethoxycarbonyl)-methoxy]-phenyl}-benzolsulfonamid als Öl. MS: m/e = 349.

b) Die weitere Umsetzung erfolgte wie in Beispiel 1. Stufe b. Man erhielt N-Methyl-N-{3-[(carboxy)-methoxy]-phenyl}-benzolsulfonamid. Fp.: 110-111 °C. MS: m/e = 321.

c ) N-Methyl-N-{3-[(pyridin-4-ylaminocarbonyl)-methoxy]-pheny]}-benzolsulfonamid Fp.: 76 - 78 °C. MS m/e = 397.

d) Titelverbindung. Man versetzte die freie Base mit 2 N Salzsäure. extrahierte mit Essigester und dampfte die wässrige Phase zur Trockene ein. Man erhielt ein Öl, das nach Anreiben mit Isopropanol kristallisierte. Ausbeute 46%. Fp.: 174 - 176 °C.

**Beispiel 13**

N-Ethyl-N-{3-[2-(pyridin-4-ylamino)-ethoxy]-phenyl}-benzolsulfonamid

Die Herstellung erfolgte analog Beispiel 12, nur daß man in Stufe a) an Stelle von Iodmethan Iodethan verwendete.

a ) N-Ethyl-N-{3-[(ethoxycarbonyl)-methoxy]-phenyl}-benzolsulfonamid als Öl. MS: m/e = 363.

b) N-Ethyl-N-{3-[(carboxy)-methoxy]-phenyl}-benzolsulfonamid. Fp.: 122 °C. MS: m/e = 335.

c) N-Ethyl-N-{3-[(pyridin-4-ylaminocarbonyl)-methoxy]-phenyl}-benzolsulfonamid. MS m/e = 411.

d) Titelverbindung als Öl. MS m/e = 397.

**Beispiel 14**

N-Propyl-N-{3-[2-(pyridin-4-ylamino)-ethoxy]-phenyl}-benzolsulfonamid

Die Herstellung erfolgte analog Beispiel 12, nur daß man in Stufe a) an Stelle von Iodmethan Iodpropan verwendete.

a ) N-Propyl-N-{3-[(ethoxycarbonyl)-methoxy]-phenyl}-benzolsulfonamid als Öl. MS: m/e = 377.

b) N-Propyl-N-{3-[(carboxy)-methoxy]-phenyl}-benzolsulfonamid, Fp.: 147 °C. MS: m/e = 349.

c ) N-Propyl-N-{3-[(pyridin-4-ylaminocarbonyl)-methoxy]-phenyl}-benzolsulfonamid Fp.: 105 °C. MS m/e = 425.

d) Titelverbindung als Öl. MS m/e = 411.

**Beispiel 15**

N-Benzyl-N-{3-[2-(pyridin-4-ylamino)-ethoxy]-phenyl}-benzolsulfonamid

Die Herstellung erfolgte analog Beispiel 12, nur daß man in Stufe a) an Stelle von Iodmethan Benzylbromid verwendete.

a) N-Benzyl-N-{3-[(ethoxycarbonyl)-methoxy]-phenyl}-benzolsulfonamid als Öl, MS: m/e = 425.

b) N-Benzyl-N-{3-[(carboxy)-methoxy]-phenyl}-benzolsulfonamid. Fp.: 190 °C. MS: m/e = 397.

c) N-Benzyl-N-{3-[(pyridin-4-ylaminocarbonyl)-methoxy]-phenyl}-benzolsulfonamid. Fp. 140 °C. MS m/e = 473.

d) Titelverbindung. Fp.: 128 °C. MS m/e = 459.

**Beispiel 16**

N-Allyl-N-{3-[2-(pyridin-4-ylamino)-ethoxy]-phenyl}-benzolsulfonamid

Die Herstellung erfolgte analog Beispiel 12, nur daß man in Stufe a) an SteUe von Iodmethan Allylbromid verwendete.

a) N-Allyl-N-{3-[(ethoxycarbonyl)-methoxy]-phenyl}-benzolsulfonamid als Öl, MS: m/e = 375.

b) N-Allyl-N-{3-[(carboxy)-methoxy]-phenyl}-benzolsulfonamid. MS: m/e = 347

c) N-Allyl-N-{3-[(pyridin-4-ylaminocarbonyl)-methoxy]-phenyl}-benzolsulfonamid. MS m/e = 423.

d) Titelverbindung als Öl. MS m/e = 409.

**Beispiel 17**

N-{5-Methyl-3-[2-(pyridin-4-ylamino)-ethoxy]-phenyl}-benzolsulfonamid Hydrochlorid

Die Herstellung erfolgte analog Beispiel 1, nur daß in Stufe a) an Stelle von 2-Naphthalinsulfonylchlorid Benzol-sulfonylchlorid und an Stelle von 3-Amino phenoxyessigsäure- Ethylester 3-Amino- 5-methylphenoxyessigsäure-Ethylester verwendet wurde.

Zwischenstufen :

a) N-{5-Methyl-3-[(ethoxycarbonyl)-methoxy]-phenyl}-benzolsulfonamid als Öl. MS m/e = 349.

b) N-{5-Methyl-3-[(carboxy)-methoxy]-phenyl}-benzolsulfonamid. Fp. : 156 - 159 °C. FAB-MS: M+H = 322.

c) N-{5-Methyl-3-[(pyridin-4-ylaminocarbonyl)-methoxy]-phenyl}-benzolsulfonamid Fp.: 193 - 196 °C. MS m/e = 397.

d) Titelverbindung. Ausbeute 56%. Fp. 170 °C. MS: m/e = 383.

**Beispiel 18**

Benzolsulfonsäure-3-[2-(pyridin-4-ylamino))-ethoxy]-phenylester

a) Zu 2.5 g (0.01 mol) Benzolsulfonsäure-3-hydroxy-phenyl-ester in 30 ml Acetonitril gibt man unter Kühlen bei 10°C 0.44 g (0.011 mol) Natriumhydrid (60% in Weißöl) und rührt 1 h bei dieser Temperatur. Innnerhalb von 30 min tropft man 2.2 ml (0.02 mol) Bromessigsäureethylester in 10 ml Acetonitril zu und rührte 3 h bei Raumtemperatur. Nach Zugabe von 5 ml Isopropanol entfernt man das Lösungs- mittel i. Vak. Zum Rückstand gibt man 30 ml Ethanol, 50 ml Wasser und 0.8 g (0.015 mol) Kaliumhydroxid. Nach 16h bei Raumtemperatur entfernt man das Ethanol i. Vak und extra- hiert die wässrige Lösung dreimal mit Ether. Man säuert die wässrige Phase mit Salzsäure an und extrahiert mit Ether. Man entfernt den Ether i. Vak und erhält 1.5 g (48%) 2-[3-(Phenylsulfonyloxy)-phenyloxy]-essigsäure mit dem Fp. 152 - 155°C.

b) 1.4 g (4.5 mmol) dieser Verbindung und 958 mg (5.9 mmol) Carbonyldiimidazol rührt man bei 45°C 30 min. Man gibt 0.64 g (6.8 mmol) 4-Aminopyridin zu und rührt zwei Tage bei 60°C. Man entfernt das Lösungsmittel i. Vak. und löst den Rückstand in Essigester, der 0.5 % Essigsäure enthielt. Man trocknet die organische Phase, filtriert und entfernt das Lösungsmittel i. Vak. Man reibt den Rückstand mit Ether an. filtriert und erhält 1.8 g (94%) N-(4-Pyridinyl)-2-[3-(phenylsulfonyloxy)-phenyloxy]-acetamid mit dem Fp. 127 - 130°C.

c) Unter Kühlen im Eisbad versetzt man 192 mg (8.8 mmol) Lithiumborhydrid in 5 ml trockenem Tetrahydrofuran bei 5°C mit 2.23 ml (18 mmol) Chlortrimethylsilan. Nach 30min gibt man bei 5°C langsam 1.7 g (4.4 mmol) der Verbindung aus Stufe b) zu. Nach 16 h bei Raumtemperatur zersetzte man mit 3 ml Methanol und entfernt das Lösungs-mittel i.Vak.. Man nimmt den Rückstand in Essigester und Bicarbonatlösung auf. Die Essigesterphase reinigt man über eine Kieselgelsäule (Essigester / Methanol = 9 : 1). Man entfernt das Lösungsmittel i. Vak. und erhält 1.3 g der Titelverbindung (80%) als viskoses Öl. FAB-MS: M+H 371.

**Beispiel 19**

N-Methyl-N-phenyl-3-[2-(pyridin-4-ylamino)-ethoxy]-benzol-sulfonamid

a) 3 -Nitrobenzolsulfonsäure-N-methyl-anilid
5 g 3-Nitrobenzolsulfonsäurechlorid werden in 20 ml absol. Pyridin gelöst und unter Eiskühlung und Rühren mit 2.7ml N-Methylanilin versetzt. Man rührt 2 Stdn. bei Raumtemperatur weiter, gibt das Reaktionsgemisch auf Eiswasser und säurt mit verdünnter Salzsäure an. Die wäßrige Phase wird mit Essigester extrahiert, die Essig- esterphase über Natriumsulfat getrocknet und eingedampft. Der Rückstand wird aus Alkohol umkristallisiert. Ausb.: 6,3 g. Fp.: 90° C.

b ) 3-Aminobenzolsulfonsäure-N-methyl-anilid
6 g 3-Nitrobenzolsulfonsäure-N-methyl-anilid werden in 100 ml absol. Tetrahydrofuran gelöst und nach Zugabe von 0,5 g Pd/C-(10%)-Katalysator hydriert. Nach Aufnahme der berechneten Menge an Wasserstoff wird vom Katalysator ab- filtriert und das Filtrat eingedampft. Ausb.: 5,5 g. Fp.: 104° C.

c) 3-Hydroxybenzolsulfonsäure-N-methyl-anilid
5 g 3-Aminobenzolsulfonsäure-N-methylanilid werden in 20ml 50%iger Schwefelsäure gelöst. Hierzu tropft man

unter Eiskühlung und Rühren eine Lösung von 1.75 g Natriumnitrit in 5 ml Wasser. Nach beendeter Diazotierung erhitzt man die Reaktionsmischung 20 min. auf 100° C, läßt abkühlen und extrahiert mit Essigester. Die Essigesterphase wird über Natriumsulfat getrocknet und eingedampft. Der Rückstand ist für die Weiterverarbeitung genügend rein.

d) [3-(Methyl-phenyl-sulfamoyl)-phenoxy]-essigsäure-ethylester

3,5 g 3-Hydroxybenzolsulfonsäure-N-methyl-anilid werden in 20 ml absol. Dimethylformamid gelöst. Hierzu gibt man 2g Kaliumcarbonat und 1.9 ml Bromessigsäureethylester und erhitzt die Mischung 3 Stdn. auf 100° C. Man kühlt ab und destilliert i. Vak. vom Lösungsmittel ab. Der erhaltene Rückstand (4,3 g) ist für die Weiterverarbeitung genügend rein.

e) [3-(Methyl-phenyl-sulfamoyl)-phenoxy]-essigsäure

4,2 g [3-(Methyl-phenyl-sulfamoyl)-phenoxy]-essigsäure-ethylester werden in 40 ml Ethanol gelöst. Hierzu gibt man 1g Kaliumhydroxid und rührt die Mischung eine Stunde bei 90° C. Man kühlt auf Raumtemperatur ab. säuert mit verdünnter Salzsäure an und extrahiert mit Methylenchlorid. Die Methylenchloridphase wird über Natriumsulfat getrocknet und eingedampft. Man erhält 4g [3-(Methyl-phenyl-sulfamoyl)-phenoxy]-essigsäure als amorphen Feststoff.

f) 2-[3-(Methyl-phenyl-sulfamoyl)-phenoxy]-N-pyridin-4-yl-acetamid

2 g [3-(Methyl-phenyl-sulfamoyl)-phenoxy]-essigsäure werden in 20 ml absol. Tetrahydrofuran gelöst. Hierzu gibt man 1,35 g Carbonyldiimidazol und erwärmt die Mischung 20min auf 45° C. Man kühlt auf Raumtemperatur ab, setzt 900 mg 4-Amino-pyridin zu und rührt 3 Stdn. bei 60°C weiter. Man destilliert das Lösungsmittel ab, löst den Rückstand in Essigester und schüttelt mit Wasser aus. Die Essigesterphase wird über Natriumsulfat getrocknet und eingedampft. Der Rückstand wird zur Reinigung an einer Kieselgelsäule chromatographiert. (Elutionsmittel: Methylenchlorid/Methanol 9:5). Nach Eindampfen der Säulenfraktionen erhält man 1,5 g der Titelverbindung als amorphen Feststoff. FAB-MS:M+H 398.

g) N-Methyl-N-phenyl-3-[2-(pyridin-4-ylamino-ethoxy]-benzol-sulfonamid

800 mg 2-[3-(Methyl-phenyl-sulfamoyl)-phenoxy]-N-pyridin-4-yl-acetamid werden in 15 ml absol. Tetrahydrofuran ge- löst. Hierzu gibt man unter Stickstoff 320 mg Lithiumaluminiumhydrid und erhitzt die Mischung anschließend eine Stunde auf Rückflußtemperatur. Man kühlt ab und zersetzt den Reaktionsansatz mit gesättigter Ammoniumsulfatlösung. Man saugt vom Ungelösten ab, wäscht den Filterrückstand mit Ether, trocknet das Filtrat über Natriumsulfat und dampft ein. Der Rückstand wird zur Reinigung an einer Kieselgelsäule chromatographiert. (Elutionsmittel:Methylenchlorid/Methanol 8:2). Nach Eindampfen der Säulenfraktionen erhält man 420 mg der Titelverbindung als amorphe Substanz. FAB-MS: M+H 384.

**Beispiel 20**

N-Benzyl-N-phenyl-3-[2-(pyridin-4-ylamino)-ethoxy]-benzol-sulfonamid

Die Darstellung der Titelverbindung erfolgte analog Beispiel 19, nur daß in Stufe a) anstelle von N-Methyl-anilin N-Benzyl-anilin eingesetzt wurde. Amorphe Substanz. FAB-MS:M+H 460.

**Beispiel 21**

N-phenyl-3-[2-(pyridin-4-ylamino)-ethoxy]-benzolsulfonamid

300 mg N-Benzyl-N-phenyl-3-[2-(pyridin-4-ylamino)-ethoxy]-benzolsulfonamid (Bsp. 20) werden in 20 ml Metanol gelöst und nach Zugabe von 100 mg Pd/C(10%ig)-Katalysator hydriert. Nach Beendigung der Wasserstoffaufnahme wird vom Katalysator abfiltriert und eingedampft. Man erhält 240 mg der Titelverbindung als amorphe Substanz. FAB-MS:M+H 370.

**Beispiel 22**

N-Methyl-N-pyridin-2-yl-3-[2-(pyridin-4-ylamino)-ethoxy]-benzolsulfonamid

Die Darstellung der Titelverbindung erfolgte analog Beispiel 19, nur daß in Stufe a) anstelle von N-Methyl-anilin N-Methyl-2-amino-pyridin eingesetzt wurde. Amorphe Substanz. FAB-MS:M+H:385.

**Beispiel 23**

N-{3-[2-(pyridin-4-ylamino)-ethoxy]-phenyl}-2-propansulfonamid Hydrochlorid

Zu 0.26 g (11.4 mmol) $LiBH_4$ in 50 mL Tetrahydrofuran tropfte man 2. 88 mL (22.9 mmol) Chlortrimethylsilan. rührte 5 min bei Raumtemperatur und gab portionsweise 2.00 g (5.72 mmol) N-{3-[(Pyridin-4-ylaminocarbonyl)-methoxy]-phenyl}-2-propansulfonamid zu, das man analog dem Beispiel 1 hergestellt hatte. Man erhitzte 30 min unter Rückfluß zum Sieden, tropfte nach Abkühlung vorsichtig 20 mL Methanol zu, gefolgt von 30 mL 2 N Natronlauge. Man entfernte das Lösungsmittel zum größten Teil i. Vak. und extrahierte mit Methylenchlorid. Man trocknete. entfernte das Lösungsmittel i. Vak. und versetzte den Rückstand mit etherischer Salzsäure. Man entfernte das Lösungsmittel i. Vak., rieb den Rückstand mit tert.Butylmethylether an und erhielt 1.5 g (70%) der Titelverbindung mit dem Fp. 204 - 207 °C.

**Beispiel 24**

N-{3-[2-(Pyridin-4-ylamino)-ethoxy]-phenyl}-cyclopentansulfonamid Hydrochlorid

stellte man analog Beispiel 23 her. Fp. 129 - 134 °C.

**Beispiel 25**

N-Methyl-N-{3-[2-(pyridin-4-ylamino)-ethoxy]-phenyl}-4-fluorphenylsulfonamid Hydrochlorid

stellte man analog Beispiel 23 her. Fp. 140 - 143 °C.

**Beispiel 26**

N-{3-[2-(Pyridin-4-ylamino)-ethoxy]-phenyl}-benzolsulfonamid

stellte man analog Beispiel 1 her. Öl. MS: [EI] = 383.

**Beispiel 27**

N-{3-[2-(Pyridin-4-ylamino)-ethoxy]-phenyl}-4-*tert.*butylbenzolsulfonamid

stellte man analog Beispiel 1 her. Öl. MS [EI] = 425.

**Beispiel 28**

N-{3-[2-(Pyridin-4-ylamino)-ethoxy]-phenyl}-1,2,3,4-tetrahydronaphtalin-6-sulfonamid Hydrochlorid

stellte man analog Beispiel 23 her. Fp. 235 - 237 °C.

**Beispiel 29**

N-{3-[2-(Pyridin-4-ylamino)-ethoxy]-phenyl}-indan-5-sulfonamid

stellte man analog Beispiel 23 als freie Base her. Fp. 140°C (Zers.).

**Beispiel 30**

N-{3-[2-(Pyridin-4-ylamino)-ethoxy]-phenyl}-2-biphenylsulfonamid

stellte man analog Beispiel 23 als freie Base her. Fp. 214 - 216 °C.

**Beispiel 31**

N-Methyl-N-{5-methyl-3-[2-(pyridin-4-ylamino)-ethoxy]-phenyl}-4-fluorbenzolsulfonamid Hydrochlorid

stellte man analog Beispiel 23 her. Fp. 122 - 129 °C. Das Ausgangsmaterial N-{5-Methyl-3-[(pyridin-4-ylaminocarbonyl)-methoxy]-phenyl}-4-fluorbenzolsulfonamid (MS m/e = 429) stellte man analog Beispiel 17 her.

**Beispiel 32**

N-{5-Methyl-3-[2-(pyridin-4-ylamino)-ethoxy]-phenyl}-2-chlor-4-fluorbenzolsulfonamid Hydrochlorid

stellte man analog Beispiel 23 her. Fp. 198 - 200 °C. Das Ausgangsmaterial N-{5-Methyl-3-[(pyridin-4-ylaminocarbonyl)-methoxy]-phenyl}-2-chlor-4-fluorbenzolsulfonamid (MS m/e = 449) stellte man analog Beispiel 17 her.

**Beispiel 33**

N-Methyl-N-{5-methyl-3-[2-(pyridin-4-ylamino)-ethoxy]-phenyl}-2-trifluorbenzolsulfonamid Hydrochlorid

stellte man analog Beispiel 23 her. Fp. 203 - 207 °C. Das Ausgangsmaterial N-{5-Methyl-3-[(pyridin-4-ylaminocarbonyl)-methoxy]-phenyl}-2-chlor-4-fluorbenzolsulfonamid (MS m/e = 479) stellte man analog Beispiel 17 her.

**Beispiel 34**

N-{5-Methyl-3-[2-(pyridin-4-ylamino)-ethoxy]-phenyl}-2-methylbenzolsulfonamid Hydrochlorid

stellte man analog Beispiel 23 her. Fp. 135 °C (Zers.). Das Ausgangsmaterial N-{5-Methyl-3-[(pyridin-4-ylaminocarbonyl)-methoxy]-phenyl}-2-methylbenzolsulfonamid (MS m/e = 411) stellte man analog Beispiel 17 her.

**Beispiel 35**

N-Methyl-N-{5-methyl-3-[2-(pyridin-4-ylamino)-ethoxy]-phenyl}-2-methyl-4-fluorbenzolsulfonamid Hydrochlorid

stellte man analog Beispiel 23 her. Fp. 146 °C. Das Ausgangsmaterial N-Methyl-N-{5-methyl-3-[(pyridin-4-ylaminocarbonyl)-methoxy]-phenyl}-2-methyl-4-fluorbenzolsulfonamid (MS m/e = 443) stellte man analog Beispiel 17 her.

**Beispiel 36**

N-{5-Methyl-3-[2-(pyridin-4-ylamino)-ethoxy]-phenyl}-2-methyl-4-fluorbenzolsulfonamid Hydrochlorid

stellte man analog Beispiel 23 her. Fp. 193 °C. Das Ausgangsmaterial N-{5-Methyl-3-[(pyridin-4-ylaminocarbonyl)-methoxy]-phenyl}-2-methyl-4-fluorbenzolsulfonamid (MS m/e = 429) stellte man analog Beispiel 17 her.

**Beispiel 37**

N-{5-Methyl-3-[2-(pyridin-4-ylamino)-ethoxy]-phenyl}-2-methyl-5-fluorbenzolsulfonamid Hydrochlorid

stellte man analog Beispiel 23 her. Fp. 246 - 247 °C. Das Ausgangsmaterial N-{5-Methyl-3-[(pyridin-4-ylaminocarbonyl)-methoxy]-phenyl}-2-methyl-5-fluorbenzolsulfonamid (MS m/e = 429; Fp 211 - 213 °C) stellte man analog Beispiel 17 her.

**Beispiel 38**

N-Methyl-N-{5-methyl-3-[2-(pyridin-4-ylamino)-ethoxy]-phenyl}-2-methyl-5-fluorbenzolsulfonamid Hydrochlorid

stellte man analog Beispiel 23 her. Fp. 165 - 166 °C. Das Ausgangsmaterial N-Methyl-N-{5-Methyl-3-[(pyridin-4-ylaminocarbonyl)-methoxy]-phenyl}-2-methyl-5-fluorbenzolsulfonamid (MS m/e = 443) stellte man analog Beispiel 17 her.

**Beispiel 39**

N-{5-Methyl-3-[2-(pyridin-4-ylamino)-ethoxy]-phenyl}-2,4-difluorbenzolsulfonamid Hydrochlorid

stellte man analog Beispiel 23 her. Fp. 227 - 228 °C. Das Ausgangsmaterial N-{5-Methyl-3-[(pyridin-4-ylaminocarbonyl)-methoxy]-phenyl}-2,4-difluorbenzolsulfonamid (MS m/e = 433; Fp. 194 °C) stellte man analog Beispiel 17 her.

**Beispiel 40**

N-{5-Methyl-3-[2-(pyridin-4-ylamino)-ethoxy]-phenyl}-3,5-dimethyl-4-pyrazolsulfonamid

stellte man analog Beispiel 23 als freie Base her. Fp. 121 °C. Das Ausgangsmaterial N-{5-Methyl-3-[(pyridin-4-ylaminocarbonyl)-methoxy]-phenyl}-3,5-dimethyl-4-pyrazolsulfonamid (MS m/e = 415) stellte man analog Beispiel 17 her.

**Beispiel 41**

N-{5-Methyl-3-[2-(pyridin-4-ylamino)-ethoxy]-phenyl}-4-fluorbenzolsulfonamid Hydrochlorid

stellte man analog Beispiel 23 her. Fp. 232 °C. Das Ausgangsmaterial N-{5-Methyl-3-[(pyridin-4-ylaminocarbonyl)-methoxy]-phenyl}-4-fluorbenzolsulfonamid (MS m/e = 415; Fp. 156 - 159 °C) stellte man analog Beispiel 17 her.

**Beispiel 42**

N-{5-Methyl-3-[2-(pyridin-4-ylamino)-ethoxy]-phenyl}-2-fluorbenzolsulfonamid Hydrochlorid

stellte man analog Beispiel 23 her. Fp. 263 °C. Das Ausgangsmaterial N-{5-Methyl-3-[(pyridin-4-ylaminocarbonyl)-methoxy]-phenyl}-2-fluorbenzolsulfonamid (MS m/e = 415) stellte man analog Beispiel 17 her.

**Beispiel 43**

N-{5-Methyl-3-[2-(pyridin-4-ylamino)-ethoxy]-phenyl}-2-trifluormethylbenzolsulfonamid Hydrochlorid

stellte man analog Beispiel 23 her. Fp. 217 - 222. °C. Das Ausgangsmaterial N-{5-Methyl-3-[(pyridin-4-ylaminocarbonyl)-methoxy]-phenyl}-2-trifluormethylbenzolsulfonamid (MS m/e = 465) stellte man analog Beispiel 17 her.

**Beispiel 44**

N-Methyl-N-{5-methyl-3-[2-(pyridin-4-ylamino)-ethoxy]-phenyl}-4-methylbenzolsulfonamid Hydrochlorid

stellte man analog Beispiel 23 her. Fp.180 °C. Das Ausgangsmaterial N-Methyl-N-{5-methyl-3-[(pyridin-4-ylaminocarbonyl)-methoxy]-phenyl}-4-methylbenzolsulfonamid (MS m/e = 425) stellte man analog Beispiel 17 her.

**Beispiel 45**

N-{5-Methyl-3-[2-(pyridin-4-ylamino)-ethoxy]-phenyl}-2,6-difluorbenzolsulfonamid Hydrochlorid

stellte man analog Beispiel 23 her. Fp. 263 °C. Das Ausgangsmaterial N-{5-Methyl-3-[(pyridin-4-ylaminocarbonyl)-methoxy]-phenyl}-2,6-difluorbenzolsulfonamid (MS m/e = 433; Fp = 234 - 242 °C) stellte man analog Beispiel 17 her.

**Beispiel 46**

N-{5-Methyl-3-[2-(pyridin-4-ylamino)-ethoxy]-phenyl}-2-hydroxy-3-*tert.*butyl-5-methylbenzolsulfonamid Hydrochlorid

a) 27.2 g (87.0 mmol) (3-*tert.*Butyloxycarbonylamino-5-methyl-phenoxy)-essigsäureethylester (Beispiel 57b) in 300 mL Methanol versetzte man mit 50 mL (100 mmol) 2 N Natronlauge und rührte 3 d bei Raumtemperatur. Man entfernte das Lösungsmittel zum Teil i.Vak., extrahierte mit Essigester, säuerte die wäßrige Phase mit Salzsäure an und extrahierte mit Ether. Man trocknete und entfernte das Lösungsmittel i. Vak. Man erhielt 15.6 g (3-*tert.*

Butyloxycarbonylamino-5-methyl-phenoxy)-essigsäure mit dem Fp. 120-122 °C.

b) Diese Verbindung setzte man mit 4-Aminopyridin um wie in Beispiel 1c) beschrieben und erhielt N-(4-Pyridinyl-(3-*tert*.butyloxycarbonylamino-5-methyl-phenoxy)-essigsäureamid mit dem Fp. 204 - 205 °C.

c) 5.00 g (14.0 mmol) dieser Verbindung versetzte man mit 25 mL Trifluoressigsäure, rührte 30 min bei Raumtemperatur, alkalisierte mit Natronlauge und saugte den Niederschlag ab. Man erhielt 2.93 g (78%) N-(4-Pyridinyl)-3-(amino-5-methylphenoxy)-essigsäureamid. mit dem Fp. 163 °C.

d) Diese Verbindung reduzierte man wie in Beispiel 23 beschrieben und erhielt in 50 % Ausbeute 3-[2-(Pyridin-4-ylamino)-ethoxy]-5-methyl-anilin mit dem Fp. 208 °C.

e) Diese Verbindung setzte man mit 2-Hydroxy-3-*tert*.butyl-5-methyl-benzolsulfonylchlorid um wie in Beispiel la) beschrieben und erhielt die Titelverbindung. Amorph. MS +FAB: 470.

**Beispiel 47**

N-{5-Methyl-3-[2-(pyridin-4-ylamino)-ethoxy]-phenyl}-3-benzothiophen-sulfonamid Hydrochlorid

stellte man analog Beispiel 46 her. Dazu setzte man die in Bsp. 46d) erhaltene Verbindung mit Benzothiophen-3-sulfonylchlorid um Fp. 130 °C (Zers.)

**Beispiel 48**

N-{5-Methyl-3-[2-(pyridin-4-ylamino)-ethoxy]-phenyl}-benzo-2,3,1-thiadiazol-4-sulfonamid Hydrochlorid

stellte man analog Beispiel 46 her, Dazu setzte man die in Bsp. 46d) erhaltene Verbindung mit Benzo-2,3,1-thiadiazol-4-sulfonylchlorid um. Fp. 110 °C.

**Beispiel 49**

N-{5-Methoxy-3-[2-(pyridin-4-ylamino)-ethoxy]-phenyl}-4-fluor-benzolsulfonamid Hydrochlorid

a) 16.0 g (115 mmol) 3-Hydroxy-5-methoxy-phenol (G. Rodighiero, C. Antonello, Il Farmaco, Ed. Sci. 10, 889 - 896, (1955)), 3.7 g Ammoniumchlorid, 13.8 mL Wasser und 24 mL konz. Ammoniak erhitzte man in einem 100 mL-Autoklaven 12 h auf 130 °C. Nach dem Abkühlen spülte man den Autoklaveninhalt mit Methanol aus, entfernte das Lösungsmittel i.Vak., verrieb den Rückstand mit Essigester, filtrierte von Unlöslichem (6.5 g) ab. entfernte das Lösungsmittel i. Vak, gab den öligen Rückstand auf eine Nutsche mit Kieselgel und wusch mit Heptan / Essigester 1:1 nach. Man entfernte das Lösungsmittel des Filtrats und erhielt 10.4 g 3-Hydroxy-5-methoxy-ani1in als rotes Öl.

b) Diese Verbindung (10.4 g, 75.0 mmol) acetylierte man in 100 mL Methylenchlorid in Gegenart von 0.1 g 4-Dimethylaminopyridin mit 100 mL Acetanhydrid 12 h bei Raumtemperatur. Man entfernte das Lösungsmittel i.Vak, gab zum Rückstand (hauptsächlich Diacetylverbindung) 200 mL Methanol und 20 mL gesättigte Natriumcarbonat-Lösung und rührte 3 h bei Raumtemperatur. Man entfernte das Lösungsmittel i. Vak., gab 250 mL Wasser zu. säuerte mit konz. Salzsäure an und extrahierte mit Essigester. Entfernen des Lösungsmittels lieferte 11 g (81%) N-(3-Hvdroxy-5-methoxy-phenyl)-acetamid mit Fp. 126 °C.

c) Diese Verbindung (11.0 g, 61.0 mmol) alkylierte man in 100 mL trockenem Dimethylformamid in Gegenwart von 9.1 g (65 mmol) Kaliumcarbonat mit 6.9 mL (65 mmol) Chloressigsäureethylester 8 h bei 60 °C. Man verdünnte mit Wasser. säuerte mit Salzsäure an und extrahierte mit Essigester. Die organische Phase extrahierte man mit Wasser. trocknete die organische Phase über Magnesiumsulfat. filtrierte und entfernte das Lösungsmittel i.Vak. Man erhielt 10.8 g (66%) 2-(3-Acetamido-5-methoxy-phenoxy)-essigsäure-ethylester als Öl.

d) Diese Verbindung (10.8 g, 40 mmol) in 70 mL Ethanol rührte man 4 h mit 30 mL 2 N Natronlauge, entfernte das Lösungsmittel i.Vak., versetzte mit Wasser und säuerte an. Man saugte den Niederschlag (5.5 g Carbonsäure) ab, löste in 50 mL Ethanol, gab 50 mL 10 N Natronlauge zu und erhitzte 8 h unter Rückfluß zum Sieden. Man säuerte mit konz. Salzsäure an, entfernte das Lösungsmittelte i. Vak., gab 100 mL Methanol zu und rührte 12 h bei Raumtemperatur. Man entfernte das Lösungsmittel i. Vak digerierte mit Essigester. saugte ab und erhielt 5.6

g 2-(3-Amino-5-methoxy-phenoxy)-essigsäuremethylester (MS. m/e = 211).

e) Wie in Beispiel la) beschrieben, erhielt man daraus durch Umsetzung mit 4-Fluorbenzolsulfonylchlorid 2-[3-(4-Fluorbenzolsulfonylamino)-5-methoxy-phenoxy)-essigsäure-methylester als Öl (MS (m/e) = 369).

f) Wie in Beispiel 1b) beschrieben erhielt man daraus in 95% Ausbeute 2-[3-(4-Fluorbenzolsulfonylamino)-5-methoxy-phenoxy)-essigsäure mit dem Fp. 161 °C.

g) Wie in Beispiel 1c) beschrieben erhielt man daraus in 58% Ausbeute N-(4-Pyridinyl)-2-[3-(4-fluorbenzolsulfonylamino)-5-methoxy-phenoxy)-acetamid mit dem Fp. 161 °C.

h) Wie in Beispiel 23 beschrieben, erhielt man daraus in 76% Ausbeute die Titelverbindung mit dem Fp. 62 °C.

**Beispiel 50**

N-{3-[2-(Pyridin-4-ylamino)-ethoxy]-phenyl}-2-chlorbenzolsulfonamid

stellte man analog Beispiel 1 in 58% Ausbeute her. Schmp. 221 - 223 °C.

**Beispiel 51**

N-Methyl-N-{3-[2-(pyridin-4-ylamino)-ethoxy]-phenyl}-2-chlorbenzolsulfonamid

stellte man analog Beispiel 12 in 22% Ausbeute her. Schmp. 188 - 190 °C.

**Beispiel 52**

N-2-Propyl-N-{3-[2-(pyridin-4-ylamino)-ethoxy]-phenyl}-benzolsulfonamid

stellte man analog Beispiel 12 in 47% Ausbeute her. Öl. MS (m/e) = 411.

**Beispiel 53**

N-Methyl-N-{3-[2-(pyridin-4-ylamino)-ethoxy]-phenyl}-2-thiophensulfonamid

stellte man analog Beispiel 12 in 12 % Ausbeute her. Schmp. 179 - 181 °C.

**Beispiel 54**

N-{5-Methyl-3-[2-(pyridin-4-ylamino)-ethoxy]-phenyl}-1-naphthalinsulfonamid Hydrochlorid

stellte man analog Beispiel 17 in 14% Ausbeute her. Schmp. 215 - 218 °C.

**Beispiel 55**

N-{5-Methyl-3-[2-(pyridin-4-ylamino)-ethoxy]-phenyl}-2-thiophensulfonamid Hydrochlorid

stellte man analog Beispiel 17 in 24% Ausbeute her. Schmp. 252-254 °C.

**Beispiel 56**

N-{5-Methyl-3-[2-(pyridin-4-ylamino)-ethoxy]-phenyl}-2-chlorbenzolsulfonamid Hydrochlorid

stellte man analog Beispiel 17 in 42% Ausbeute her. Schmp. 254 - 258 °C.

**Beispiel 57**

N-Methyl-N-{5-methyl-3-[2-(pyridin-4-ylamino)-ethoxy]-phenyl}-1-chlorbenzolsulfonamid Hydrochlorid

a) 96 g (0.78 mol) 3-Hydroxy-5-methyl-anilin (F. Wessely, H. EibeL G. Friedrich, Monatshefte Chem 83, 24 - 30, (1952)) in 1.2 L Dioxan und 840 mL Wasser versetzte man mit 420 mL 2 N Natronlauge und unter Eiskühlung mit 171 g (0.78 mol) Di-tert.butyl-dicarbonat. Man rührte 12 h bei Raumtemperatur, entfernte das Lösungsmittel i.Vak. säuerte unter Eiskühlung bis aufpH = 2 - 3 an und extrahierte mit Essigester. Man trocknete die organische Phase über Natriumsulfat, filtrierte und entfernte das Lösungsmittel i. Vak. Man erhielt 174 g (quantitativ) N-(tert.Butyloxycarbonyl)-3-hydroxy-5-methyl-anilin als Öl. MS (m/e) = 223.

b) 132 g (0.59 mol) dieser Verbindung in 400 mL trockenem Dimethylformamid. 90 g (0.65 mol) Kaliumcarbonat und 69 mL (0.65 mol) Chloressigsäure-ethylester erhitzte man 3 h auf 70 °C. Man schüttete in 1 L Eiswasser, extrahierte mit Essigester, trocknete die organische Phase über Natriusulfat, filtrierte und entfernte das Lösungsmittel i.Vak. Man erhielt 174 g (95%) 2-(3-tert.butyloxycarbonylamino-5-methyl-phenoxy)-essigsäure-ethylester als Öl. MS (m/e) = 309.

c) 174 g (0.562 mol) dieser Verbindung versetzte man unter Eiskühlung mit 200 mL Trifluoressigsäure. rührte 2 h bei Raumtemperatur und enfernte das Lösungsmittel i.Vak. Den Rückstand versetzte man mit 2 N Salzsäure, extrahierte mit Essigester, stellte die wäßrige Phase alkalisch mit Natronlauge und extrahierte mit Essigester. Man trocknete die organische Phase mit Natriumsulfat, filtrierte und entfernte das Lösungsmittel i.Vak. Man erhielt 87.5 g (74%) 2-(3-Amino-5-methyl-phenyloxy)-essigsäure-ethylester als Öl, MS (m/e) = 209.

d) Wie in Beispiel 17a) beschrieben. setzte man diese Verbindung mit 2-Chlorbenzolsulfonylchlorid um und erhielt in 56% Ausbeute 2-[3-(2-Chlorbenzolsulfonylamino)-5-methyl-phenoxy]-essigsäure-ethylester mit dem Fp. 133 - 137 °C.

e) Diese Verbindung methylierte man wie in Beispiel 12a) beschrieben und erhielt in quantitativer Ausbeute N-Methyl-2-[3-(2-chlorbenzolsulfonylamino)-5-methylphenoxy]-essigsäure-ethylester. Öl. MS (m/e) = 398.

f) Diese Verbindung verseifte man wie in Beispiel 1b) beschrieben und erhielt in quantitativer Ausbeute N-Methyl-2-[3-(2-chlorbenzolsulfonylamino)-5-methylphenoxy]-essigsäure. Fp. 113 - 115 °C.

g) Diese Verbindung setzte man mit 4-Aminopyridin um wie in Beispiel 1c) beschrieben und erhielt in 68% Ausbeute 2-{3-[(2-Chlorbenzolsulfonyl)-methyl-amino]-5-methylphenoxy}-N-pyridin-4-yl-acetamid als Öl.

h) Diese Verbindung reduzierte man wie in Beispiel ld) beschrieben und erhielt die Titelverbindung in 41% Ausbeute. Fp. 213 - 215 °C.

**Beispiel 58**

N-Methyl-N-{5-methyl-3-[2-(pyridin-4-ylamino)-ethoxy]-phenyl}-benzolsulfonamid Hydrochlorid

Die Verbindung aus Beispiel 57 (0.86 g, 2 mmol) hydrierte man in 30 mL Ethanol in Gegenwart von 0.3 g 10% Palladium auf Kohle bei Raumtemperatur und Normaldruck. Wasserstoffaufnahme 55 mL. Man filtrierte und entfernte das Lösungsmittel i.Vak. Man digerierte mit Ether und erhielt 0.75 g (86%) der Titelverbindung mit dem FP. 182 - 184 °C.

**Beispiel 59**

N-{2-Methoxy-5-[2-(pyridin-4-ylamino)-ethoxy]-phenyl}-benzolsulfonamid

a) Zu 8.5 g (50 mmol) 2-Hydroxy-4-nitroanisol und 13.8 g (100 mmol) Kaliumcarbonat in 120 mL Acetonitril tropfte man im Eisbad 8.4 g (50 mmol) Bromessigsäure-ethylester. Man rührte 12 h bei Raumtemperatur. entfernte das Lösungsmittel i. Vak.. versetzte den Rückstand mit Wasser und extrahierte mit Essigester. Man trocknete die organische Phase mit Natriumsulfat, filtrierte und entfernte das Lösungsmittel i. Vak. Man erhielt 12.7 g (quantitativ) 2-(2-Methoxy-5-nitro-phenoxy)-essigsäure-ethylester als Öl. MS (m/e) = 255.

b) Diese Verbindung (12.1 g, 47 mmol) hydrierte man in 300 mL Methanol in Gegenwart von 5 g Raney-Nickel bei

Normaldruck und Raumtemperatur. Nachdem 3.4 L Wasserstoff aufgenommen waren. filtrierte man und entfernte das Lösungsmittel i.Vak. Man erhielt 10.7 g (quantitativ) 2-(2-Methoxy-5-amino-phenoxy)-essigsäure-ethylester als Öl. MS (m/e) = 225.

c) Diese Verbindung (10.7 g, 47 mmol) setzte man mit Benzolsulfonylchlorid um wie in Beispiel 1a) beschrieben und erhielt 17.2 g (quantitativ) 2-(2-Methoxy-5-benzolsulfonylamino-phenoxy)-essigsäure-ethylester.MS (m/e) = 413.

d) Aus dieser Verbindung erhielt man nach der Vorschrift von Bsp. Ib) 11.8 g (74%) 2-(2-Methoxy-5-benzolsulfonylamino-phenoxy)-essigsäure. MS (m/e) = 337.

e) Aus dieser Verbindung erhielt man nach der Vorschrift von Bsp. Ic) 5 g (35%) N-(4-Pyridinyl)-2-(2-methoxy-5-benzolsulfonylamino-phenoxy)-acetamid mit dem Fp. 179 °C.

f) Aus dieser Verbindung erhielt man nach der Vorschrift von Bsp. 23 die Titelverbindung nüt dem Fp. 188 - 189 °C.

Beispiel 60

N-{2-Methyl-5-[2-(pyridin-4-ylamino)-ethoxy]-phenyl}-benzolsulfonamid

stellte man analog zu Beispiel 59 her, nur daß man in Stufe a) an Stelle von 2-Hydroxy-4-nitranisol 2-Hydroxy-4-nitrotoluol verwendete. Diese Vorstufe stellte man wie folgt her: 100 g 2-Amino-4-nitrotoluol rührte man bei 50 °C in 300 mL konz. Schwefelsäure ein, bis alles gelöst war (30 min), gab 2.5 kg Eis zu, kühlte auf-15 °C ab und tropfte eine Lösung von 50 g Natriumnitrit in 200 mL Wasser so zu, daß die Temperatur 0 °C nicht überschritt. Diese Lösung gab man in eine unter Rückfluß siedende Mischung aus 500 mL konzentrierter Schwefelsäure und 1 L Wasser. Man erhitzte 1 h unter Rückfluß zum Sieden. ließ 12 h stehen und saugte den Niederschlag ab. Man erhielt 87.1 g (87%) 2-Hydroxy-4-nitrotoluol mit dem Fp. 117 - 120 °C.

a) 2-(2-Methyl-5-nitro-phenoxy)-essigsäure-ethylester als Öl. MS (m/e) = 239.

b) 2-(2-Methyl-5-amino-phenoxy)-essigsäure-ethylester als Öl. MS (m/e) = 209.

c) 2-(2-Methyl-5-benzolsulfonylamino-phenoxy)-essigsäure-ethylester. Fp. 78 - 83 °C.

d) 2-(2-Methyl-5-benzolsulfonylamino-phenoxy)-essigsäure. Fp. 157 - 160 °C.

e) N-(4-Pyridinyl)-2-(2-methyl-5-benzolsulfonylamino-phenoxy)-acetamid mit dem Fp. 157 - 161 °C.

f) Titelverbindung mit dem Fp. 179 - 180 °C.

**Beispiel 61**

Benzolsulfonsäure-5-methyl-3-[2-(pyridin-4-ylamino)-ethoxy]-phenylester

a) 7.1 g (50 mmol) 5-Methyl-resorcin, 10 g (100 mmol) Kaliumhydrogencarbonat und 12.6 g (55 mmol) Bromessigsäurebenzylester erhitzte man in 70 mL Acetonitril 24 h unter Rückfluß zum Sieden. Man entfernte das Lösungsmittel i. Vak., gab Wasser zum Rückstand und extrahierte mit Ether., extrahierte die Etherphase dreimal mit 0.1 n Natronlauge, trocknete die Etherphase über Natriumsulfat, filtrierte und entfernte das Lösungsmittel i. Vak. Den Rückstand (7.75 g) trennte man über Kieselgel mit Isohexan / Essigester (9:1) und erhielt 4.0 g (29%) 2-(3-Hydroxy-5-methyl-phenoxy)-essigsäure-benzylester als Öl. MS (m/e) = 272.

b) 2.0 g (7.5 mmol) dieser Verbindung setzte man mit Benzolsulfonylchlorid um analog Bsp 1a) und erhielt 2.1 g (68%) 2-(3-Benzolsulfonyloxy-5-methyl-phenoxy)-essigsäurebenzylester als Öl. MS (m/e) = 412.

c) 2.0 g ( 5mmol) dieser Verbindung in 150 mL Methanol hydrierte man in Gegenwart von 0.5 g 10% Palladium aufKohle 1 h bei Raumtemperatur und Normaldruck. bis 140 mL Wasserstoff aufgenommen waren. Man filtrierte, gab Ether zu und extrahierte dreimal mit Natriumbicarbonat-Lösung. Man säuerte die Natriumbicarbonatlösung mit 2 N Schwefelsäure an, extrahierte mit Ether, trocknete. entfernte das Lösungsmittel i. Vak. und erhielt 600 mg

(38%) 2-(3-Benzolsulfonyloxy-5-methyl-phenoxy)-essigsäure. MS (m/e) = 322.

d) 0.6 g (2 mmol) dieser Verbindung setzte man mit 4-Aminopyridin um analog Bsp. 1c) und erhielt N-(Pyridin-4-yl)-2-(3-Benzolsulfonyloxy-5-methyl-phenoxy)-acetamid (16%). MS (m/e) = 392.

e) Daraus erhielt man die Titelverbindung anaolg Bsp. 23. Fp. 144 - 146 °C.

**Beispiel 62**

2-Chlorbenzolsulfonsäure-5-methyl-3-[2-(pyridin-4-ylamino)-ethoxy]-phenylester

stellte man analog dem Bsp. 61 her. Fp 156 - 158 °C. Zwischenstufen: 2-[3-(2-Chlorbenzolsulfonyloxy)-5-methyl-phenoxy]-essigsäure: Fp. 157 - 161 °C. N-(Pyridin-4-yl)-2- [3-[2-chlorbenzolsulfonyloxy)-5-methyl-phenoxy]-acetamid. MS (m/e) = 432.

**Beispiel 63**

4-Fluorbenzolsulfonsäure-5-methyl-3-[2-(pyridin-4-ylamino)-ethoxy]-phenylester

stellte man analog Bsp. 61 her. nur daß man in Stufe b) 4-Fluorbenzolsulfonylchlorid benutzte. Fp. 161 - 163 °C.

**Beispiel 64**

1-Naphthalinsulfonsäure-5-methyl-3-[2-(pyridin-4-ylamino)-ethoxy]-phenylester

stellte man analog Bsp. 61 her. nur daß man in Stufe b) 1-Naphthalinsulfonylchlorid benutzte. Fp. 95 - 99 °C.

**Beispiel 65**

2-Thiophensulfonsäure-5-methyl-3-[2-(pyridin-4-ylamino)-ethoxy]-phenylester

a) 24.8 g (200 mmol) 5-Methylresorcin, 43.8 g (240 mmol) 2-Thiophensulfonylchlorid und 1.5 g festes Natriumhydrogencarbonat in 200 mL Wasser überschichtete man mit 100 mL Ether und hielt mit einer Dosiergerät mit gesättigter Natriumbicarbonatlösung pH = 7.2 ein. Man rührte 12 h bei Raumtemperatur bei pH = 7.2, trennte die Wasserphase ab, trocknete die Etherphase mit Natriumsulfat, filtrierte und entfernte das Lösungsmittel i.Vak. Man erhielt in quantitativer Ausbeute Thiophensulfonsäure-3-hydroxy-5-methylphenyl-ester als Öl. MS (m/e) = 270.

b) Diese Verbindung setzte man mit Bromessigsäure-ethylester analog Bsp. 18a um und erhielt 2-[3-(2-Thiophensulfonyloxy)-5-methyl-phenoxy]-essigsäure-ethylester. MS (m/e) = 356.

c) Aus dieser Verbindung erhielt man analog Bsp. 1b) 2-[3-(2-Thiophensulfonyloxy)-5-methyl-phenoxy]-essigsäure mit Fp. 142 - 143 °C.

d) Aus dieser Verbindung erhielt man anaolg Bsp 1c) N-(Pyridin-4-yl)-2-[3-(2-Thiophensulfonyloxy)-5-methyl-phenoxy]-acetamid. Fp. 136 - 138 °C.

e) Aus dieser Verbindung erhielt man die Titelverbindung mit dem Fp. 176 °C.

**Beispiel 66**

(2-Benzyloxycarbonyl-benzolsulfonsäure)-5-methyl-3-[2-(pyridin-4-ylamino)-ethoxy]-phenylester

a) Analog Beispiel 61a) setzte man 5-Methylresorcin mit Bromessigsäure-ethylester umj und erhielt 2-(3-Hydroxy-5-methyl-phenoxy)-essigsäure-ethylester als Öl. MS (m/e) = 210.

b ) Analog Bsp. 61b) erhielt man daraus durch Umsetzung mit 2-Benzyloxycarbonyl-benzolsulfonylchlorid 2-[3-(2-Benzyloxycarbonyl)-benzolsulfonyloxy-5-methylphenoxy]-essigsäure-ethylester als Öl. MS (m/e) = 484.

c) Diese Verbindung verseifte man 4 h bei Raumtemperatur analog Bsp. 1b) und erhielt 63% 2-[3-(2-Benzyloxycarbonyl-benzolsulfonyloxy)-5-methyl-phenoxy]-essigsäure. MS (m/e) = 456.

d) Daraus erhielt man N-(Pyridin-4-yl)-2-[3-(2-benzyloxycarbonyl-benzolsulfonyloxy)-5-methyl-phenoxy)-acetamid.MS (m/e) = 532.

e) Daraus erhielt man die Titelverbindung analog Bsp. 23. MS (m/e) = 518.

**Beispiel 67**

(2-Carboxy-benzolsulfonsäure)-5-methyl-3-[2-(pyridin-4-ylamino)-ethoxy]-phenylester

2.5 g (1 mmol) der Verbindung aus Bsp. 66 hydrierte man in 100 mL methanolischer Ammoniaklösung in Gegenwart von 1 g 10% Palladium auf Kohle bei Normaldruck und Raumtemperatur. Man filtrierte. entfernte das Lösungsmittel i. Vak.. verrieb den Rückstand mit IsopropanoL saugte ab und kristallisierte aus Ethanol um. Man erhielt 0.4 g (14%) der Titelverbindung mit dem Fp. 189 °C.

**Beispiel 68**

(2-Methyl-benzolsulfonsäure)-5-methyl-3-[2-(pyridin-4-ylamino)-ethoxy]-phenylester

stellte man analog Bsp 61 her. Fp. 152 - 154 °C. Vorstufe: N-(Pyridin-4-yl)-2-[3-(2-methylbenzolsulfonyloxy)-5-methyl-phenoxy]-acetamid. Fp. 158 - 160 °C.

**Beispiel 69**

(2-Methoxy-benzolsulfonsäure)-5-methyl-3-[2-(pyridin-4-ylamino)-ethoxy]-phenylester

stellte man analog Bsp 61 her. Fp. 116 - 119 °C. Vorstufe: N-(Pyridin-4-yl)-2-[3-(2-methoxybenzolsulfonyloxy)-5-methyl-phenoxy]-acetamid. Fp. 156 - 159 °C.

**Beispiel 70**

(2-Nitro-benzolsulfonsäure)-5-methyl-3-[2-(pyridin-4-ylamino)ethoxy]-phenylester

stellte man analog Bsp 61 her. Fp. 137-140 °C. Vorstufe: N-(Pyridin-4-yl)-2-[3-(2-nitrobenzolsulfonyloxy)-5-methyl-phenoxy]-acetamid. MS (m/e) = 453.

**Beispiel 71**

(2-Amino-benzolsulfonsäure)-5-methyl-3-[2-(pyridin-4-ylamino)-ethoxy]-phenylester

1.0 g (2.33 mmol) der Verbindung aus Bsp. 70 hydrierte man in 40 mL Methanol in Gegenwart von 1 g Raney-Nickel 1.5 h bei Raumtemperatur und Normaldruck. Man filtrierte, entfernte das Lösungsmittel i. Vak, versetzte den Rückstand mit 25 mL Tetrahydrofuran und 25 mL Ether. extrahierte mit 0.05 M Natronlauge, trocknete die organische Phase. filtrierte und entfernte das Lösungsmittel i.Vak. Man erhielt 0.5 g (54%) der Titelverbindung mit dem Fp. 168-171 °C.

**Beispiel 72**

2-Chlorbenzolsulfonsäure-5-methyl-3-[2-(N-methyl-pyridin-4-ylamino)-ethoxy]-phenylester

a) Durch Umsetzung von 2-[3-(2-Chlorbenzolsulfonyloxy)-5-methyl-phenoxy]-essigsäure nach Beispiel 62 mit 4-Methylamino-pyridin erhielt man N-Methyl-N-(pyridin-4-yl)-2-[3-[2-chlorbenzolsulfonyloxy)-5-methyl-phenoxy]-acetamid. MS (m/e) = 447.

b) Daraus erhielt man analog Bsp. 23 die Titelverbindung mit dem Fp. 152 - 161 °C.

**Beispiel 73**

Benzolsulfonsäure-5-chlor-3-[2-pyridin-4-ylamino)-ethoxy]-phenylester

a) 98.8 g (0.57 mol) 5-Chlorresorcin-dimethylether und108 mL (1.14 mol) Bortribromid in 400 mL Methylenchlorid rührte man 72 h bei Raumtemperatur. Man extrahierte mit Wasser. extrahierte die wäßrige Phase mit n-Butanol entfernte das n-Butanol zum größten Teil i. Vak. und ließ 12 h bei 4 °C kristallisieren. Man erhielt 19.5 g (24%) 5-Chlor-resorcin mit dem Fp. 70 - 71 °C.

b) 3.0 g (21 mmol) dieser Verbindung in 50 mL Wasser überschichtete man mit 20 mL Ether, gab gesättigte Natriumhydrogencarbonat-Lösung bis zu pH = 5.2 zu. Man gab unter Einhaltung des pH-Wertes langsam 8.6 mL (21 mmol) Benzolsulfonylchlorid zu, erhöhte den pH auf 7.0 und rührte unter Konstanthaltung des pH-Wertes 48 h bei Raumtemperatur. Man extrahierte mit Ether, extrahierte die Etherphase mit 0.1 N Natronlauge. säuerte die Natronlauge mit 2 N Schwefelsäure an und extrahierte dreimal mit Ether. Man entfernte das Lösungsmittel i. Vak und erhielt 1.7 g (28%) Benzolsulfonsäure-3-chlor- 5-hydroxy-phenylester. MS (m/e) = 284.

c) Diese Verbindung setzte man analog Bsp. 18a) mit Bromessigsäure-ethylester um und erhielt in 95% Ausbeute 2-[3-Chlor-5-(phenylsulfonyloxy)-phenoxy]-essigsäureethylester. MS (m/e) = 370.

d) Diese Verbindung verseifte man analog zu Bsp. 1b) in 80% Ausbeute zu 2-[3-Chlor-5-(phenylsulfonyl-oxy)-phenoxy]-essigsäure mit dem Fp. 136 - 138 °C.

e) Diese Verbindung setzte man mit 4-Aminpyridin um analog Bsp lc) und erhielt in 70% Ausbeute N-(Pyridin-4-yl)-2-[3-chlor-5-(phenylsulfonyl-oxy)-phenoxy]-acetamid mit dem Fp. 173 - 176 °C.

f) Diese Verbindung reduzierte man analog Bsp.23) und erhielt die Titelverbindung mit dem Fp. 144 - 146 °C. Hydrochlorid: Fp. 173 - 176 °C.

**Beispiel 74**

2-Chlorbenzolsulfonsäure-5-chlor-3-[2-(pyridin-4-ylamino)-ethoxy]-phenylester

stellte man analog Bsp. 73 her. Zwischenstufen:

b) 2-Chlorbenzolsulfonsäure-3-chlor-5-hydroxy-phenylester. Fp. 99 - 105 °C.

c ) 2-[3-Chlor-5-(2-chlor-phenylsulfonyloxy)-phenoxy]-essigsäure-ethylester als Öl. MS (m/e) = 405.

d) 2-[3-Chlor-5-(2-chlor-phenylsulfonyloxy)-phenoxy]-essigsäure. Fp. 140 - 142 °C.

e) N-(Pyridin-4-yl)-2-[3-chlor-5-(2-chlorphenylsulfonyloxy)-phenoxy]-acetamid. Fp. 158 - 160 °C.

f) Titelverbindung. Fp. 149 - 150 °C.

**Beispiel 75**

Benzolsulfonsäure-3-[2-(pyridin-4-ylamino)-ethylamino]-phenylester

a) 15 g (54 mmol) Benzolsulfonsäure-(3-nitro-phenylester) in 200 mL Methanol hydrierte man in Gegenwart von 2.5 g 10 % Palladium auf Kohle bei Normaldruck und Raumtemperatur. Man filtrierte und entfernte das Lösungsmittel i. Vak. Den Rückstand (13 g Benzolsulfonsäure-(3-amino-phenylester)), 4.3 g Natriumacetat und 8.7 g Bromessigsäure-ethylester in 10 mL Ethanol erhitzte man 12 h unter Rückfluß zum Sieden. Man gab Wasser zu und extrahierte mit Ether. Man entfernte den Ether i. Vak. und erhielt 17.3 g (99%) 2-[3-(Phenylsulfonyloxy)-phenylamino]-essigsäure-ethylester als Öl. MS (m/e) = 335.

b) Diese Verbindung verseifte man analog Bsp. 1b) zu 2-[3-(Phenylsulfonyloxyphenylamino]-essigsäure. Ausbeute 65%. MS (m/e) = 307.

c) Diese Verbindung setzte man mit 4-Aminopyridin um analog Bsp. 1c) zu N-(Pyridin-4-yl)-2-[3-(phenylsulfonyloxy)-phenylamino]-acetamid. Öl. MS (m/e) = 383.

d) Daraus erhielt man die Titelverbindung analog zu Bsp. 23. Man löste 0.6 g der als Öl angefallenen Verbindung in 10 mL Essigester und versetzte mit einer Lösung von 220 mg Cyclohexansulfaminsäure in 10 mL Essigester. Man gab einige Tropfen Isopropanol zu und ließ kristallisieren. Man erhielt 0.3 g des Cyclaminats der Titelverbindung mit dem Fp. 106 - 111 °C.

**Beispiel 76**

Benzolsulfonsäure-3-methyl-5-[2-(pyridin-4-ylamino)-ethylamino]-phenylester

a) 12.3 g (100 mmol) 3-Hydoxy-5-methyl-anilin (siehe Bsp. 57) und 25.1 g (170 mmol) Phthalsäureanhydrid in 250 mL Essigsäure erhitze man 1 h unter Rückfluß zum Sieden. Man gab 250 mL Wasser zu, filtrierte heiß, gab 250 mL Wasser zum Filtrat und ließ kristallisieren. Man filtrierte, löste den Niederschlag in 400 mL heißem Methanol, versetzte mit Aktivkohle, entfernte das Wasser i. Vak. und erhielt 21.7 g (86%) 3-Phthalimido-5-methyl-phenol mit dem Fp. 170 - 175 °C.

b) Analog Bsp. 1a) erhielt man daraus in quantitativer Ausbeute Benzolsulfonsäure-3-methyl-5-phthalimido-phenylester. MS (m/e) = 393.

c) 3.9 g (10 mmol) dieser Verbindung und 0.7 mL (15 mmol) Hydrazinhydrat in 10 mL Ethanol und 30 mL Methylenchlorid rührte man 12 h bei Raumtemperatur, gab 4 mL konz. Salzsäure zu. rührte 2 h bei Raumtemperatur, filtrierte, entfernte das Lösungsmittel i. Vak, gab zum Rückstand 2 N Natronlauge und extrahierte mit Ether, wusch die Etherphaase mit Wasser und gesättigter Kochsalz-Lösung, entfernte den Ether i. Vak und erhielt 2.5 g (96%) Benzolsulfonsäure-3-methyl-5-amino-phenylester. MS (m/e) = 263.

d) 2.5 g (9.5 mmol) dieser Verbindung setzte man analog Bsp. la) mit Tosylchlorid um und erhielt in quantitativer Ausbeute Benzolsulfonsäure-3-methyl-5-(4-methylphenylsulfonylamino)-phenylester. MS (m/e) = 417.

e) Diese Verbindung alkylierte man analog Bsp. 18a) mit Bromessigsäure-ethylester und erhielt 5 g (quantitative Ausbeute) [4-Methyl-benzolsulfonyl-(3-benzolsulfonyloxy-5-methyl-phenyl)-amino]-essigsäure-ethylester. MS (m/e) = 503.

f) 5 g (10 mmol) dieser Verbindung in 60 mL 6 N Salzsäure erhitzte man 6 h unter Rückfluß zum Sieden. Man entfernte das Lösungsmittel i. Vak., gab Wasser zu, neutralisierte mit Natriumhydrogencarbonat, extrahierte mit Essigester. stellte die wäßrige Phase mit 2 N Salzsäure aufpH = 3 ein und extrahierte mit Essigester. Man entfemte den Essigester i. Vak. und erhielt 2 g (62%) (3-Benzolsulfonyloxy-5-methylphenyl)-amino-essigsäure. MS (m/e) = 321.

g) Diese Verbindung setzte man analog Bsp. 1c) um und erhielt in 12% Ausbeute N-(Pyridin-4-yl)-benzolsulfonyloxy-5-methyl-phenyl)-amino-acetamid. MS (m/e) = 397.

h) Aus dieser Verbindung erhilet man analog Bsp. 23) die Titelverbindung als Öl. MS (m/e) = 383.

**Beispiel 77**

N-{3-[2-(Pyridin-4-ylamino)-ethylamino]-phenyl}-benzolsulfonamid

a) 13.8 g (100 mmol) 3-Nitro-anilin, 12.3 g Natriumacetat (150 mmol) und 25 g (150 mmol) Bromessigsäure-ethylester in 5 mL Dimethylsulfoxid erhitzte man 48 h auf 80 °C. Man goß auf400 mL 0.5 N Salzsäure, gab 15 mL Isohexan und 10 mL Ether zu und ließ kristallisieren. Man filtrierte und erhielt 18.7 g (84%) 3-Nitro-phenylamino-essigsäure-ethylester. Fp. 92 °C.

b) Analog Bsp. 1b) erhielt man daraus in 90% Ausbeute 3-Nitro-phenylamino-essigsäure. Fp. 159 - 162 °C.

c) Analog Bsp. Ic) erhielt man daraus in 89% Ausbeute N-(Pyridin-4-yl)-3-nitrophenylamino-acetamid. Fp. 196 - 198 °C.

d) 10.4 g (38 mmol) dieser Verbindung hydrierte man in 200 mL Methanol und 100 mL Essigester in Gegenwart von 10 g Raney-Nickel bei Normaldruck und Raumtemperatur. Man filtrierte, entfernte das Lösungsmittel i.Vak. und erhielt 7.7 g (82%) N-(Pyridin-4-yl)-(3-amino-phenylamino)-acetamid. MS (m/e) = 292.

e) Aus dieser Verbindung erhielt man analog Bsp. la) in 68% Ausbeute N-(Pyridin-4-yl)-(3-phenylsulfonylamino-phenylamino)-acetamid. MS (m/e) = 382.

f) Daraus erhielt man analog Bsp. 23) die Titelverbindung in 40% Ausbeute. MS (m/e) = 368.

**Beispiel 78**

N-{3-[2-(Pyridin-4-ylamino)-ethylamino]-phenyl}-thiophen-2-sulfonamid

a) Durch Umsetzung der Verbindung aus Bsp. 77d) mit 2-Thiophensulfonylchlorid analog Bsp. 1a ) erhielt man N-(Pyridin-4-yl)-[3-(thiophen-2-ylsulfonylamino)-phenylamino]-acetamid in 59 % Ausbeute. MS (m/e) = 388.

b) Daraus erhielt man analog Bsp. 23) die Titelverbindung in 24% Ausbeute. Fp. 196 - 198 °C.

**Beispiel 79**

N-{3-[2-(Pyridin-4-ylamino)-ethylamino]-5-trifluormethyl-phenyl}-benzolsulfonamid

a) Zu 24.5 g (100 mmol) 3,5-Dinitrobenzotrifluorid in 180 mL siedendem Eisessig gab man portionsweise 15 g (270 mmol) Eisenpulver. Man goß auf Wasser, extrahierte mit Essigester und neutralisierte die Essigesterphase mit festem Natriumbicabrbonat. Man filtrierte, entfernte das Lösungsmittel i. Vak., gab den Rückstand (26.3 g) auf Kieselgel und eluierte mit Isohexan / Essigester ( 8 : 2). Man erhielt 13.0 g (63%) 3-Nitro-5-trifluormethyl-anilin mit dem Fp. 80 - 84 °C.

b) 5.0 g (24 mmol) dieser Verbindung löste man in 20 mL Schwefelsäure und 17 mL Wasser, kühlte auf 0°C und gab eine Lösung von 1.9 (27 mmol) Natriumnitrit in 10 mL Wasser zu. Die kalte Lösung gab man in 250 mL siedende, konzentrierte Kupfersulfatlösung. Nach Beendigung der Stickstoffentwicklung extrahierte man mit Ether. Die Etherphase extrahierte man mit 0.05 N Natronlauge, säuerte die wäßrige Phase mit verdünnter Schwefelsäure an und extrahierte mit Ether. Man entfernte den Ether i. Vak. und erhielt 3.4 g (68%) 3-Nitro-5-trifluormethyl-phenol mit dem Fp. 82 - 84 °C.

c) 36.7 g (600 mmol) Ethanolamin und 80.7 g (550 mmol) Phthalsäureanhydrid in 290 mL Toluol erhitzte man 2 h unter Rückfluß am Wasserabscheider. Nach Abscheidung von 9.3 mL Wasser ließ man abkühlen, filtrierte und erhielt 95.1 g (90%) N-(2-Hydroxyethyl)-phthalimid mit dem Fp. 128 - 132 °C.

d) 28.8 g (150 mmol) dieser Verbindung und 42.9 g (225 mmol) Tosylchlorid in 200 mL Pyridin rührte man 3 h bei Raumtemperatur, säuerte mit 2 N Salzsäure an und extrahierte mit Essigester. Man entfernte den Essigester i. Vak. und erhielt 48.3 g (85%) 4-Toluolsulfonsäure-(2-phthalimido-ethyl)-ester mit dem Fp. 144 - 148 °C.

e) 1.7 g (12.5 mmol) dieser Verbindung, 2.6 g (12.5 mmol) der Verbindung 79c) und 4.1 g Kaliumcarbonat in 80 mL Dimethylsulfoxid rührte man 12 h bei 50 °C. Man goß aufEis, extrahierte mit Essigester. wusch den Essigester mit 0.01 N Natriumhydroxid und gesättigter Kochsalz-Lösung, entfernte den Essigester i. Vak. und erhielt 1.9 g (40%) N-{2-[2-(3-Nitro-5-trifluormethyl-phenoxy)-ethyl]}-phthalimid mit dem Fp. 146 - 148 °C.

f) Analog Bsp.76c) erhielt man daraus quantitativ 2-(3-Nitro-5-trifluormethyl-phenoxy)-ethylamin. MS (m/e) = 250.

g) 1.0 g (4 mmol) dieser Verbindung, 1.15 g (4.4 mmol) 4-Nitro-tetrachlor-pyridin (M. Roberts, H. Suschitzky, J. Chem. Soc. C 1968, 2844 - 2848) und 0.48 mL (4.4 mmol) N-Methyl-morpholin in 20 mL Dioxan rührte man 3 h bei Raumtemperatur. Man gab Wasser zu, extrahierte mit Essigester, wusch den Essigester mit Wasser und gesättigter Kochsalzlösung, entfernte den Essigester i. Vak. und erhielt 1.4 g (75%) N-(Tetrachlorpyridin-4-yl)-2-(3-nitro-5-trifluormethyl-phenoxy)-ethylamin mit dem Fp. 126 - 129 °C.

h) Diese Verbindung reduzierte man analog der Stufe a) und erhielt N-(Tetrachlorpyridin-4-yl)-2-(3-amino-5-trifluormethyl-phenoxy)-ethylamin mit dem Fp. 144 - 146 °C.

i) 0.4 g (0.92 mmol) dieser Verbindung und 0.12 mL (0.92 mmol) Benzolsulfonylchlorid in 5 mL Pyridin rührte man 3 h bei Raumtemperatur, säuerte mit 2 N Salzsäure an, extrahierte mit Essigester, wusch mit gesättigter Kochsalzlösung, entfernte den Essigester i. Vak und erhielt 0.4 g N-{3-[2-(Tetrachlorpyridin-4-ylamino)-ethoxy]-5-trifluormethyl-phenyl}-benzolsulfonamid mit dem Fp. 139 - 143 °C.

j) 0.4 g (0.7 mmol) dieser Verbindung hydrierte man in 50 mL Methanol in Gegenwart von 3.5 mmol Natriummethylat und 0.5 g 10% Palladium aufKohle bei Raumtemperatur und Normaldruck. Man filtrierte, gab Wasser zu und extrahierte mit Essigester. Man entfernte den Essigester i. Vak. und erhielt 0.2 g der Titelverbindung mit dem Fp. 140 - 144 °C.

**Beispiel 80**

3-Methoxy-N-methyl-N-phenyl-5-[2-(pyridin-4-ylamino)-ethoxy]-benzolsulfonamid

a) 92 g (0.4 mol) 3.5-Dinitrobenzoylchlorid und 28.4 g (0.44 mol) Natriumazid in 240 mL Eisessig rührte man 8 h bei Raumtemperatur, gab 400 mL Wasser zu, filtrierte den Niederschlag und erhielt 80.8 g (85%) 3,5-Dinitrobenzoylazid mit dem Fp. 105 °C. (Zers.).

b) 80.8 g (0.34 mol) dieser Verbindung in 500 mL Essigsäureanhydrid erhitzte man vorsichtig bis zum Einsetzen der Gasentwicklung (90 - 100 °C) und hielt 4 h bei dieser Temperatur. Man entfernte das Lösungsmittel i.Vak, digerierte den Rückstand mit Wasser und erhielt 136 g (quantitativ) N-(3,5-Dinitrophenyl)-acetamid Fp. 163 °C.

c) 136 g (0.34 mol) dieser Verbindung erhitzte man in 500 mL Ethanol und 500 mL konzentrierter Salzsäure 3 h unter Rückfluß zum Sieden, filtrierte von Ungelöstem ab, goß das Filtrat auf 2 L Wasser und saugte den gelben Niederschlag ab und erhielt 41.4 g (66%) 3,5-Dinitroanilin. Fp. 140 °C (Zers.).

d) 25 g (137 mmol) dieser Verbindung löste man in 50 mL Eisessig und 100 mL konzentrierter Salzsäure, tropfte bei -5 °C 10.4 g (155 mmol) Natriumnitrit in 20 mL Wasser innerhalb von 5 min zu, rührte noch 15 min bei dieser Temperatur, kühlte die braune Suspension auf-20 °C ab und gab sie innerhalb 15 min zu einer auf 0°C gekühlten und mit Schwefeldioxid gesättigten Lösung von 2.7 g Kupferdichloriddihydrat in 200 mL Eisessig. Man extrahierte mit Essigester, entfernte den Essigester i. Vak. und trocknete bei $10^{-2}$ Torr. Man erhielt 35.4 g (97%) Dinitrobenzolsulfonylchlorid als braunen Feststoff, den man ohne weitere Reinigung einsetzte.

e) Aus 10.2 g (38.2 mmol) dieser Verbindung und 4.5 mL (42 mmol) N-Methyl-anilin erhielt man analog Bsp. 79i) 4.9 g (38%) N-Methyl-N-phenyl-3,5-dinitrobenzolsulfonamid mit dem Fp. 175 - 178 °C.

f) 3.5 g (10.4 mmol) dieser Verbindung in 31 mL 0.4 molarer methanolischer Natriummethylat-Lösung erhitzte man 1 h unter Rückfluß zum Sieden. Man entfernte das Lösungsmittel i.Vak., digerierte den Rückstand mit Essigester und reinigte über eine Kieselgelsäule (100 g Kieselgel). Man eluierte mit Isohexan / Essigester 2 : 1 und erhielt 2.5 g (75%) N-Methyl-N-phenyl-3-methoxy-5-nitro-benzolsulfonamid. Fp. 112 °C.

g) Diese Verbindung hydrierte man analog Bsp 58) und erhielt 2.3 g N-Methyl-N-phenyl-3-methoxy-5-amino-benzolsulfonamid als Öl. MS (m/e) = 292.

h) Zu einer einer auf 0 °C gekühlten Suspension von 2.3 g (7.8 mmol) dieser Verbindung in 10 mL Wasser und 5 mL konzentrierter Schwefelsäure tropfte man eine Lösung von 630 mg (9 mmol) Natriumnitrit in 2 mL Wasser, rührte 2 h bei dieser Temperatur, zersetzte mit Harnstoff erhitzte 15 min auf 110 °C, extrahierte mit Essigester, wusch den Essigester mit 2 N Natronlauge, entfernte das Lösungsmittel i. Vak. und erhielt 300 mg (13%) N-Methyl-N-phenyl-3-methoxy-5-hydoxy-beuzolsulfonamid als Öl. MS (m/e) = 293.

i) 250 mg (0.85 mmol) dieser Verbindung alkylierte man mit 0.14 mL Bromessigester analog Bsp. 18a) und erhielt 360 mg (quant.) [3-Methoxy-5-(methyl-phenyl-sulfamoyl)-phenoxy]-essigsäure-ethylester als Öl. MS (m/e) = 379.

j) Diese Verbindung verseifte man analog Bsp. 1b) und erhielt 300 mg [3-Methoxy-5-(methyl-phenyl-sulfamoyl)-phenoxy]-essigsäure als zähe Masse. MS (m/e) = 351.

k) Aus dieser Verbindung erhielt man analog Bsp. 1c) 120 mg (33%) N-(Pyridin-4-yl)-[3-methoxy-5-(methyl-phenyl-sulfamoyl)-phenoxy]-acetamid Fp. 105 °C.

l) Aus 100 mg dieser Verbindung erhielt man analog Bsp. 1d) 45 mg (46%) der Titelverbindung. MS (m/e) = 413.

**Beispiel 81**

3-Methoxy-N-benzyl-N-phenyl-5-[2-(pyridin-4-ylamino)-ethoxy]-benzolsulfonamid

a) Analog Bsp. 80d) erhielt man aus der Verbindung 80c) und N-Benzylanilin in 65% Ausbeute N-Benzyl-N-phenyl-3,5-dinitro-benzolsulfonamid. Fp. 200 °C.

b) Analog Bsp. 80e) erhielt man daraus in quant. Ausbeute N-Benzyl-N-phenyl-3-methoxy-5-nitro-benzolsulfonamid. Fp. 142 °C.

c) Daraus erhielt man analog 79a) in 56% Ausbeute N-Benzyl-N-phenyl-3-methoxy-5-amino-benzolsulfonamid als zähe Masse. MS (m/e) = 368.

d) Daraus erhielt man analog Bsp. 80g) N-Benzyl-N-phenyl-3-methoxy-5-hydroxybenzolsulfonamid. MS (m/e) = 369.

e) Daraus erheilt man analog Bsp. 18a) in 30% Ausbeute [3-Methoxy-5-(benzyl-phenylsulfamoyl)-phenoxy]-essigsäure-ethylester. MS (m/e) = 455.

f) Daraus erhielt man analog Bsp. 1b) quantitativ [3-Methoxy-5-(methyl-phenylsulfamoyl)-phenoxy]-essigsäure. MS (m/e) = 427.

g) Daraus erheilt man analog Bsp. 1c) in 42% Ausbeute N-(Pyridin-4-yl)-[3-methoxy-5-(methyl-phenyl-sulfamoyl)-phenoxy]-acetamid. Fp. 175 °C.

h) Daraus erhielt man analog Bsp. 1d) die Titelverbindung in 50% Ausbeute als amorphes Pulver. MS (m/e) = 489.

**Beispiel 82**

3-Methoxy-N-phenyl-5-[2-(pyridin-4-ylamino)-ethoxy]-benzolsulfonamid

60 mg (0.12 mmol) der Verbindung Bsp. 81) hydrierte man anlog Bsp.58) und erhielt 20 mg (40%) der Titelverbindung als amorphes Pulver. MS (m/e) = 399.

**Beispiel 83**

N-Methyl-N-{3-[2-(pyridin-4-ylamino)-ethoxy]-5-methoxy-phenyl}-benzolsulfonamid

a) 18.3 g (100 mmol) der Verbindung 80c) setzte man analog Bsp. 79i) mit 14.3 g (110 mmol) Benzolsulfonylchlorid um und erhielt 32.5 g (quant.) N-(3,5-Dinitrophenyl)benzolsulfonamid. Fp. 165 °C.

b) 44 g (136 mmol) dieser Verbindung methylierte man analog Bsp.12a) und erhielt 25.1 g (54%) N-Methyl-N-(3,5-dinitrophenyl)-benzolsulfonamid. Fp. 125 °C.

c) 6.8 g (20 mmol) dieser Verbindung reduzierte man analog 79a) und erhielt 6.1 g (quant.) N-Methyl-N-(3-amino-5-nitro-phenyl)-benzolsulfonamid als amorphes Pulver. MS (m/e) = 307.

d) Aus 6.1 g (20 mmol) dieser Verbindung erhielt man analog Bsp. 80g) 1.8 g (30%) N-Methyl-N-(3-hydroxy-5-nitro-phenyl)-benzolsulfonamid als amorphes Pulver. MS (m/e) = 308. (380 nach Silylierung).

e) 1.2 g (4 mmol) dieser Verbindung, 6 mL 1 N Natronlauge, 1.3 g Tetrabutylammonium-Bromid. 6 mL Dichlormethan und 0.4 mL Iodmethan rührte man 12 h bei Raumtemperatur. Man trennte die organische Phase ab, entfernte das Lösungsmittel i. Vak. und reinigte den Rückstand über Kieselgel (150 g). Man eluierte mit Isohexan / Essigester = 2 : 1 und erhielt 240 mg (18%) N-Methyl-N-(3-methoxy-5-nitro-phenyl)-benzolsulfonamid. Fp. 136 °C.

f) Diese Verbindung hydrierte man analog Bsp. 58 und erhielt quantitativ N-Methyl-N-(3-methoxy-5-amino-phenyl)-benzolsulfonamid als amorphes Pulver. MS (m/e) = 292.

g) Analog Bsp. 80g) erhielt man daraus in 74% Ausbeute N-Methyl-N-(3-methoxy-5-hydroxy-phenyl)-benzolsulfonamid als amorphes Pulver. MS (m/e) = 293.

h) Analog Bsp 18a) erhielt man daraus in 89% Ausbeute [3-Methoxy-5-(N-methyl-phenylsulfonyl-amino)-phenoxy]-essigsäure-ethylester als amorphes Pulver. MS (m/e) = 379.

i) Analog Bsp. 81f) bis 81h) erhielt man daraus [3-Methoxy-5-(N-methyl-phenylsulfonylamino)-phenoxy]-essigsäure (74%; MS = 351), N-(Pyridin-4-yl)-[3-Methoxy-5-(N-methyl-phenylsulfonyl-amino)-phenoxy]-acetamid (34%; MS = 427) und die Titelverbindung als amorphes Pulver. MS (m/e) = 413.

**Beispiel 84**

3-Chlor-N-methyl-N-phenyl-5-[2-(pyridin-4-ylamino)-ethoxy]-benzolsulfonamid

a) Die Verbindung 80e) reduzierte man analog Bsp. 79a) und erhielt in 50% Ausbeute N-Methyl-N-phenyl-3-amino-5-nitro-benzolsulfonamid. Fp. 175 °C.

b) Zu 3.5 g (10 mmol) dieser Verbindung in 40 mL 6 N Salzsäure tropfte man bei 0°C eine Lösung von 760 mg (11 mmol) Natriumnitrit in 2 mL Wasser und goß dann die erhaltene Suspension in eine Lösung, die man wie folgendermaßen bereitet hatte: 3.75 g Kupfersulfat-pentahydrat und 1.35 g Kochsalz löste man in 12 mL warmem Wasser, tropfte eine Lösung von 950 mg (7.5 mmol) Natriumsulfit in 3 mL Wasser dazu, filtrierte den Niederschlag rasch ab und löste ihn in 6 mL konzentrierter Salzsäure. Man erhitzte langsam auf 100 °C. kühlte ab, extrahierte mit Essigester, und filtrierte über eine Kieselgelsäure (100 g Kieselgel). Man eluierte mit Isohexan / Essigester und erhielt 1.45 g (43%) N-Methyl-N-phenyl-3-chlor-5-nitro-benzolsulfonamid. Fp. 143 °C.

c) Diese Verbindung reduzierte man quantitativ analog Bsp. 79a) zu N-Methyl-N-phenyl-3-chlor-5-amino-benzolsulfonamid. Amorphes Pulver. MS (m/e) = 296.

d) Analog den Beispielen 83g) bis 83i) stellte man daraus her: N-Methyl-N-phenyl-3-chlor-5-hydroxy-benzolsulfonamid (69%, amorph, MS = 297); [3-Chlor-5-(methyl-phenylsulfamoyl)-phenoxy]-essigsäure-ethylester (92%, amorph, MS = 383); [3-Chlor-5-(methyl-phenyl-sulfamoyl)-phenoxy]-essigsäure (quant., amorph, MS = 355); N-(Pyridin-4-yl)-[3-chlor-5-(methyl-phenyl-sulfamoyl)-phenoxy]-acetamid (32%, amorph, MS = 431); Titelverbindung (40%: amorph MS = 417).

**Beispiel 85**

3-Chlor-N-benzyl-N-phenyl-5-[2-(pyridin-4-ylamino)-ethoxy]-benzolsulfonamid

a) 12.3 g (46 mmol) der Verbindung 80d) setzte man anolog dem Beispiel 79i mit 9.2 g (50 mmol) Benzylamin um und erhielt 31.3 g (83%) N-Benzyl-N-phenyl-3,5-dinitrobenzolsulfonamid. Fp. 205 °C.

b) Diese Verbindung reduzierte man analog Bsp. 79a) und erhielt quantitativ N-Benzyl-N-phenyl-3-amino-5-nitro-benzolsulfonamid. Fp. 170 °C.

c) Analog Bsp.84b) erhielt man daraus in 37% Ausbeute N-Benzyl-N-phenyl-3-chlor-5-nitro-benzolsulfonamid. Fp. 160 °C.

d) Analog dem Beispiel 84c) bis 84d) stellte man die folgenden Verbindungen her: N-Benzyl-N-phenyl-3-chlor-5-amino-benzolsulfonamid (quant., amorph, MS = 372); N-Benzyl-N-phenyl-3-chlor-5-hydroxy-benzolsulfonamid (quant.. amorph, MS = 373); [3-Chlor-5-(benzyl-phenyl-sulfamoyl)-phenoxy]-essigsäure-ethylester (15%, Öl, MS = 459): [3-Chlor-5-(benzyl-phenyl-sulfamoyl)-phenoxy]-essigsäure (50%, amorph, MS = 431);N-(Pyridin-4-yl)-[3-chlor- 5-(benzyl-phenyl-sulfamoyl)-phenoxy]-acetamid (44%, amorph. MS = 507); Titelverbindung (40%, amorph, MS = 493)

**Beispiel 86**

3-Chlor-N-benzyl-N-phenyl-5-[2-(pyridin-4-ylamino)-ethylamino]-benzolsulfonamid

Aus N-Benzyl-N-phenyl-3-chlor-5-amino-benzolsulfonamid (Bsp. 85d) stellte man analog Bsp.83h) bis 83i) die folgenden Verbindungen her: [3-Chlor-5-(benzyl-phenylsulfamoyl)-phenylamino]-essigsäure-ethylester (18%, Öl, MS = 458); [3-Chlor-5-(benzyl-phenyl-sulfamoyl)-phenylamino]-essigsäure (quant., amorph, MS = 430); N-(Pyridin-4-yl)-[3-chlor-5-(benzyl-phenyl-sulfamoyl)-phenylamino]-acetamid (25%, amorph, MS = 506); Titelverbindung (50%, amorph, MS (m/e) = 492).

**Beispiel 87**

N-Methyl-N-{3-[2-(pyridin-4-ylamino)-ethoxy]-phenyl}-benzolsulfonamid

a) N-Methyl-N-(3-amino-5-nitro-phenyl)-benzolsulfonamid (Bsp. 83c) setzte man analog Bsp. 84b) zu N-Methyl-N-(3-chlor-5-nitro-phenyl)-benzolsulfonamid um (52%, amorph, MS (m/e) = 326)

b) Diese Verbindung reduzierte man analog Bsp 79a) zu N-Methyl-N-(3-chlor-5-aminophenyl)-benzolsulfonamid (42%, Öl, MS = 296); [3-Chlor-5-(N-methyl-phenylsulfonylamino)-phenoxy]-essigsäure-ethylester (89%, amorph. MS (m/e) = 383); [3-Chlor-5-(N-methyl-phenylsulfonyl-amino)-phenoxy]-essigsäure (88%; MS = 355); N-(Pyridin-4-yl)-[3-chlor-5-(N-methyl-phenylsulfonyl-amino)-phenoxy]-acetamid (28%; MS = 431) und die Titelverbindung (56%) als amorphes Pulver. MS (m/e) = 417.

**Beispiel 88**

N-Benzyl-N-{3-[2-(pyridin-4-ylamino)-ethoxy]-5-chlor-phenyl}-benzolsulfonamid

a) Analog Bsp.92c) benzylierte man die Verbindung Bsp. 83a) mit Benzylbromid und erhielt N-Benzyl-N-(3,5-dinitrophenyl)-benzolsulfonamid (quant.).Fp. 170 °C.

b) Diese Verbindung reduzierte man analog 79a) und erhielt 27% N-Benzyl-N-(3-amino-5-nitro-phenyl)-benzolsulfonamid als amorphes Pulver. MS (m/e) = 383.

c) Aus dieser Verbindung erhielt man analog Bsp. 84b) in 45% Ausbeute N-Benzyl-N-(3-chlor-5-nitro-phenyl)-benzolsulfonamid. Fp. 148 °C.

d) Diese Verbindung reduzierte man analog Bsp. 79a und erhielt 34% N-Benzyl-N-(3-chlor-5-amino-phenyl)-benzolsulfonamid. Fp. 145 °C.

e) Analog Bsp. 83g) bis 83i) erhielt man daraus die folgenden Verbindungen: N-Benzyl-N-(3-chlor-5-hydroxy-phenyl)-benzolsulfonamid (quant., amorph, MS (m/e) = 373); [3-Chlor-5-(N-benzyl-phenylsulfonyl-amino)-phenoxy]-essigsäure-ethylester (quant., amorph, MS (m/e) = 459); [3-Chlor-5-(N-benzyl-phenylsulfonyl-amino)-phenoxy]-essigsäure (82%, Fp. 180 °C (Zers.)); N-(Pyridin-4-yl)-[3-chlor-5-(N-benzylphenylsulfonyl-amino)-phenoxy]-acetamid (54%; Fp. 178 °C) und die Titelverbindung als amorphes Pulver. MS (m/e) = 493.

**Beispiel 89**

N-{3-[2-(Pyridin-4-ylamino)-ethylamino]-5-brom-phenyl}-benzolsulfonamid

a) Zu 18.3 g (100 mmol) 3,5-Dinitroanilin (Bsp. 80c) in 100 mL Eisessig und 100 mL 47%iger wäßriger Bromwasserstoffsäure tropfte man bei 0°C eine Lösung von 7.6 g (110 mmol) Natriumnitrit in 15 mL Wasser in 15 min zu und verfuhr weiter wie in Bsp.84b) beschrieben. Man erhielt 21.7 g (88%) 3,5-Dinitro-brom-benzol. Fp. 65 °C.

b ) Diese Verbindung reduzierte man analog Bsp. 79a) und erhielt 17.4 g (91%) 3-Brom-5-nitro-anilin. Fp. 105 °C.

c) Diese Verbindung alkylierte man analog Bsp. 18a) und erhielt quantitativ 3-Brom-5-nitro-phenyl-amino-essigsäure-ethylester. Amorph. MS (m/e) = 303.

d) Diese Verbindung verseifte man analog Bsp. 1b) und erhielt Brom-5-nitro-phenyl-aminoessigsäure (22%. amorph, MS (m/e) = 274).

e) Diese Verbindung setzte man analog Bsp. lc) um und erhielt in 29%b Ausbeute N-(Pyridin-4-yl)-(3-brom-5-nitro-phenyl-amino)-acetamid. Fp. 240 °C.

f) Diese Verbindung hydrierte man analog Bsp. 58) und erhielt quantitativ N-(Pyridin-4-yl)-(3-brom-5-amino-phenyl-amino)-acetamid. Amorph. MS (m/e) = 321.

g) Analog Bsp.79i) erhielt man daraus N-(Pyridin-4-yl)-[3-brom-5-benzolsulfonylaminophenyl-amino)-acetamid. Amorph. MS (m/e) = 460.

h) Daraus erhielt man analog Bsp. ld) die Titelverbindung. Amorph. MS (m/e) = 446.

**Beispiel 90**

Benzolsulfonsäure-3-ethyl-5-[2-(pyridin-4-ylamino)-ethoxy]-phenyl-ester

a) 20 g 3,5-Dimethoxybenzoesäure (110 mmol) in 80 mL Thionylchlorid erhitzte man 1 h unter Rückfluß zum Sieden. Man entfernte das Lösungsmittel i. Vak., nahm den Rückstand in 500 mL trockenem Methylenchlorid aufund leitete 90 min trockenen Ammoniak uber die eisgekühlte Lösung. Man rührte noch 2 h bei raumtemperatur, entfernte das Lösungsmittel i. Vak., rührte den Rückstand 12 h in 200 mL Wasser und 50 mL gesättigter Natriumhydrogencarbonatlösung, filtrierte, löste den Niederschlag in Essigester. filtrierte über Aktivkohle und entfernte den Essigester bis zur beginnenden Kristallisation. Man erhielt 10.8 g 3,5-Dimethoxybenzamid. Fp. 145°C.

b) Zu 7.6 g (310 mmol) Magnesium in 10 mL trockenem Ether tropfte man 19.5 mL (310 mmol) Iodmethan in 30 mL Ether, erhitzte 30 min unter Rückfluß zum Sieden und gab dann portionsweise 11.6 g (63 mmol) 3,5-Dimethoxybenzamid zu. Man erhitzte 22h unter Rückfluß zum Sieden. tropfte unter Eiskühlung 125 mL 6 N Salzsäure zu. rührte 16 h bei Raumtemperatur. wusch die organische Phase mit Wasser, entfernte das Lösungsmittel i. Vak. und erhielt 9.4 g 3,5-Dimethoxy-acetophenon als Öl. MS (m/e) = 180.

c) 9.4 g (52 mmol) dieser Verbindung in 150 mL Ethanol und 2 mL konzentrierter Salzsäure hydrierte man in Gegenwart von 1 g Palladium bei 50 °C und 5 bar Druck. Man filtrierte, entfernte das Lösungsmittel i.Vak.und erhielt 6.8 g (78 %) 3,5-Dimethoxy-ethylbenzol als Öl. MS (m/e) = 166.

d) 6.8 g (41 mmol) dieser Verbindung in 65 mL Eisessig und 25 mL konzentrierter 47%iger Bromwasserstoffsäure erhitzte man 4 h unter Rückfluß zum Sieden. Man entfernte das Lösungsmittel i.Vak, gab Wasser zum Rückstand, extrahierte mit Essigester. wusch den Essigester mit Wasser. entfernte das Lösungsmittel i. Vak. und filtrierte den Rückstand über Kieselgel (Isohexan / Essigester = 3 : 1). Man erhielt 3.9 g (69%) 5-Ethyl-resorcin als Öl. MS (m/e) = 138.

e) 3.9 g (28 mmol) dieser Verbindung und 4.3 mL (33 mmol) Benzolsulfonylchlorid in 30 mL Ether und 60 mL gesättigter Natriumhydrogencarbonatlösung rührte man 48 h bei Raumtemperatur. Man trennte die Etherphase ab, entfernte das Lösungsmittel i.Vak., filtirerte den Rückstand über Kieselgel (Isohexan : Essigester 3 : 1) und erhielt 5.4 g (69%) Benzolsulfonsäure-3-hydroxy-5-ethyl-phenyl-ester als Öl. Ms (m/e) = 278.

f) Analog dem Beispiel 81e) bis 81h) erhielt man daraus die folgenden Verbindungen: (3-Benzolsulfonyloxy-5-ethyl-phenyloxy)-essigsäure-ethylester (77%, Öl, MS (m/e) = 364); (3-Benzolsulfonyloxy-5-ethyl-phenyloxy)-essigsäure (59%, amorph, MS (m/e) = 336); N-(Pyridin-4-yl)-(3-benzolsulfonyloxy-5-ethyl-phenyloxy)-acetamid (70%, amorph, MS (m/e) = 412); Titelverbindung (10%, Fp. 136 °C).

**Beispiel 91**

N-Benzyl-N-{3-[2-(Pyridin-4-ylamino)-ethoxy]-5-methyl-phenyl}-benzolsulfonamid

a ) 107 g (1 mol) para-Toluidin setzte man analgo Bsp. 79i) mit p-Tosylchlorid um und erhielt 280 g (quant.) N-(4-Methylphenyl)-4-methyl-benzolsulfonamid als Öl, das man ohne weitere Reinigung umsetzte. Kristalle aus Ether mit Fp. 105 °C.

b) 21 g (80 mmol) dieser Verbindung trug man unter Eiskühlung in 56 mL rauchende Salpetersäure ein. tropfte dann langsam 32 mL konzentrierte Schwefelsäure zu, goß auf Eis. wusch den Niederschlag mit Wasser und erhielt 46.6 g (73%) gelben Feststoffmit Fp. 170 °C, den man in 80 mL konzentrierter Schwefelsäure 10 min auf 100 °C erhitzte, auf Eiswasser goß und mit Essigester extrahierte. Man entfernte das Lösungsmittel i. Vak. und erhielt 20 g (88%) 2,6-Dinitro-4-methyl-anilin mit dem Fp. 171 °C.

c) Zu 20.5 g (300 mmol) Natriumnitrit in 220 mL Schwefelsäure gab man 800 mL Eisessig und portionsweise 53.5 g (270 mmol) 2,6-Dinitro-4-methyl-anilin, man rührte 3 h bei 40 °C. bis sich alles gelöst hatte und tropfte diese Lösung zu einer eisgekühlten Suspension von 20 g Kupferoxid in EthanoL entfernte das Lösungsmittel i. Vak, gab Wasser zu und extrahierte mit Essigester. Diesen Ansatz führte man noch ein zweitesmal durch und erhielt zusammen 88 g (88%) 3,5-Dinitrotoluol als amorphes Pulver.

d) 88g (480 mmol) dieser Verbindung in 520 mL Methanol sättigte man mit 53 g Ammoniak und leitete dann 15 min Schwefelwasserstoff ein, wobei die Temperatur auf 52 °C anstieg. Man erhitzte 30 min unter Rückfluß zum Sieden, goß auf 1 L Wasser und erhielt 60.5 g (82%) 3-Methyl-5-nitro-anilin. Fp. 97 °C.

e) Analog Bsp. 79i) erhielt man daraus N-(3-Methyl-5-nitro-phenyl)-benzolsulfonamid (quantitativ) Fp. 165 °C.

f) Analog Bsp. 88a) bis 88b) erhielt man daraus N-Benzyl-N-(3-methyl-5-nitro-phenyl)-benzolsulfonamid (83%) Fp. 154 °C und N-Benzyl-N-(3-methyl-5-amino-phenyl)-benzolsulfonamid (34%), amorph, MS (m/e) = 352.

g) Analog dem Beispiel 81e) bis 81h) erhielt man daraus die folgenden Verbindungen: [3-(Benzolsulfonyl-benzyl-amino)-5-methyl-phenylamino]-essigsäure-ethylester (quant., Öl, MS (m/e) = 438); [3-(Benzolsulfonyl-benzyl-amino)-5-methyl-phenylamino)-essigsäure (90%. amorph, MS (m/e) = 410); N-(Pyridin-4-yl)-[3-(benzolsulfonyl-benzyl-amino)-5-methyl-phenylamino)-acetamid (10%. amorph, MS (m/e) = 486); Titelverbindung (47%, amorph, MS (m/e) = 472).

**Beispiel 92**

(Benzolsulfonyl-{3-methyl-5-[2-(pyridin-4-yl-amino)-ethoxy]-phenyl}-amino)-essigsäureethylester

a) 100 g 4-Nitro-tetrachlorpyridin und 50.6 mL Ethanolamin in 1.2 L Dioxan rührte man 90 min bei Ramtemperatur. entfernte das Lösungsmittel i.Vak., gab Wasser zum Rückstand, extrahierte mit Essigester. wusch den Essigester mit Wasser, entfernte das Lösungsmittel i. Vak. und erhielt 105 g (72%) 4-(2-Hydroxyethyl-amino)-tetrachlorpyridin. Fp. 131 - 133 °C.

b) 36.3 g (130 mmol) dieser Verbindung und 12.1 mL (170 mmol) Acetylchlorid in 450 mL Eisessig rührte man 12 h bei Raumtemperatur, goß auf Eis, neutralisierte mit konzentriertem Ammoniak, extrahierte mit Essigester, wusch den Essigester mit Wasser, entfernte das Lösungsmittel i.Vak. und erhielt 41.6 g (99%) Essigsäure-2-(tetrachlorpyridin-4-ylamino)-ethylester. Fp. 72 - 75 °C.

c) Zu 109 g (340 mmol) dieser Verbindung in 700 mL trockenem Dimethylformamid gab man eine Suspension von 10.8 g Natriumhydrid in 150 mL Dimethylformamid und tropfte anschließend bei 10 °C eine Lösung von 54 mL Benzylbromid in 350 mL Dimethylformamid zu. Man rührte 2 h bei Raumtemperatur, goß auf 7 L Eiswasser, filtrierte. wusch den Niederschlag mit Wasser, löste ihn in 800 mL Methanol und 200 mL Dichlormethan. engte bis zur beginnenden Kristallisation ein und ließ kristallisieren. Man erhielt 120 g Essigsäure-2-(benzyl-tetrachlorpyridin-4-yl-amino)-ethylester. Fp. 97 - 100 °C.

d) Zu 108 g (260 mmol) dieser Verbindung in 1.2 L Ethanol und 390 mL 2 N Natronlauge gab man soviel Dimethylformamid, bis sich alles gelöst hatte (ca. 0.5 L). Man rührte 12 h bei Raumtemperatur, entfernte das Lösungsmittel i. Vak (zuletzt bei $10^{-2}$ Torr), gab zum Rückstand 3 L Wasser und extrahierte mit 1 L Essigester, wusch den Essigester mit 3 L Wasser, entfernte das Lösungsmittel i. Vak. und erhielt 99g (quant.) N-Benzyl-N-(2-hydroxyethyl)-N-(tetrachlorpyridin-4-yl)-amin als Öl. Ms (m/e) = 366.

e) Zu 107 g (290 mmol) dieser Verbindung in 800 mL Dichlormethan gab man 73 mL (520 mmol) Triethylamin. kühlte auf 0 °C ab und tropfte eine Lösung von 67.1 g (350 mmol) 4-Toluolsulfonylchlorid in 500 mL Dichlormethan zu. Man bewahrte die Lösung 16 h bei 4 °C auf, gab Wasser zu, trennte die organische Phase ab und entfernte das Lösungsmittel i. Vak. Man erhielt 90.6 g (64%) 4-Toluolsulfonsäure-2-(benzyltetrachlorpyridin-4-yl-amino)-ethylester. Fp. 114 - 116 °C.

f) 57.2 g (240 mmol) dieser Verbindung, 64.8 g (260 mmol) 3-Phthalimido-5-methylphenol (Bsp. 76a) und 66.2 g (480 mmol) Kaliumcarbonat in 1.15 L Dimethylsulfoxid rührte man 72 h bei Raumtemperatur, goß auf3 L Wasser, filtrierte, digerierte den Rückstand mit Diisopropylether und erhielt 63 g (38%) N-Benzyl-N-(tetrachlorpyridin-4-yl)-N-[2-(3-phthalimido-5-methyl-phenoxy)-ethyl]-amin. Fp. 142 - 144 °C.

g) 19.1 g (31.8 mmol) dieser Verbindung, 2.3 mL (47.6 mmol) Hydrazin-Hydrat und 50 mL Ethanol in 100 mL Dichlormethan rührte man 12 h bei Raumtemperatur, entfernte das Lösungsmittel i. Vak.. suspendierte den Rückstand in 150 mL 2 N Natronalauge, extrahierte mit Dichlormethan, wusch die organische Phase mit Wasser, entfernte das Lösungsmittel i.Vak., digerierte den Rückstand mit Methanol und erhielt 11.5 g (77%) N-Benzyl-N-(tetrachlorpyridin-4-yl)-N-[2-(3-amino-5-methyl-phenoxy)-ethyl]-amin. Fp. 91 - 93 °C.

h) 11.5 g (25.5 mmol) dieser Verbindung setzte man analog Bsp.79i) mit 3.51 mL (27.0 mmol) Benzolsulfonylchlorid um und erhielt 13.7 g (91%) N-{3-[2-(Benzyltetrachlorpyridin-4-yl-amino)-ethoxy]-5-methyl-phenyl}-benzolsulfonamid. Fp. 147 - 149 °C.

i) 3.0 g (4.00 mmol) dieser Verbindung in 15 mL trockenem Dimethylformamid gab man zu 147 mg (6.38 mmol) Natriumhydrid in 2 mL Dimethylformamid und tropfte dann 0.6 mL (5.4 mmol) Bromessigsäure-ethylester zu. Man goß auf 300 mL Wasser, extrahierte mit Essigester, wusch die organische Phase mit Wasser, entfernte das Lösungsmittel i. Vak. und erhielt 3.15 g (91%) (Benzolsulfonyl-{3-methyl-5-[2-(benzyl-tetrachlorpyridin-4-yl-amino)-ethoxy]-phenyl}-amino)-essigsäure-ethylester. Fp. 141 - 142 °C.

j) 3.15 g (4.5 mmol) dieser Verbindung, 50 mL Trifluoressigsäure und 6.8 mL 1,2,3-Trimethylbenzol rührte man 12 h bei Raumtemperatur, goß aufEiswasser, neutralisierte mit konzentriertem Ammoniak extrahierte mit Ether, wusch die Etherphase mit Wasser, entfernte das Lösungsmittel i. Vak., filtrierte den Rückstand über Kieselgel (Essigester / Isohexan 1 : 2.5) und erhielt2.50 g (91%) (Benzolsulfonyl-{3-methyl-5-[2-(tetrachlorpyridin-4-ylamino)-ethoxy]-phenyl}-amino)-essigsäure-ethylester als Öl. MS (m/e) = 605.

k) 3.5 g (5.76 mmol) dieser Verbindung und 4.0 g Kaliumcarbonat in 40 mL Tetrahydrofuran und 40 mL Methanol hydrierte man in Gegenwart von 1.0 g 10% Palladium auf Kohle bei 4 bar Druck. Nach 48 h filtrierte man, entfernte das Lösungsmittel i. Vak., , filtrierte über Kieselgel (Methylenchlorid / Methanol = 4 : 1) und erhielt 2.1 g (78%) der Titelverbindung. Amorph. MS (m/e) = 469.

**Beispiel 93**

(Benzolsulfonyl-{3-methyl-5-[2-(pyridin-4-yl-amino)-ethoxy]-phenyl}-amino)-essigsäure

1.20 g (2.55 mmol) der Verbindung Bsp. 92) verseifte man in 20 mL Ethanol mit 5.1 mL 1 N Natronlauge 2 h bei 45 °C. Man neutralisierte mit 1 N Salzsäure, entfernte das Lösungsmittel i. Vak., nahm den Rückstand in 20 mL Wasser aufund ließ kristallisieren. Man erhielt 0.97 g (86%) der Titelverbindung. Fp. 100 - 102 °C (Zers.).

**Beispiel 94**

(Benzolsulfonyl-{3-methyl-5-[2-(pyridin-4-yl-amino)-ethoxy]-phenyl}-amino)-acetamid

1.5 g (3.2 mmol) der Verbindung Bsp.92) löste man in 12 mL konzentriertem Ammoniak und 30 mL Methanol. Nach 12 h bei Raumtemperatur filtrierte man und erhielt 0.99 g (70%) der Titelverbindung. Fp. 163 - 165 °C.

**Beispiel 95**

N-(2-Hydroxyethyl)-(benzolsulfonyl-{3-methyl-5-[2-(pyridin-4-yl-amino)-ethoxy]-phenyl}-amino)-acetamid

erhielt man in 60% Ausbeute analog Bsp. 94 mit Ethanolamin an Stelle von Ammoniak. Fp. 169 - 170 °C.

**Beispiel 96**

N-(3-Hydroxyethyl)-(benzolsulfonyl-{3-methyl-5-[2-(pyridin-4-yl-amino)-ethoxy]-phenyl}-amino)-acetamid

erhielt man in 70% Ausbeute analog Bsp. 94 mit 3-Propanolamin an Stelle von Ammoniak. Fp. 148 - 152 °C.

**Beispiel 97**

N-Methyl-(benzolsulfonyl-{3-methyl-5-[2-(pyridin-4-yl-amino)ethoxy]-phenyl}-amino)-acetamid

erhielt man in 58% Ausbeute analog Bsp. 94 mit 25%iger ethanolischer Methylamin-Lösung an Stelle von Ammoniak. Fp. 136 - 140 °C.

**Beispiel 98**

N,N-Dimethyl-(benzolsulfonyl-{3-methyl-5-[2-(pyridin-4-yl-amino)-ethoxy]-phenyl}-aminoacetamid

erhielt man in 38% Ausbeute analog Bsp. 94 mit 41%iger ethanolischer Dimethylamin-Lösung an Stelle von Ammoniak. Amorph. MS (m/e) = 468.

**Beispiel 99**

N-(2-Aminoethyl)-(benzolsulfonyl-{3-methyl-5-[2-(pyridin-4-yl-amino)-ethoxy]-phenyl}-amino)-acetamid

erhielt man in 45% Ausbeute analog Bsp. 94 mit Ethylendiamin an Stelle von Ammoniak. Amorph. MS (m/e) = 483.

**Beispiel 100**

N-(2-Aminoethyl)-N-{3-[2-(pyridin-4-ylamino)-ethoxy]-5-methyl-phenyl}-benzolsulfonamid

Die Verbindung aus Bsp.94) reduzierte man analog Bsp. 18c) und erhielt die Titelverbindung in 34% Ausbeute. Amorph. MS (m/e) = 426.

**Beispiel 101**

N-(2,3-Dihydroxypropyl)-(benzolsulfonyl-{3-methyl-5-[2-(pyridin-4-yl-amino)-ethoxy]-phenyl}-amino)-acetamid

erhielt man in 40% Ausbeute analog Bsp. 94 mit 2,3-Dihydroxypropylamin an Stelle von Ammoniak. Fp. 148 - 151 °C.

**Beispiel 102**

N-(2,3-Dihydroxypropyl)-N-{3-[2-(pyridin-4-ylamino)-ethoxy]-5-methyl-phenyl}-benzolsulfonamid

a) 10.0 g (75.7 mmol) 2,2-Dimethyl-4-hydroxymethyl-1,3-dioxolan und 14.3 g (75 mmol) 4-Toluolsulfonsäurechlorid in 7 mL Pyridin rührte man 16 h bei Raumtemperatur, goß auf 200 mL Wasser, extrahierte mit Essigester und entfernte das Lösungsmittel i. Vak. Man kristallisierte aus Isohexan um und erhielt 10.3 g (48%) 4-Methylbenzolsulfonsäure-(2,2-dimethyl-1,3-dioxolan-4-yl-methyl)-ester. Fp. 45 - 47 °C.

b) 0.24 g (0.83 mmol) dieser Verbindung gab man zu einer Lösung von 23 mg Natriumhydrid und 0.46 g (0.75 mmol) der Verbindung Bsp.92h) in 3 mL Dimethylformamid, rührte 8 h bei 110 °C, goß auf Wasser, extrahierte mit Essigester, entfernte das Lösungsmittel i. Vak. und erhielt 300 mg (55%) N-(2,2-Dimethyl-1,3-dioxolan-4-yl-methyl-N-{3-[2-(benzyl-tetrachlorpyridin-4-yl-amino)-ethoxy]-5-methyl-phenyl}-benzolsulfonamid als Öl. MS (m/e) = 725.

c) Aus dieser Verbindung erhielt man analog Bsp. 92j) N-(2,3-Dihydroxypropyl)-N-{3-[2-(benzyl-tetrachlorpyridin-4-yl-amino)-ethoxy]-5-methyl-phenyl}-benzolsulfonamid (38%, Fp. 133 - 136 °C).

d) Aus dieser Verbindung erhielt man analog Bsp. 96k) die Titelverbindung (66%, amorph. MS (m/e) = 457).

**Beispiel 103**

4-(Benzolsulfonyl-{3-methyl-5-[2-(pyridin-4-yl-amino)-ethoxy]-phenyl}-amino)-buttersäure-ethylester

Analog Bsp. 92i) bis 92k) erhielt man die folgenden Verbindungen, indem man in Bsp. 92i) an Stelle des Bromessigsäure-ethylesters 4-Brombuttersäure-ethylester verwendete: 4-(Benzolsulfonyl-3-methyl-5-[2-(benzyl-tetrachlorpyridin-4-yl-amino)-ethoxy]-phenyl}-amino)-buttersäure-ethylester (78%, Fp. 106 - 108 °C); 4-(Benzolsulfonyl-{3-methyl-5-[2-(tetrachlorpyridin-4-ylamino)-ethoxy]-phenyl}-amino)-buttersäure-ethylester (Öl, MS (m/e) = 633); Titelverbindung (75%, amorph, MS (m/e) = 497).

**Beispiel 104**

5-(Benzolsulfonyl-{3-methyl-5-[2-pyridin-4-yl-amino)-ethoxy]-phenyl}-amino)-pentansäure-ethylester

Analog Bsp. 92i) bis 92k) erhielt man die folgenden Verbindungen, indem man in Bsp. 92i) an Stelle des Bromessigsäure-ethylesters 5-Brompentansäure-ethylester verwendete: 5-(Benzolsulfonyl-{3-methyl-5-[2-(benzyl-tetrachlorpyridin-4-yl-amino)-ethoxy]-phenyl}-amino)-pentansäure-ethylester (72%, Fp. 90-91 °C); 5-(Benzolsulfonyl-{3-methyl-5-[2-(tetrachlorpyridin-4-ylamino)-ethoxy]-phenyl}-amino)-pentansäure-ethylester (86%, Öl, MS (m/e) = 647); Titelverbindung (78%, amorph, MS (m/e) = 511).

**Beispiel 105**

6-(Benzolsulfonyl-{3-methyl-5-[2-(pyridin-4-yl-amino)-ethoxy]-phenyl}-amino)-hexansäure-ethylester

Analog Bsp. 92i) bis 92k) erhielt man die folgenden Verbindungen, indem man in Bsp. 92i) an Stelle des Bromessigsäure-ethylesters 6-Bromhexansäure-ethylester verwendete: 6-(Benzolsulfonyl-{3-methyl-5-[2-(benzyl-tetrachlorpyridin-4-yl-amino)-ethoxy]-phenyl}-amino)-hexansäure-ethylester (75%. Öl, MS (m/e) = 751); 6-(Benzolsulfonyl-{3-methyl-5-[2-(tetrachlorpyridin-4-ylamino)-ethoxy]-phenyl}-amino)-hexansäure-ethylester (49%. ÖL MS (m/e) = 661); Titelverbindung (56%. amorph. MS (m/e) = 525).

**Beispiel 106**

4-(Benzolsulfonyl-{3-methyl-5-[2-(pyridin-4-yl-amino)-ethoxy]-phenyl}-amino)-buttersäure

erhielt man analog Bsp. 93) aus der Verbindung Bsp. 103). 65% Ausbeute, amorph, MS (m/e) = 469.

**Beispiel 107**

5-(Benzolsulfonyl-{3-methyl-5-[2-(pyridin-4-yl-amino)-ethoxy]-phenyl}-amino)-pentansäure

erhielt man analog Bsp. 93) aus der Verbindung Bsp. 104). 53% Ausbeute, Fp. 117 - 120 °C.

**Beispiel 108**

6-(Benzolsulfonyl-{3-methyl-5-[2-(pyridin-4-yl-amino)-ethoxy]-phenyl}-amino)-hexansäure

erhielt man analog Bsp. 93) aus der Verbindung Bsp. 105). 53% Ausbeute, amorph. MS (m/e) =498.

**Beispiel 109**

(2-Methoxy-benzolsulfonyl-{3-methyl-5-[2-(pyridin-4-yl-amino)-ethoxy]-phenyl}-amino)-essigsäure-ethylester

Analog Bsp. 92h) bis 92 k) erhielt man die folgenden Verbindungen, indem man in Stufe 92h) an Stelle von Benzolsulfonylchlorid 2-Methoxybenzolsulfonylchlorid verwendete: 2-Methoxy-N-{3-[2-(benzyl-tetrachlorpyridin-4-yl-amino)-ethoxy]-5-methyl-phenyl}-benzolsulfonamid (65%, Fp. 175 °C); (2-Methoxy-benzolsulfonyl-{3-methyl-5-[2-(benzyl-tetrachlorpyridin-4-yl-amino)-ethoxy]-phenyl}-amino)-essigsäure-ethylester (91%, Fp. 128 - 130 °C); (2-Methoxybenzolsulfonyl-{3-methyl-5-[2-(tetrachlorpyridin-4-ylamino)-ethoxy]-phenyl}-amino)-essigsäure-ethylester (89%, Fp. 126 °C); Titelverbindung (81%, Fp. 58 - 63 °C).

**Beispiel 110**

(2-Methoxy-benzolsulfonyl-{3-methyl-5-[2-(pyridin-4-yl-amino)-ethoxy]-phenyl}-amino)-essigsäure

erhielt man aus der Verbindung Bsp. 109) analog dem Bsp.93). 82%, Fp. 218 - 222 °C.

**Beispiel 111**

(2-Methoxy-benzolsulfonyl-{3-methyl-5-[2-pyridin-4-yl-amino)-ethoxy]-phenyl}-amino-acetamid

erhielt man analog dem Bsp 94) aus der Verbindung Bsp.109. (63%. Fp. 205 °C.)

**Beispiel 112**

N-(2-Hydroxyethyl)-(2-methoxy-benzolsulfonyl-{3-methyl-5-[2-(pyridin-4-yl-amino)-ethoxy]-phenyl}-amino)-acetamid

erhielt man in 93% Ausbeute analog Bsp. 95. aus der Verbindung Bsp.109. Fp. 175 °C.

**Beispiel 113**

N-(3-Hydroxypropyl)-(2-methoxy-benzolsulfonyl-{3-methyl-5-[2-(pyridin-4-yl-amino)-ethoxy]-phenyl}-amino)-acetamid

erhielt man in 95% Ausbeute analog Bsp. 96 aus der Verbindung Bsp. 109. Fp. 165 - 167 °C.

**Beispiel 114**

N-Methyl-(2-methoxy-benzolsulfonyl-{3-methyl-5-[2-(pyridin-4-yl-amino)-ethoxy]-phenyl}-amino)-acetamid

erhielt man in 96% Ausbeute analog Bsp. 97 aus der Verbindung Bsp.109. Amorph. Ms (m/e) = 484.

**Beispiel 115**

N,N-Dimethyl-(2-methoxy-benzolsulfonyl-{3-methyl-5-[2-(pyridin-4-yl-amino)-ethoxy]-phenyl}-amino)-acetamid

erhielt man in 73% Ausbeute analog Bsp. 98 aus der Verbindung Bsp.109. Amorph. MS (m/e) = 498.

**Beispiel 116**

N-(2-Aminoethyl)-(2-methoxy-benzolsulfonyl-{3-methyl-5-[2-(pyridin-4-yl-amino)-ethoxy]-phenyl}-amino)-acetamid

erhielt man in 93% Ausbeute analog Bsp. 99 aus der Verbindung Bsp. 109. Amorph. MS (m/e) = 513.

**Beispiel 117**

N-(2,3-Dihydroxypropyl)-(2-methoxy-benzolsulfonyl-{3-methyl-5-[2-(pyridin-4-yl-amino)-ethoxy]-phenyl}-amino)-acetamid

erhielt man in 75% Ausbeute analog Bsp. 101 aus der Verbindung Bsp. 109. Amorph. MS (m/e) = 544.

**Beispiel 118**

(2-Methoxy-benzolsulfonyl-{3-methyl-5-[2-(pyridin-4-yl-amino)-ethoxy]-phenyl}-amino)-acetonitril

Zu 94 mg (0.2 mmol) der Verbindung aus Bsp. 111) in 0.5 mL Dichlormethan und 60 µL Triethylamin gab man bei 0 °C 250 µL Trichloracetylchlorid in 0.5 mL Dichlormethan. Nach 5 min neutralisierte man mit Triethylamin. entfernte das Lösungsmittel i. Vak., und filtrierte den Rückstand über Kieselgel (Essigester / methanol. Ammoniak = 4 : 1). man erhielt 60 mg der Titelverbindung als amorphe Masse. MS (m/e) = 452.

**Beispiel 119**

N-(2-Aminoethyl)-N-{3-[2-(pyridin-4-ylamino)-ethoxy]- 5-methyl-phenyl}-2-methoxy-benzolsulfonamid

Durch Umsetzung von 2-Methoxy-N-{3-[2-(benzyl-tetrachlorpyridin-4-yl-amino)-ethoxy]-5-methyl-phenyl}-benzolsulfonamid (Bsp. 109) mit Chloracetonitril analog Bsp. 92i) erhielt man (2-Methoxy-benzolsulfonyl-{3-methyl-5-[2-(benzyl-tetrachlorpyridin-4-yl-amino)-ethoxy]-phenyl}-amino)-acetonitril (92%. Fp. 154 °C), aus dem man analog Bsp. 92j) . (2-Methoxybenzolsulfonyl-{3-methyl-5-[2-(tetrachlorpyridin-4-yl-amino)-ethoxy]-phenyl}-amino)-acetonitril gewann (85%. amorph, MS (m/e) = 588), das man analog dem Bsp. 92k) zur Titelverbindung umsetzte (10%, Amorph, MS (m/e) = 456).

**Beispiel 120**

2-{3-[2-(Benzyl-pyridin-4-yl-amino)-ethoxy]-5-methyl-phenyl-sulfamoyl}-benzoesäuremethylester

a ) 4-Toluolsulfonsäure-2-(benzyl-tetrachlorpyridin-4-yl-amino)-ethylester (Bsp. 92e) sezte man mit 3-Nitrophenol um analog Bsp 92f) und erhielt N-Benzyl-N-(tetrachlorpyridin-4-yl)-N-[2-(3-nitro-phenoxy)-ethyl]-amin.(61%, Fp. 120 - 122 °C.

b) Diese Verbindung reduzierte man analog Bsp. 79a) und erhielt N-Benzyl-N-(tetrachlorpyridin-4-yl)-N-[2-(3-amino-phenoxy)-ethyl]-amin.(41 %, Fp. 105 - 107 °C).

c) Diese Verbindung setzte man analog Bsp 92h) um zu N-(3-[2-(Benzyl-tetrachlorpyridin-4-yl-amino)-ethoxy]-phenyl}-benzolsulfonamid. (78%, Fp. 120 - 122 °C).

d) Diese Verbindung hydrierte man analog Bsp. 92k) und erhielt die Titelverbindung (63%, amorph, MS (m/e) = 517).

**Beispiel 121**

2-(Methyl-{3-methyl-5-[2-(pyridin-4-ylamino)-ethoxy-phenyl}-sulfamoyl)-phenoxy]-essigsäure-ethylester

a) N-Benzyl-N-(tetrachlorpyridin-4-yl)-N-[2-(3-amino-5-methyl-phenoxy)-ethyl]-amin (Bsp.92g) setzte man analog Bsp 92h) mit 2-Benzyloxy-benzolsulfonylchlorid um und erhielt N- {3-[2-(Benzyl-tetrachlorpyridin-4-yl-amino)-ethoxy]-5-methyl-phenyl}-2-benzyloxy-benzolsulfonamid. (55%. Fp. 176 °C).

b) Diese Verbindung methylierte man analog Bsp. 12) und erhielt N-Methyl-N-{3-[2-(benzyl-tetrachlorpyridin-4-yl-amino)-ethoxy]-5-methyl-phenyl}-2-benzyloxybenzolsulfonamid (78%, amorph, MS (m/e) = 729).

c) Aus dieser Verbindung erhielt man anlog Bsp. 92j) N-Methyl-N-{3-[2-(tetrachlorpyridin-4-ylamino)-ethoxy]-5-methyl-phenyl}-2-hydroxy-benzolsulfonamid (87%. amorph. MS (m/e)= 549).

d) Diese Verbindung setzte man analog Bsp. 18) um zu [2-(Methyl-(3-methyl-5-[2-(tetrachlorpyridin-4-ylamino)-ethoxy-phenyl}-sulfamoyl)-phenoxy]-essigsäure-ethylester (quant.. Öl, MS (m/e) = 635).

e) Diese Verbindung hydrierte man analog Bsp. 92k) und erhielt die Titelverbindung (50%, amorph, MS (m/e) = 499)

**Beispiel 122**

N-{3-Methyl-5-[2-(pyridin-4-ylamino)-ethoxy]-phenyl}-2-hydroxy-benzolsulfonamid

a ) N-{3-[2-(Benzyl-tetrachlorpyridin-4-yl-amino)-ethoxy]-5-methyl-phenyl}-2-benzyloxybenzolsulfonamid (Bsp. 121a) setzte man analog Bsp. 92j) um zu N-{3-[2-(Tetrachlorpyridin-4-yl-amino)-ethoxy]-5-methyl-phenyl}-2-hydroxy-benzolsulfonamid (70%. amorph, MS (m/e) = 535).

b) Daraus erhielt man analog Bsp. 92k) die Titelverbindung. (81%, amorph, MS (m/e) = 399)

**Beispiel 123**

N-Methyl-N-{3-methyl-5-[2-(pyridin-4-ylamino)-ethoxy]-phenyl}-2-hydroxybenzolsulfonamid

N-Methyl-N-{3-[2-(benzyl-tetrachlorpyridin-4-yl-amino)-ethoxy]-5-methyl-phenyl}-2-benzyloxy-benzolsulfonamid (Bsp 121b) setzte man anlog Bsp. 122) um und erhielt N-Methyl-N-{3-[2-(tetrachlorpyridin-4-yl-amino)-ethoxy]-5-methyl-phenyl}-2-hydroxy-benzolsulfonamid (65%. amorph, MS (m/e) = 549) und die Titelverbindung (27%, amorph, MS (m/e) = 413).

**Beispiel 124**

[2-(Methyl-{3-methyl-5-[2-(pyridin-4-ylamino)-ethoxy-phenyl}-sulfamoyl)-phenoxy]-essigsäure

a ) [2-(Methyl-{3-methyl-5-[2-(tetrachlorpyridin-4-ylamino)-ethoxy-phenyl}-sulfamoyl)-phenoxy]-essigsäure-ethylester (Bsp.121d) verseifte man analog Bsp. 93) und erhielt [2-(Methyl-{3-methyl-5-[2-(tetrachlorpyridin-4-ylamino)-ethoxy-phenyl)-sulfamoyl)phenoxy]-essigsäure (94%, amorph, MS (m/e) = 607).

b) Daraus erhielt man analog Bsp. 92k) die Titelverbindung (75%, amorph, MS (m/e) = 471).

**Beispiel 125**

N-Ethoxycarbonyl-N-{3-methyl-5-[2-(pyridin-4-ylamino)-ethoxy]-phenyl}-2-methoxybenzolsulfonamid

a ) 2-Methoxy-N-{3-[2-(benzyl-tetrachlorpyridin-4-yl-amino)-ethoxy]-5-methyl-phenyl}-benzolsulfonamid (Bsp. 109) setzte man analog Bsp. 92i) mit Chlorameisensäureethylester um und erhielt N-Ethoxycarbonyl-N-{3-[2-(benzyl-tetrachlorpyridin-4-yl-amino)-ethoxy]-5-methyl-phenyl}-2-methoxy-benzolsulfonamid (quant., amorph, MS (m/e) = 711).

b) Analog Bsp. 92j) erhielt man daraus N-Ethoxycarbonyl-N-{3-[2-(tetrachlorpyridin-4-yl-amino)-ethoxy]-5-methyl-phenyl}-2-methoxy-benzolsulfonamid (80%, Fp. 156 °C).

c) Analog Bsp. 92k) erhielt man daraus die Titelverbindung (66%, amorph, MS (m/e) = 485)

**Beispiel 126**

N-(2-Hydroxyethyl)-N-{3-methyl-5-[2-(pyridin-4-ylamino)-ethoxy]-phenyl)-2-methoxy-benzolsulfonamid

Zu 150 mg (0.3 mmol) (2-Methoxy-benzolsulfonyl-(3-methyl-5-[2-(pyridin-4-yl-amino)-ethoxy]-phenyl}-amino)-essigsäure-ethylester (Bsp. 109) in 5 mL trockenem Tetrahydrofuran gab man 24 mg (0.6 mmol) Lithium-aluminiumhydrid und erhitzte 2 h unter Rückfluß zum Sieden. Man zersetzte mit 1 Tropfen Wasser und 3 Tropfen 2 N Salzsäure, filtrierte und entfernte das Lösungsmittel i. Vak. Den Rückstand filtrierte man über 50 g Kieselgel (Methylenchlorid / Methanol = 4 : 1) und erhielt 50 mg (36%) der Titelverbindung als amorphen Feststoff, MS (m/e) = 457.

**Beispiel 127**

N-{3-Methyl-5-[2-(pyridin-4-ylamino)-ethoxy]-phenyl}-pyridin-3-sulfonamid

erhielt man analog Bsp. 57), indem man in der Stufe 57d) 3-Pyridinsulfonylchlorid einsetzte. Amorph. MS (m/e) = 385.

**Beispiel 128**

**Pharmakologische Versuchsbeschreibung**

Thrombinzeit

Ein in der klinischen Gerinnungsdiagnostik gebräuchlicher Test ist die Thrombinzeit. Dieser Parameter erfaßt die Thrombinwirkung auf Fibrinogen und die Gerinnselbildung. Inhibitoren von Thrombin bewirken eine Verlängerung der

Thrombinzeit.

Zur Plasmagewinnung wurden 9 Teile frisches Blut gesunder Spender mit einem Teil Natriumcitratlösung (0.11 Mol/l) gemischt und bei ca. 3000 U/min 10 Min. bei Raumtemperatur zentrifugiert. Das Plasma wurde abpipettiert und kann bei Raumtemperatur ca. 8 h aufbewahrt werden.

200 µl Citratplasma wurden in einem Kugelkoagulometer (KC 10 der Firma Amelung) 2 Min. bei 37 °C inkubiert, Zu 190 µl vortemperiertem Thrombin-Reagenz (Boehringer Mannheim GmbH; enthält ca. 3 U/ml Pferdethrombin und 0.0125 M $Ca^{++}$) gab man 10 µl Dimethylsulfoxid (DMSO) oder eine Lösung der Wirksubstanz in DMSO. Mit Zugabe dieser 200 µl Lösung zum Plasma wurde eine Stoppuhr gestartet und der Zeitpunkt bis zum Eintritt der Gerinnung bestimmt. Die Thrombinzeit betrug bei den Kontrollmessungen ca. 24 Sek. und wurde durch die Wirksubstanzen deutlich verlängert. War die Thrombinzeit in Gegenwart einer Beispielverbindung länger als 5 Min.. wurde der Versuch abgebrochen.

In der folgenden Tabelle sind die gemessenen Thrombinzeiten in Sekunden als Differenz zur Kontrolle angegeben. Die Konzentrationen der Wirksubstanzen betrugen im Endvolumen 250 µM (TT250), 25 µM (TT25) und 2.5 µM (TT2.5).

Thrombin-Inhibierung

Die kinetischen Messungen wurden in 0,1 M Phosphatpuffer, der 0.2 M Kochsalz und 0.5 % Polyethylenglycol 6000 enthielt. bei einem pH = 7.5 und 25 °C mit dem Substrat H-(D)-Phe-Pro-Arg-pNA (S-2238 Kabi) und humanem α-Thrombin (Sigma, spezifische Aktivität = 2150 NIH-units/mg) in Polystyrol-Halbmikroküvetten in einem Gesamtvolumen von 1 ml durchgeführt.

In einem Vorversuch wurde mit jeder Wirksubstanz bestimmt, ob sie Thrombin schnell oder langsam inhibiert. Dazu wurde die Reaktion einmal durch Zugabe von 0.03 NIH-units Thrombin zu einer 100 µM-Lösung des Substrats und des Wirkstoffs gestartet. In einem zweiten Versuch wurde Substrat zu einer 5 Min. inkubierten Lösung des Thrombins und des Wirkstoffs gegeben. Die Zunahme der Konzentration von p-Nitroanilin mit der Zeit wurde spektroskopisch (UV-VIS-Spektrophotometer Lambda-2 der Firma Perkin-Elmer) bei 405 nm 12 Min. verfolgt. Da die bei beiden Versuchen erhaltenen Messkurven linear und parallel waren, handelt es sich bei den Wirkstoffen der folgenden Tabelle um schnelle Thrombin-Inhibitoren. Die Inhibitionskonstanten $K_i$ wurden dann wie folgt bestimmt. Das Substrat wurde in den Konzentrationen 100 µM, 50 µM. 30 µM, 20 µM eingesetzt und bei jeder Substratkonzentration eine Messung ohne Inhibitor und drei Messungen in Gegenwart unterschiedlicher Konzentrationen der in der folgenden Tabelle aufgeführten Inhibitoren durchgeführt. Die Reaktionen wurden durch Zugabe von Thrombin gestartet. Die Zunahme der Extinktion bei 405 nm durch das entstehende p-Nitroanilin über einen Zeitraum von 12 Min. verfolgt. Im Abstand von 20 Sek. wurden Meßpunkte (Zeit vs. Extinktion) auf einen PC übertragen. Aus den Daten wurden die Geschwindigkeiten $V_o$ (Extinktionsänderung pro Sek.: Messungen ohne Inhibitor) und $V_i$ (Messungen mit Inhibitor) durch lineare Regression bestimmt. Benutzt wurde nur der Teil jeder Messung. bei dem sich die Substratkonzentration um weniger als 15 % vermindert hatte. Aus einer Meßreihe (konstante Inhibitorkonzentration, variable Substratkonzentrationen) bestimmte man $K_m'$ und $V_{max}$ durch nichtlinearen Fit auf die Gleichung

$$V = \frac{V_{max} * [S]}{[S] + K_m'}$$

Aus den gesamten Meßreihen berechnete man schließlich $K_i$ durch nichtlinearen Fit auf die Gleichung

$$V = \frac{V_{max} * [S]}{K_m * (1 + [S]/K_i) + [S]}$$

Die Michaeliskonstante $K_m$ betrug in allen Messungen 3.8 ± 2 µM.
Die Inhibitionskonstanten $K_i$ der Wirksubstanzen sind in der folgenden Tabelle in der Einheit µM angegeben.

Inhibierung von Trypsin und Plasmin

10 mg Bovines pancreatisches Trypsin (Sigma) wurden in 100 ml 1 mM Salzsäure gelöst und im Kühlschrank aufbewahrt. 20 µl davon wurden mit 980 µl 1 mM Salzsäure versetzt. 25 µl davon wurde für jede Messung verwendet. Die Messung wurde wie für Thrombin beschrieben durchgeführt. $K_m$ = 45 µM. Die in der folgenden Tabelle aufgeführten Substanzen hemmen Trypsin nicht ($K_i$ > 400 µM).

Die Messungen mit humanem Plasmin (Sigma, 10 Units) wurden mit dem Substrat S-2251 (H-(D)-Val-Leu-Lys-pNA. Kabi) wie für Thrombin beschrieben durchgeführt, Pro Messung wurden 0.01 Units Plasmin verwendet. $K_m$ = 250

µM. Die in der folgenden Tabelle aufgeführten Substanzen hemmen Plasmin nicht ($K_i$ > 400 µM).

| Bsp. Nr. | Ki Thrombin | TT250 | TT25 | TT2.5 |
|---|---|---|---|---|
| 2 | 0.300 | 150 | 40 | 6 |
| 8 | 1.000 | 288 | 58 | 13 |
| 10 | 3.000 | 157 | 43 | 7 |
| 15 | 0.130 | 270 | 144 | 15 |
| 48 | 0.600 | 300 | 238 | 46 |
| 59 | 6.000 | 53 | 8 | 0 |
| 60 | 0.600 | 137 | 62 | 13 |
| 72 | 0.024 | 300 | 300 | 159 |
| 79 | 5.000 | 30 | 8 | 0 |
| 80 | 0.150 | 300 | 213 | 44 |
| 83 | 0.100 | 300 | 192 | 48 |
| 86 | 0.040 | 300 | 167 | 11 |
| 89 | 1.000 | 132 | 38 | 6 |
| 90 | 0.024 | 300 | 300 | 125 |
| 100 | 0.083 | 300 | 300 | 186 |
| 108 | 0.055 | 300 | 300 | 62 |
| 124 | 0.250 | 300 | 300 | 104 |
| 125 | 0.150 | 300 | 273 | 73 |

**Patentansprüche**

**1.** 4-Aminopyridine der allgemeinen Formel I

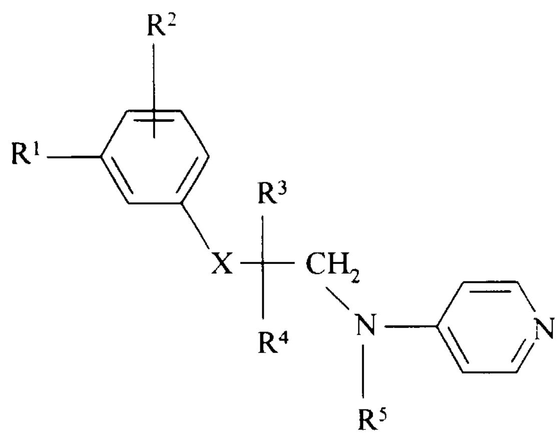

in der

$R^1$ die Gruppe $R^6$-SO-$NR^7$-, $R^6$-$SO_2$-$NR^7$-, $R^6$-$NR^7$-SO-, $R^6$-$NR^7$-$SO_2$-, $R^6$-SO-O-, $R^6$-$SO_2$-O-, $R^6$-O-SO- oder $R^6$-O-$SO_2$- bedeutet,

$R^2$ ein Wasserstoff- oder Halogenatom, eine Cyano-, $C_1$-$C_6$-Alkyl-, $C_1$-$C_6$-Alkoxy- oder Halogen-$C_1$-$C_6$-alkylgruppe bedeutet.

X ein Sauerstoffatom, ein Schwefelatom oder die NH-Gruppe bedeutet,

$R^3$ und $R^4$ gleich oder verschieden sind und Wasserstoffatome oder $C_1$-$C_6$-Alkylgruppen bedeuten,

| | |
|---|---|
| $R^5$ | ein Wasserstoffatom, eine $C_1$-$C_6$-Alkylgruppe oder die Aralkylgruppe bedeutet, |
| $R^6$ | eine $C_1$-$C_6$-Alkyl-, $C_3$-$C_7$-Cycloalkyl-, Aryl-, Aralkyl- oder ein mono-, bi- oder tricyclisches aromatisches System mit Heteroatomen wie Stickstoff, Sauerstoff und Schwefel bedeutet, das mit einer $C_1$-$C_6$-Alkylgruppe verknüpft sein kann, das ebenso wie die Arylreste ein- oder mehrfach durch Nitro, Halogen, Nitril, Hydroxy, Amino, Carboxy, $C_1$-C6-Alkoxycarbonyl, $C_2$-$C_6$-Alkenyloxycarbonyl, $C_2$-$C_6$-Alkinyloxycarbonyl, Aralkoxycarbonyl, $C_1$-$C_6$-Alkyl, $C_3$-$C_7$-Cycloalkyl, $C_2$-$C_6$-Alkenyl, $C_2$-$C_6$-Alkinyl, Cyan-$C_1$-$C_6$-alkyl, $C_1$-$C_6$-Alkoxy, $C_2$-$C_6$-Alkenyloxy, $C_2$-$C_6$-Alkinyloxy, Aralkoxy, Cyan-$C_1$-$C_6$-alkyloxy, $C_1$-$C_6$-Alkylthio, $C_1$-$C_6$-Alkylsulfinyl, $C_1$-$C_6$-Alkylsulfonyl, Amino, $C_1$-$C_6$-Alkylamino, Di-$C_1$-$C_6$-alkylamino, Aralkylamino, Di-aralkyl-amino, $C_1$-$C_6$-Alkylsulfonylamino, $C_1$-$C_6$-Alkylcarbonylamino, Formylamino, Aminocarbonyl, $C_1$-$C_6$-Alkylaminocarbonyl, Di- $C_1$-$C_6$-alkylaminocarbonyl oder durch eine oder mehrere der Gruppen -Y-$CO_2R^8$, -S-Y-$CO_2R^8$, -O-Y-$CO_2R^8$, -NH-Y-$CO_2R^8$, -S-Y-$CONR^8R^9$, -O-Y-$CONR^8R^9$ oder -NH-Y-$CONR^8R^9$ substituiert sein kann, wobei die Alkyl-, Alkenyl- oder Alkinylfragmente ein- oder mehrfach durch Halogen-, Hydroxy-, $C_1$-$C_6$-Alkoxy-, $C_1$-$C_6$-Alkylcarbonyloxy-, Amino- oder Carboxygruppen substituiert sein können, |
| $R^7$ | ein Wasserstoffatom, einen $C_1$-$C_6$-Alkyl-, $C_3$-$C_7$-Cycloalkyl-, $C_2$-$C_6$-Alkenyl- oder $C_2$-$C_6$-Alkinylrest bedeutet, wobei diese Reste ein- oder merfach durch Halogen, Hydroxy, $C_1$-$C_6$-Alkoxy, Amino, $C_1$-$C_6$-Alkylamino, Di-$C_1$-$C_6$-alkylamino, Carboxy, $C_1$-$C_6$-Alkylcarbonyl oder $C_1$-$C_6$-Alkoxycarbonyl substituiert sein können, oder die $C_1$-$C_6$-Alkoxycarbonyl-, Cyan-$C_1$-$C_6$-alkyl-, Aryl-, Aralkyl- oder ein mono-, bi- oder tricyclisches aromatisches System mit Heteroatomen wie Stickstoff, Sauerstoff und Schwefel bedeutet, das mit einer $C_1$-$C_6$-Alkylgruppe verknüpft sein kann, das ebenso wie die Arylreste ein- oder mehrfach durch Halogen, Nitril, $C_1$-$C_6$-Alkyl, $C_2$-$C_6$-Alkenyl, $C_2$-$C_6$-Alkinyl, Halogen-$C_1$-$C_6$-alkyl, $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, Halogen-$C_1$-$C_6$-alkoxy, Hydroxy, Carboxy, Hydroxy-$C_1$-$C_6$-alkyl, Carboxy-$C_1$-$C_6$-alkyl, $C_1$-$C_6$-Alkoxycarbonyl, Amino, $C_1$-$C_6$-Alkylamino, Di-$C_1$-$C_6$-alkylamino, $C_1$-$C_6$-Alkylsulfonylamino, $C_1$-$C_6$-Alkylcarbonylamino, Formylamino, Aminocarbonyl oder Phenyl substituiert sein kann, oder eine Gruppe -Y-$CO_2R^8$ oder -Y-$CONR^8R^9$ bedeutet, |
| Y | eine lineare oder verzweigte $C_1$-$C_6$-Alkylenkette bedeutet, |
| $R^8$ und $R^9$ | gleich oder verschieden sind und Wasserstoffatome, Aralkyl-, $C_3$-$C_7$-Cycloalkyl- oder $C_1$-$C_6$-Alkylgruppen bedeuten, die ein oder mehrfach durch Halogen, Hydroxy, $C_1$-$C_6$-Alkoxy, $C_1$-$C_6$-Alkylcarbonyloxy, Amin oder Carboxy substitiert sein können, oder $R^8$ und $R^9$ zusammen mit dem N-Atom, an das sie gebunden sind, einen gesättigten Ring bilden, der ein zusätzliches Sauerstoff, Schwefel- oder Stickstoffatome enthalten kann, |

sowie Hydrate, Solvate und physiologisch verträgliche Salze davon, und deren optisch aktiven Formen, Racemate und Diastereomerengemische.

2. 4-Aminopyridine der Formel I gemäß Anspruch 1, dadurch gekennzeichnet, daß $R^1$ die $R^6$-$SO_2$-$NR^7$-, $R^6$-$NR^7$-$SO_2$-, $R^6$-$SO_2$-O- oder $R^6$-O-$SO_2$- bedeutet.

3. 4-Aminopyridine der Formel I gemäß Anspruch 1 oder 2, dadurch gekennzeichnet, daß $R^2$ ein Wasserstoff-, Chlor- oder Bromatom, oder eine $C_1$-$C_6$-Alkylgruppe, eine $C_1$-$C_6$-Alkoxygruppe oder die Trifluormethylgruppe bedeutet.

4. 4-Aminopyridine der Formel I gemäß einem der Ansprüche 1 bis 3, dadurch gekennzeichnet, daß X ein Sauerstoffatom oder die NH-Gruppe bedeutet.

5. 4-Aminopyridine der Formel I gemäß einem der Ansprüche 1 bis 4, dadurch gekennzeichnet, daß $R^3$ und $R^4$ gleich oder verschieden sind und Wasserstoffatome oder $C_1$-$C_6$-Alkylgruppen darstellen.

6. 4-Aminopyridine der Formel I gemäß einem der Ansprüche 1 bis 5, dadurch gekennzeichnet, daß $R^5$ ein Wasserstoffatom, eine $C_1$-$C_6$-Alkylgruppe oder die Benzylgruppe darstellt.

7. 4-Aminopyridine der Formel I gemäß einem der Ansprüche 1 bis 6, dadurch gekennzeichnet, daß $R^6$ eine $C_1$-$C_6$-Alkylgruppe, eine $C_3$-$C_7$-Cycloalkylgruppe, eine unsubsituierte oder ein- oder mehrfach durch Fluor, Chlor, $C_1$-$C_6$-Alkyl, $C_1$-$C_6$-Alkoxy, Nitro, Amino, Hydroxy, Carboxy, Benzyloxycarbonyl, $C_1$-$C_6$-Alkoxycarbonly, Trifluormethyl

oder die Gruppe -O-Y-$CO_2R^8$ substituierte Phenyl- oder Benzylgruppe; eine Naphthyl-, Tetrahydronaphthyl-, Biphenyl- oder Indanylgruppe, eine Thienyl-, Pyrazolyl-, oder Pyridyl-, Benzthienyl- oder Benzothiadiazinylgruppe bedeutet.

**8.** 4-Aminopyridine der Formel I gemäß einem der Ansprüche 1 bis 7, dadurch gekennzeichnet, daß $R^7$ ein Wasserstoffatom, eine $C_1$-$C_6$-Alkyl-, $C_2$-$C_6$-Alkenylgruppe oder eine Aralkylgruppe, eine C1-C6-Alkoxycarbonylgruppe, eine Cyanoalkylgruppe, eine Hydroxyalkylgruppe oder eine Aminoalkylgruppe, eine Gruppe -Y-$COR^8$ oder eine Gruppe -Y-$CONR^8R^9$ bedeutet.

**9.** 4-Aminopyridine der Formel 1 gemäß einem der Ansprüche 1 bis 8, dadurch gekennzeichnet, daß Y eine Methylen-, Propylen-, Butylen- oder Pentylengruppe bedeutet.

**10.** 4-Aminopyridine der Formel I gemäß einem der Ansprüche 1 bis 9, dadurch gekennzeichnet, daß $R^8$ ein Wasserstoffatom oder eine $C_1$-$C_6$-Alkylgruppe, eine Hydroxy-$C_1$-$C_6$-alkylgruppe oder eine Amino-$C_1$-$C_6$-alkylgruppe bedeutet.

**11.** 4-Aminopyridine der Formel 1 gemäß einem der Ansprüche 1 bis 10, dadurch gekennzeichnet, daß $R^9$ ein Wasserstoffatom oder eine $C_1$-$C_6$-Alkylgruppe bedeutet.

**12.** Arzneimittel, enthaltend mindestens eine Verbindung der Formel I gemäß einem der Ansprüche 1 bis 11 neben pharmazeutischen Träger- und Hilfsstoffen.

**13.** Verwendung von Verbindungen der Formel I gemäß einem der Ansprüche 1 bis 11 zur Herstellung von Arzneimitteln zur Behandlung von thromboembolischen Erkrankungen.

## Claims

**1.** 4-Aminopyridines of the general formula I

(I)

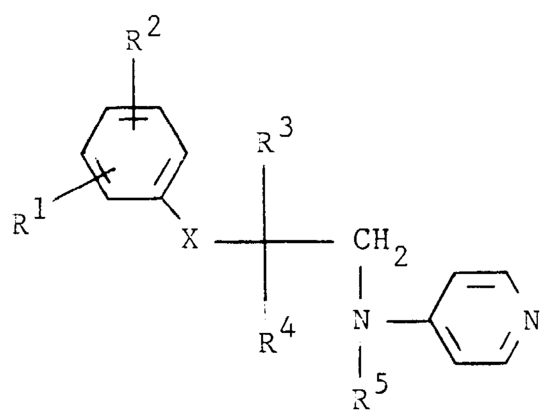

in which

$R^1$ denotes the group $R^6$-SO-$NR^7$-, $R^6$-$SO_2$-$NR^7$-, $R^6$-$NR^7$-SO-, $R^6$-$NR^7$-$SO_2$-, $R^6$-SO-O-, $R^6$-$SO_2$-O-, $R^6$-O-SO- or $R^6$-O-$SO_2$-,
$R^2$ denotes a hydrogen or halogen atom, a cyano, $C_1$-$C_6$-alkyl, $C_1$-$C_6$-alkoxy or halo-$C_1$-$C_6$-alkyl group,
X denotes an oxygen atom, a sulphur atom or the NH group,
$R^3$ and $R^4$ are the same or different and denote hydrogen atoms or alkyl groups,
$R^5$ denotes a hydrogen atom, a $C_1$-$C_6$-alkyl group or the aralkyl group,
$R^6$ denotes a $C_1$-$C_6$-alkyl, $C_3$-$C_7$-cycloalkyl, aryl, aralkyl or a mono-, bi- or tricyclic aromatic system with heteroatoms, such as nitrogen, oxygen and sulphur, which can be linked with a $C_1$-$C_6$-alkyl group, which, like the aryl radical, can be substituted one or several times by nitro, halogen, nitrile, hydroxy, amino, carboxy, $C_1$-$C_6$-alkoxycarbonyl, $C_2$-$C_6$-alkenyloxycarbonyl, $C_2$-$C_6$-alkenyloxycarbonyl, $C_2$-$C_6$-alkynyloxycarbonyl, aralkoxycarbonyl, $C_1$-$C_6$-alkyl, $C_3$-$C_7$-cycloalkyl, $C_2$-$C_6$-alkenyl, $C_2$-$C_6$-alkynyl, cyano-$C_2$-$C_6$-alkyl, $C_1$-$C_6$-alkoxy, $C_2$-

$C_6$-alkenyloxy, $C_2$-$C_6$-alkynyloxy, aralkyloxy, cyano-$C_1$-$C_6$-alkyloxy, $C_1$-$C_6$-alkylthio, $C_1$-$C_6$-alkylsulfinyl, $C_1$-$C_6$-alkylsulfonyl, amino, $C_1$-$C_6$-alkylamino, di-$C_1$-$C_6$-alkylamino, aralkylamino, diaralkylamino, $C_1$-$C_6$-alkylsulfonylamino, $C_1$-$C_6$-alkylcarbonylamino, formylamino, aminocarbonyl, $C_1$-$C_6$-alkylaminocarbonyl, di-$C_1$-$C_6$-alkylaminocarbonyl or by one or more of the groups -Y-$CO_2R^8$, -S-Y-$CO_2R^8$, -O-Y-$CO_2R^8$, -NH-Y-$CO_2R^8$, -S-Y-$CONR^8R^9$, -O-Y-$CONR^8R^9$, whereby the alkyl, alkenyl or alkynyl fragments can be substituted one or more ties by halogen, hydroxy, $C_1$-$C_6$-alkoxy, $C_1$-$C_6$-alkylcarbonyloxy, amino or carboxy groups,

$R^7$ denotes a hydrogen atom, a $C_1$-$C_6$alkyl, $C_3$-$C_7$-cycloalkyl, $C_2$-$C_6$-alkenyl or $C_2$-$C_6$-alkynyl radical, whereby these radicals can be substituted one or more times by halogen, hydroxy, $C_1$-$C_6$-alkoxy, amino, $C_1$-$C_6$-alkylamino, di-$C_1$-$C_6$-alkylamino, carboxy, $C_1$-$C_6$-alkylcarbonyl or $C_1$-$C_6$-alkoxycarbonyl, or denote the $C_l$-$C_6$-alkoxycarbonyl, cyano-$C_1$-$C_6$-alkyl, aryl, aralkyl or a mono-, bi- or tricyclic aromatic system with heteroatoms, such as nitrogen, oxygen and sulphur, which can be linked with a $C_1$-$C_6$-alkyl group which, like the aryl radical, can be substituted one or more times by halogen, nitrile, $C_1$-$C_6$-alkyl, $C_2$-$C_6$-alkenyl, $C_2$-$C_6$-alkynyl, halo-$C_1$-$C_6$-alkyl, $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, halo-$C_1$-$C_6$-alkoxy, hydroxy, carboxy, hydroxy-$C_1$-$C_6$-alkyl, carboxy-$C_1$-$C_6$-alkyl, $C_1$-$C_6$-alkoxycarbonyl, amino, $C_1$-$C_6$-alkylamino, di-$C_1$-$C_6$-alkylamino, $C_1$-$C_6$-alkylsulfonylamino, $C_1$-$C_6$-alkylcarbonylamino, formylamino, aminocarbonyl or phenyl, or denotes a group -Y-$CO_2R^8$ or -Y-$CONR^8R^9$ group,

Y denotes a linear or branched $C_1$-$C_6$-alkylene chain,

$R^8$ and $R^9$ are the same or different and denote hydrogen atoms, aralkyl, $C_3$-$C_7$-cycloalkyl or $C_1$-$C_6$-alkyl groups, which can be substituted one or more times by halogen, hydroxy, $C_1$-$C_6$-alkoxy, $C_1$-$C_6$-alkylcarbonyloxy, amine or carboxy, or $R^8$ and $R^9$, together with the N atom to which they are bound, form a saturated ring which can contain an additional oxygen, sulphur or nitrogen atom,

as well as hydrates, solvates and physiologically compatible salts thereof, and their optically-active forms, racemates and diastereomeric mixtures.

**2.** 4-Aminopyridines of formula I according to claim 1, characterised in that $R^1$ denotes $R^6$-SO-$NR^7$-, $R^6$-$NR^7$-$SO_2$-, $R^6$-$SO_2$-O- or $R^6$-O-$SO_2$.

**3.** 4-Aminopyridines of formula I according to claim 1 or 2, characterised in that $R^2$ denotes a hydrogen, chlorine or bromine atom, or a $C_1$-$C_6$-alkyl group, a $C_1$-$C_6$-alkoxy group or the trifluoromethyl group.

**4.** 4-Aminopyridines of formula I according to one of the claims 1 to 3, characterised in that X denotes an oxygen atom or the NH group.

**5.** 4-Aminopyridines of formula I according to one of the claims 1 to 4, characterised in that $R^3$ and $R^4$ are the same or different and represent hydrogen atoms or $C_1$-$C_6$-alkyl groups.

**6.** 4-Aminopyridines of formula I according to one of the claims 1 to 5, characterised in that $R^5$ represents a hydrogen atom, a $C_1$-$C_6$-alkyl group or the benzyl group.

**7.** 4-Aminopyridines of formula I according to one of the claims 1 to 6, characterised in that $R^6$ denotes a $C_1$-$C_6$-alkyl group, a $C_3$-$C_7$-cycloalkyl group, an unsubstituted phenyl or benzyl group or a phenyl or benzyl group substituted one or more times by fluorine, chlorine, $C_1$-$C_6$-alkyl, $C_1$-$C_6$-alkoxy, nitro, amino, hydroxy, carboxy, benzyloxycarbonyl, $C_1$-$C_6$-alkoxycarbonyl, trifluoromethyl or the group -O-Y-$CO_2R^8$; a naphthyl, tetrahydronaphthyl, biphenyl or indanyl group, a thienyl, pyrazolyl or pyridyl, benzthienyl or benzothiadiazinyl group.

**8.** 4-Aminopyridines of formula I according to one of the claims 1 to 7, characterised in that $R^7$ denotes a hydrogen atom, a $C_1$-$C_6$-alkyl, $C_2$-$C_6$-alkenyl group or an aralkyl group, a $C_1$-$C_6$-alkoxycarbonyl group, a cyanoalkyl group, a hydroxyalkyl group or an aminoalkyl group, a group -Y-$COR^8$ or a group -Y-$CONR^8R^9$.

**9.** 4-Aminopyridines of formula I according to one of the claims 1 to 8, characterised in that Y denotes a methylene, propylene, butylene or pentylene group.

**10.** 4-Aminopyridines of formula I according to one of the claims 1 to 9, characterised in that $R^8$ denotes a hydrogen atom or a $C_1$-$C_6$-alkyl group, a hydroxy-$C_1$-$C_6$-alkyl group or an amino-$C_1$-$C_6$-alkyl group.

**11.** 4-Aminopyridines of formula I according to one of the claims 1 to 10, characterised in that $R^9$ denotes a hydrogen atom or a -$C_1$-$C_6$-alkyl group.

**12.** Medicaments containing at least one compound of formula I according to one of the claims 1 to 11, besides pharmaceutical carrier and auxiliary substances.

**13.** Use of compounds of formula I according to one of the claims 1 to 11 for the production of medicaments for the treatment of thromboembolic diseases.

**Revendications**

**1.** 4-Aminopyridine de formule générale I

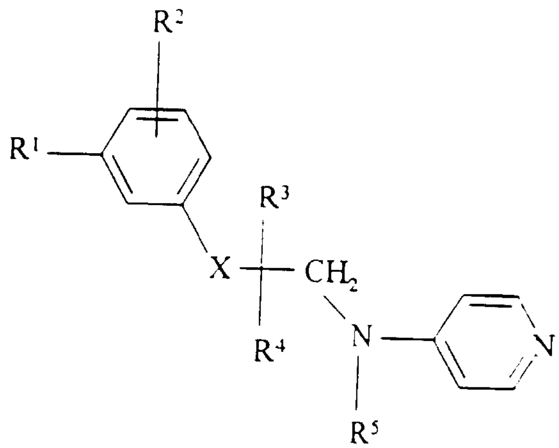

dans laquelle

$R^1$ représente le groupe $R^6$-SO-$NR^7$-, $R^6$-$SO_2$-$NR^7$-, $R^6$-$NR^7$-SO-, $R^6$-$NR^7$-$SO_2$-, $R^6$-SO-O-, $R^6$-$SO_2$-O-, $R^6$-O-SO- ou $R^6$-O-$SO_2$-,

$R^2$ représente un atome d'hydrogène ou un atome d'halogène, un groupe cyano, alkyle en $C_1$-$C_6$, alcoxy en $C_1$-$C_6$ ou halogéno-alkyle en $C_1$-$C_6$,

X représente un atome d'oxygène, un atome de soufre ou le groupe NH,

$R^3$ et $R^4$ sont identiques ou différents et représentent des atomes d'hydrogène ou des groupes alkyle en $C_1$-$C_6$,

$R^5$ représente un atome d'hydrogène, un groupe alkyle en $C_1$-$C_6$ ou un groupe aralkyle,

$R^6$ représente un groupe alkyle en $C_1$-$C_6$, cycloalkyle en $C_3$-$C_7$, aryle, aralkyle ou un système aromatique mono-, bi- ou tricyclique, comportant des hétéroatomes tels que l'azote, l'oxygène et le soufre, qui peut être lié à un groupe alkyle en $C_1$-$C_6$, qui peut être substitué, ainsi que le reste aryle, une ou plusieurs fois par un groupe nitro, halogéno, nitrile, hydroxy, amino, carboxy, alcoxy($C_1$-$C_6$)carbonyle, alcényloxy($C_2$-$C_6$)carbonyle, alcinyloxy($C_2$-$C_6$)carbonyle, aralcoxycarbonyle, alkyle en $C_1$-$C_6$, cycloalkyle en $C_3$-$C_7$, alcényle en $C_2$-$C_6$, alcinyle en $C_2$-$C_6$, cyano(alkyle en $C_1$-$C_6$), alcoxy en $C_1$-$C_6$, alcényloxy en $C_2$-$C_6$, alcinyloxy en $C_2$-$C_6$, aralcoxy, cyano(alkyloxy en $C_1$-$C_6$), alkylthio en $C_1$-$C_6$, alkyl($C_1$-$C_6$)sulfinyle, alkyl($C_1$-$C_6$)sulfonyle, amino, alkyl($C_1$-$C_6$)-amino, di(alkylamino en $C_1$-$C_6$), aralkylamino, di(aralkyl-amino), (alkyle en $C_1$-$C_6$)sulfonylamino, (alkyle en $C_1$-$C_6$)-carbonylamino, formylamino, aminocarbonyle, (alkyle en $C_1$-$C_6$)-aminocarbonyle, di-((alkyle en $C_1$-$C_6$)-aminocarbonyle), ou par un ou plusieurs des groupes -Y-$CO_2R^8$, -S-Y-$CO_2R^8$, -O-Y-$CO_2R^8$, -NH-Y-$CO_2R^8$, -S-Y-$CONR^8R^9$, -O-Y-$CONR^8R^9$ ou -NH-Y-$CONR^8R^9$, les fragments alkyle, alcényle ou alcinyle pouvant être substitués une ou plusieurs fois par un groupe halogéno, hydroxy, alcoxy en $C_1$-$C_6$, (alkyle en $C_1$-$C_6$)-carbonyloxy, amino ou carboxy,

$R^7$ représente un atome d'hydrogène, un groupe alkyle en $C_1$-$C_6$, cycloalkyle en $C_3$-$C_7$, alcényle en $C_2$-$C_6$, alcinyle en $C_2$-$C_6$, ces groupes pouvant être substitués une ou plusieurs fois par une groupe halogéno, hydroxy, alcoxy en $C_1$-$C_6$, amino, (alkyle en $C_1$-$C_6$)amino, di(alkyle en $C_1$-$C_6$)amino, carboxy, (alkyle en $C_l$-$C_6$)carbonyle ou (alcoxy en $C_1$-$C_6$)-carbonyle, ou il représente un groupe (alcoxy en $C_1$-$C_6$)carbonyle, cyano(alkyle en $C_l$-$C_6$), aryle, aralkyle, ou un système aromatique mono-, bi- ou tricyclique, comportant des hétéroatomes tels que l'azote, l'oxygène et le soufre, qui peut être lié à un groupe alkyle en $C_1$-$C_6$, qui peut être substitué, ainsi que le reste aryle, une ou plusieurs fois par un groupe halogéno, nitrile, alkyle en $C_1$-$C_6$, alcényle en $C_2$-$C_6$, alcinyle en $C_2$-$C_6$, halogéno (alkyle en $C_1$-$C_6$), alcoxy en $C_1$-$C_6$, alcényloxy en $C_2$-$C_6$, alcinyloxy en $C_2$-$C_6$, alkylthio en $C_1$-$C_6$, alkyl($C_1$-$C_6$)sulfinyle, alkyl($C_1$-$C_6$)sulfonyle, halogéno(alcoxy en $C_1$-$C_6$), hydroxy, carboxy, hydroxy (alkyle en $C_1$-$C_6$), carboxy(alkyle en $C_1$-$C_6$), (alcoxy en $C_1$-$C_6$)-carbonyle, amino, (alkyle en $C_1$-$C_6$) amino, di((alkyle en $C_1$-$C_6$)amino), alkyl($C_1$-$C_6$)sulfonylamino, alkyl($C_1$-$C_6$)carbonylamino, formylamino, aminocarbonyle ou phényle, ou un groupe -Y-$CO_2R^8$ ou -Y-$CONR^8R^9$,

Y représente une chaîne alkylène en $C_1$-$C_6$ linéaire ou ramifiée,

$R^8$ et $R^9$ sont identiques ou différents et représentent des atomes d'hydrogène, des groupes aralkyle, cycloalkyle en $C_3$-$C_7$ ou alkyle en $C_1$-$C_6$, qui peuvent être substitués une ou plusieurs fois par un groupe halogéno, hydroxy, alcoxy en $C_1$-$C_6$, (alkyle en $C_1$-$C_6$)carbonyloxy, amino ou carboxy, ou $R^8$ et $R^9$ peuvent, ensemble avec l'atome de N auquel ils sont liés, former un cycle saturé, qui peut contenir un atome d'oxygène, de soufre ou d'azote supplémentaire,

ainsi que ses hydrates, ses formes solvatées et ses sels physiologiquement acceptables, et leurs formes optiquement actives, leurs racémates et leurs mélanges de diastéréomères.

**2.** 4-Aminopyridine de formule I selon la revendication 1, caractérisée par le fait que $R^1$ représente le groupe $R^6$-$SO_2$-$NR^7$-, $R^6$-$NR^7$-$SO_2$-, $R^6$-$SO_2$-O- ou $R^6$-O-$SO_2$-.

**3.** 4-Aminopyridine de formule I selon la revendication 1 ou 2, caractérisée par le fait que $R^2$ représente un atome d'hydrogène, un atome de chlore ou de brome, ou un groupe alkyle en $C_1$-$C_6$, alcoxy en $C_1$-$C_6$ ou un groupe trifluorométhyle.

**4.** 4-Aminopyridine de formule I selon l'une quelconque des revendications 1 à 3, caractérisée par le fait que X représente un atome d'oxygène ou le groupe NH.

**5.** 4-Aminopyridine de formule I selon l'une quelconque des revendications 1 à 4, caractérisée par le fait que $R^3$ et $R^4$ sont identiques ou différents et représentent des atomes d'hydrogène ou des groupes alkyle en $C_1$-$C_6$.

**6.** 4-Aminopyridine de formule I selon l'une quelconque des revendications 1 à 5, caractérisée par le fait que $R^5$ représente un atome d'hydrogène, un groupe alkyle en $C_1$-$C_6$ ou le groupe benzyle.

**7.** 4-Aminopyridine de formule I selon l'une quelconque des revendications 1 à 6, caractérisée par le fait que $R^6$ représente un groupe alkyle en $C_1$-$C_6$, cycloalkyle en $C_3$-$C_7$, un groupe phényle ou benzyle non substitué ou substitué une ou plusieurs fois par un atome de fluor, de chlore, un groupe alkyle en $C_1$-$C_6$, alcoxy en $C_1$-$C_6$, nitro, amino, hydroxy, carboxy, benzyloxycarbonyle, (alcoxy en $C_1$-$C_6$)-carbonyle, trifluorométhyle ou le groupe -O-Y-$CO_2R^8$ ; un groupe naphtyle, tétrahydronaphtyle. biphényle ou indanyle, un groupe thiényle, pyrazolyle, ou pyridyle, benzothiényle ou benzothiadiazinyle.

**8.** 4-Aminopyridine de formule générale I selon l'une quelconque des revendications 1 à 7, caractérisée par le fait que $R^7$ représente un atome d'hydrogène, un groupe alkyle en $C_1$-$C_6$, alcényle en $C_2$-$C_6$ ou un groupe aralkyle, un groupe (alcoxy en $C_1$-$C_6$)carbonyle, un groupe cyanoalkyle, un groupe hydroxyalkyle ou un groupe aminoalkyle, un groupe -Y-$COR^8$ ou un groupe -Y-$CONR^8R^9$.

**9.** 4-Aminopyridine de formule générale I selon l'une quelconque des revendications 1 à 8, caractérisée par le fait que Y représente un groupe méthylène, propylène, butylène ou pentylène.

**10.** 4-Aminopyridine de formule générale I selon l'une quelconque des revendications 1 à 9, caractérisée par le fait que $R^8$ représente un atome d'hydrogène ou un groupe alkyle en $C_1$-$C_6$, un groupe hydroxy(alkyle en $C_1$-$C_6$) ou

un groupe amino(alkyle en $C_1$-$C_6$).

**11.** 4-Aminopyridine de formule générale I selon l'une quelconque des revendications 1 à 10, caractérisée par le fait que $R^9$ représente un atome d'hydrogène ou un groupe alkyle en $C_1$-$C_6$.

**12.** Médicament contenant au moins un composé de formule I selon l'une quelconque des revendications 1 à 11, en plus de véhicules et adjuvants pharmaceutiques.

**13.** Utilisation d'un composé de formule I selon l'une quelconque des revendications 1 à 11 pour la préparation de médicaments destinés au traitement de maladies thromboemboliques.